(19) Europäisches Patentamt
European Patent Office
Office européen des brevets

(11) EP 4 736 885 A1

(12) # EUROPEAN PATENT APPLICATION
published in accordance with Art. 153(4) EPC

(43) Date of publication:
**06.05.2026 Bulletin 2026/19**

(21) Application number: **24830941.1**

(22) Date of filing: **28.06.2024**

(51) International Patent Classification (IPC):
***A61K 47/68*** *(2017.01)* ***C07K 16/28*** *(2006.01)*
***A61K 39/395*** *(2006.01)* ***A61K 31/4745*** *(2006.01)*
***A61P 35/00*** *(2006.01)* ***A61P 35/02*** *(2006.01)*

(52) Cooperative Patent Classification (CPC):
**A61K 31/4745; A61K 39/395; A61K 47/68; A61P 35/00; A61P 35/02; C07K 16/28**

(86) International application number:
**PCT/CN2024/102249**

(87) International publication number:
**WO 2025/002310 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.06.2023 CN 202310780680**
**30.01.2024 CN 202410125580**

(71) Applicant: **CSPC Megalith Biopharmaceutical Co., Ltd.**
**Shijiazhuang, Hebei 050025 (CN)**

(72) Inventors:
- **GAO, Xiao**
  **Shijiazhuang, Hebei 050025 (CN)**
- **PAN, Fujun**
  **Shijiazhuang, Hebei 050025 (CN)**
- **HUI, Xiwu**
  **Shijiazhuang, Hebei 050025 (CN)**
- **ZHAO, Lu**
  **Shijiazhuang, Hebei 050025 (CN)**
- **FAN, Lixue**
  **Shijiazhuang, Hebei 050025 (CN)**
- **DAN, Mo**
  **Shijiazhuang, Hebei 050025 (CN)**
- **WU, Yufen**
  **Shijiazhuang, Hebei 050025 (CN)**
- **SUN, Xiongfei**
  **Shijiazhuang, Hebei 050025 (CN)**
- **WANG, Mingxiao**
  **Shijiazhuang, Hebei 050025 (CN)**
- **YAO, Bing**
  **Shijiazhuang, Hebei 050025 (CN)**

(74) Representative: **Gille Hrabal Partnerschaftsgesellschaft mbB Patentanwälte**
**Brucknerstraße 20**
**40593 Düsseldorf (DE)**

Remarks:
The complete document including Reference Table(s) and the Sequence Listing(s) can be downloaded from the EPO website

(54) ## ANTIBODY-DRUG CONJUGATE AND USE THEREOF

(57) Provided are an antibody-drug conjugate and the use thereof, in particular an antibody-drug conjugate targeting B7H3. Also provided are an antibody targeting B7H3 and the use thereof.

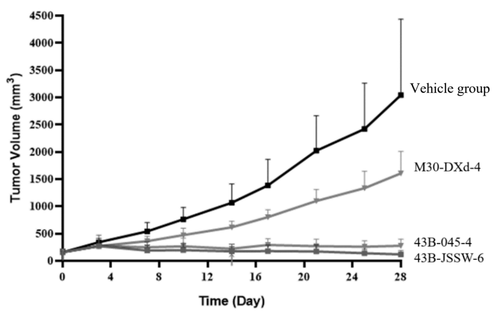


FIG. 24



EP 4 736 885 A1

## Description

## CROSS-REFERENCE TO RELATED APPLICATIONS

The present application claims priority to the prior application with the patent application No. 2023107806802 filed with China National Intellectual Property Administration on June 29, 2023, and the prior application with the patent application No. 2024101255800 filed with China National Intellectual Property Administration on January 30, 2024, both of which are entitled "ANTIBODY-DRUG CONJUGATE" and are incorporated herein by reference in their entirety.

## TECHNICAL FIELD

The present disclosure relates to the field of biopharmaceuticals, and in particular to an antibody-drug conjugate, a preparation method therefor, and use thereof.

## BACKGROUND

An antibody-drug conjugate (ADC) consists of three different components: an antibody, a linker, and a bioactive molecule (drug). The ADC technology conjugates an antibody and a drug molecule together via a linker, utilizing the specificity of the antibody to achieve targeted delivery of the drug molecule to a target tissue to exert its effect, thereby reducing the systemic toxic and side effects of the drug, widening the therapeutic window of the drug, and expanding the therapeutic potential of the antibody.

Bioactive molecules (drugs) are essential for ADC drugs to function. Camptothecin compounds have attracted widespread attention due to their excellent anti-tumor effects. The marketed ADC drugs Trodelvy and Enhertu adopt camptothecin compounds SN38 and Dxd as cytotoxic substances, respectively. Among them, Dxd exhibits 10 times higher activity compared to the common chemotherapeutic drug irinotecan; it has a strong ability to penetrate cell membranes, which enables it to kill nearby cancer cells after killing cancer cells that have ingested the ADC, thereby producing a "bystander effect"; its half-life in blood is significantly shortened, which helps to reduce the generation of toxic and side effects.

However, ADCs ultimately prepared using camptothecin compounds greatly alter the properties of monoclonal antibodies, resulting in a relatively considerable degree of degradation in both stability and half-life. Although Enhertu achieved a confirmed objective response rate (ORR) of up to 79.7% in the DESTINY-Breast 03 clinical study, the DS8201-A-J101 clinical study demonstrated that for breast cancer with low HER2 expression, Enhertu only achieved a confirmed ORR of 37.0%, indicating a significantly lower treatment efficacy compared to breast cancer with high or medium HER2 expression. Furthermore, ADC drugs have exhibited new symptoms in terms of toxic and side effects. For example, Enhertu has shown side effects such as pneumonia, interstitial lung disease, etc.

WO2020233174A1 discloses a camptothecin compound containing a valine-citrulline (Val-Cit)-PAB linker; however, this molecule cannot yield an ADC molecule of qualified quality after conjugation with an antibody (a qualified ADC product requires an aggregate content of less than 5%). Therefore, there is an urgent need in the art to provide more suitable antibody-drug conjugates based on camptothecin drugs (e.g., exatecan, belotecan, etc.) to achieve efficient, simple, and practical chemical preparation and conjugation, and to improve the pharmaceutical properties, metabolic properties, efficacy, safety, etc., of existing antibody-drug conjugates, such as improving the stability of ADC molecules, widening the therapeutic window, etc.

B7H3 (full name: B7 homolog 3), also known as CD276, is a newly identified ligand of the B7 family belonging to type I transmembrane glycoproteins. Its extracellular domain is composed of two immunoglobulin constant (IgC) and variable (IgV) domains. It primarily exists in two splice isomers: 2IgB7H3 whose extracellular region is composed of IgV-IgC and 4IgB7H3 whose extracellular region is composed of IgV-IgC-IgV-IgC. Both murine and human B7H3 include 2IgB7-H3, but humans and other primates also have the four immunoglobulin-like domain structure of 4IgB7H3.

The receptor for B7H3 has not been clearly identified, and its mechanism of action is not fully understood. Current research indicates that B7H3 possesses both immunosuppressive and immune-activating functions, with the prevailing view being that B7H3 primarily plays an immunosuppressive role. Some studies report that B7H3 is a negative regulator of T cell function, mainly affecting Th1. However, some studies have shown that it mainly inhibits CD8+ T cells and NK cells, directly suppresses NK cell activation, and has no significant effect on CD4+ T cells. B7H3 exerts its inhibitory effect on T cells by suppressing IL-2 secretion, and this effect can be reversed by exogenous addition of IL-2, indicating that B7H3 is an upstream regulatory molecule of IL-2. When DCs are co-cultured with Tregs, the DCs up-regulate B7H3 and reduce pMHC expression, resulting in impaired T cell stimulation function. B7H3 expression is negatively correlated with the response to PD-1 therapy in NSCLC. B7H3 expression is negatively correlated with CD8+ T cell infiltration. Patients with high CD8+ T cell infiltration show a better response to IO therapy.

In tumor cells, B7H3 may be involved in tumor growth and immune escape mechanisms. B7H3 can regulate the

migration, invasion, and adhesion of various cancer cells to fibronectin through the Jak2/Stat3/MMP-9 signaling pathway. Furthermore, overexpression of B7H3 in colorectal cancer and breast cancer cells can enhance the resistance to apoptosis by activating the Jak2/STAT3/survivin signaling pathway, thereby reducing the sensitivity of the tumor cells to the chemotherapeutic drug paclitaxel. B7H3 expression can promote glycolysis in cancer cells. Finally, B7H3 can promote the expression of VEGF, thereby facilitating tumor angiogenesis. Clinical data from patients with multiple cancer types show that overexpression of B7H3 is associated with poor prognosis, including tumor progression, metastasis, etc.

B7H3 mRNA is widely present in many tissues of the human body, such as the heart, prostate, pancreas, liver, colon, thymus, etc., and immunohistochemical (IHC) analysis of a large number of normal tissues shows that B7H3 is expressed at low levels or is even undetectable only in a few tissues. Staining of tissue microarrays from Biomax USA with a B7H3-specific monoclonal antibody resulted in weak cytoplasmic staining only in salivary gland acinar cells, gastric epithelial cells, and adrenal cells. In other studies, only weak staining of the basolateral membrane of the stomach, gallbladder, prostate, cervix, and endometrium was observed. B7H3 is widely expressed in various solid tumors, such as head and neck cancer, bladder cancer, lung cancer, kidney cancer, glioma, prostate cancer, breast cancer, ovarian cancer, etc., making it a good target for ADC drugs.

## SUMMARY

The present disclosure provides an antibody-drug conjugate having a structure represented by formula (I), and a tautomer, a mesomer, a racemate, an enantiomer, a diastereoisomer, a pharmaceutically acceptable salt, a hydrate, a solvate or an isotopically labeled compound thereof:

$$A\text{-}(L\text{-}D)_d \qquad (I)$$

wherein A is selected from an antibody (e.g., a monoclonal antibody) or an antigen-binding fragment thereof;
L is a linker moiety, with one end linked to A and the other end linked to a bioactive molecule D;
D is a bioactive molecule;
d represents the molar ratio of the bioactive molecule to A (also known as DAR, i.e., drug-to-antibody ratio), and is an integer or a decimal selected from 1 to 12 (e.g., 1, 2, 3, 4, 5, 6, 7, 8, 9, 10, 11, or 12);
when d is a decimal, it refers to the average number of linker-bioactive molecules (L-D) conjugated per A. In some embodiments, A is selected from a monoclonal antibody or an antigen-binding fragment thereof.

In some embodiments, A is selected from an antibody or an antigen-binding fragment thereof targeting HER2 (ErbB2), HER3 (ErbB3), HER4 (ErbB4), EGFR, DLL3, TROP2, B7H3, B7H4, TF (Tissue factor), c-Met, CD20, CD22, CD30, CD33, CD44, CD47, CD56, CD70, CD73, CD79b, CD105, CEA, A33, Cripto, EphA2, G250, MUC1, Lewis Y, EDB-FN, VEGFR, VEGF, PD-1, PD-L1, MET, RET, GPNMB, Integrin, PSMA, Tenascin-C, SLC44A4, Claudin18.2, or Mesothelin. In some embodiments, A is selected from an antibody or an antigen-binding fragment thereof targeting B7H3, HER2, TROP2, B7H4, TF, EDB-FN, and Claudin18.2.

In some embodiments, A is selected from an antibody or an antigen-binding fragment thereof targeting B7H3 or HER2.

In some embodiments, A is an antibody or an antigen-binding fragment thereof targeting B7H3; the antibody or the antigen-binding fragment thereof comprises a heavy chain and/or a light chain, wherein the heavy chain comprises 3 complementarity determining regions (CDRs), wherein the amino acid sequence of the heavy chain complementarity determining region 1 (HCDR1) is set forth in SEQ ID NO. 1, the amino acid sequence of the heavy chain complementarity determining region 2 (HCDR2) is set forth in SEQ ID NO. 2, and the amino acid sequence of the heavy chain complementarity determining region 3 (HCDR3) is set forth in SEQ ID NO. 3; the light chain comprises 3 complementarity determining regions (CDRs) of the light chain, wherein the amino acid sequence of the light chain complementarity determining region 1 (LCDR1) is set forth in SEQ ID NO. 4, the amino acid sequence of the light chain complementarity determining region 2 (LCDR2) is set forth in SEQ ID NO. 5, and the amino acid sequence of the light chain complementarity determining region 3 (LCDR3) is set forth in SEQ ID NO. 6; the CDRs are determined according to the Kabat numbering scheme.

In some embodiments, A is an antibody or an antigen-binding fragment thereof targeting B7H3, wherein the anti-B7H3 antibody or the antigen-binding fragment thereof comprises a heavy chain and/or a light chain, wherein the sequence of a variable region of the heavy chain (HV) is set forth in SEQ ID NO. 7, and the sequence of a variable region of the light chain (LV) is set forth in SEQ ID NO. 8.

In some embodiments, A is an antibody targeting B7H3, wherein the anti-B7H3 antibody further comprises a heavy chain constant region sequence or a variant thereof, and/or a light chain constant region sequence or a variant thereof.

In some embodiments, A is an antigen-binding fragment targeting B7H3, wherein the antigen-binding fragment is

selected from a Fab, a Fab', a Fab'-SH, an Fv, an scFv, and a $F(ab')_2$.

In some embodiments, A is an antibody or an antigen-binding fragment thereof targeting B7H3, wherein the anti-B7H3 antibody or the antigen-binding fragment thereof is a humanized or fully human antibody or an antigen-binding fragment thereof.

In some embodiments, A is an antibody or an antigen-binding fragment thereof targeting B7H3; the anti-B7H3 antibody comprises a heavy chain and/or a light chain, wherein the amino acid sequence of the heavy chain is set forth in SEQ ID NO. 9 or SEQ ID NO. 10, and the amino acid sequence of the light chain is set forth in SEQ ID NO. 11.

In some embodiments, A is selected from 43A or 43B targeting B7H3, DP001 targeting HER2, sacituzumab targeting TROP2, alsevalimab targeting B7H4, tisotumab targeting TF, L19A targeting EDB-FN, zolbetuximab targeting Claudin18.2, and biosimilars thereof.

43A comprises 2 identical heavy chains and 2 identical light chains, wherein the amino acid sequence of the heavy chain is set forth in SEQ ID NO. 9, and the amino acid sequence of the light chain is set forth in SEQ ID NO. 11.

> 43A heavy chain (SEQ ID NO. 9)

QVQLVQSGAEVKKPGASVKVSCKASGYTFT**DYFMN**WVRQAPGQGLEWMG**DVNPKTGS**
**PSYNQKFKG**RVTMTRDTSTSTVYMELSSLRSEDTAVYYCAR**RYGFLYSMDY**WGQGTSV
TVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPEAAG
GPSVFLFPPKPKDTLMISRTPEVTCVVVDVSHEDPEVKFNWYVDGVEVHNAKTKPREEQY
NSTYRVVSVLTVLHQDWLNGKEYKCKVSNKALAASIEKTISKAKGQPREPQVYTLPPSREE
MTKNQVSLTCLVKGFYPSDIAVEWESNGQPENNYKTTPPVLDSDGSFFLYSKLTVDKSR
WQQGNVFSCSVMHEALHNHYTQKSLSLSPGK

> 43A light chain (SEQ ID NO. 11)

DIQMTQSPSSLSASVGDRVTITC**RASQDISNYLN**WYQQKPGKAVKVLIY**YTSRLHS**GVPSR
FSGSGSGTDFTFTISSLQPEDIATYYC**QQGNTHPFT**FGGGTKVEIKRTVAAPSVFIFPPSDEQ
LKSGTASVVCLLNNFYPREAKVQWKVDNALQSGNSQESVTEQDSKDSTYSLSSTLTLSKA
DYEKHKVYACEVTHQGLSSPVTKSFNRGEC

The amino acid sequences marked in bold are the heavy chain/light chain CDR sequences, and the HCDR1, HCDR2, HCDR3, LCDR1, LCDR2, and LCDR3 are designated as SEQ ID NO. 1, SEQ ID NO. 2, SEQ ID NO. 3, SEQ ID NO. 4, SEQ ID NO. 5, and SEQ ID NO. 6, respectively:

HCDR1: DYFMN

HCDR2: DVNPKTGSPSYNQKFKG

HCDR3: RYGFLYSMDY

LCDR1: RASQDISNYLN

LCDR2: YTSRLHS

LCDR3: QQGNTHPFT

The amino acid sequences marked with underlines are the heavy chain/light chain variable region sequences, and the HV and LV are designated as SEQ ID NO. 7 and SEQ ID NO. 8, respectively.

HV:

QVQLVQSGAEVKKPGASVKVSCKASGYTFTDYFMNWVRQAPGQGLEWMGDVNPKTGSP
SYNQKFKGRVTMTRDTSTSTVYMELSSLRSEDTAVYYCARRYGFLYSMDYWGQGTSVTV
SS

LV:

DIQMTQSPSSLSASVGDRVTITCRASQDISNYLNWYQQKPGKAVKVLIYYTSRLHSGVPSRF
SGSGSGTDFTFTISSLQPEDIATYYCQQGNTHPFTFGGGTKVEIK

43B comprises 2 identical heavy chains and 2 identical light chains, wherein the amino acid sequence of the heavy chain is set forth in SEQ ID NO. 10, and the amino acid sequence of the light chain is set forth in SEQ ID NO. 11.

> 43B heavy chain (SEQ ID NO. 10)

QVQLVQSGAEVKKPGASVKVSCKASGYTFT**DYFMN**WVRQAPGQGLEWMG**DVNPKTGS**
**PSYNQKFKG**RVTMTRDTSTSTVYMELSSLRSEDTAVYYCAR**RYGFLYSMDY**WGQGTSV
TVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTSPPCPAPEAAG
GPSVFLFPPKPKDTLMISRTPEVTCVVVDVSHEDPEVKFNWYVDGVEVHNAKTKPREEQY
NSTYRVVSVLTVLHQDWLNGKEYKCKVSNKALAASIEKTISKAKGQPREPQVYTLPPSREE
MTKNQVSLTCLVKGFYPSDIAVEWESNGQPENNYKTTPPVLDSDGSFFLYSKLTVDKSR
WQQGNVFSCSVMHEALHNHYTQKSLSLSPGK

> 43B light chain (SEQ ID NO. 11)

DIQMTQSPSSLSASVGDRVTITC**RASQDISNYLN**WYQQKPGKAVKVLIY**YTSRLHS**GVPSR
FSGSGSGTDFTFTISSLQPEDIATYYC**QQGNTHPFT**FGGGTKVEIKRTVAAPSVFIFPPSDEQ
LKSGTASVVCLLNNFYPREAKVQWKVDNALQSGNSQESVTEQDSKDSTYSLSSTLTLSKA
DYEKHKVYACEVTHQGLSSPVTKSFNRGEC

The amino acid sequences marked in bold are the heavy chain/light chain CDR sequences, and the HCDR1, HCDR2, HCDR3, LCDR1, LCDR2, and LCDR3 are designated as SEQ ID NO. 1, SEQ ID NO. 2, SEQ ID NO. 3, SEQ ID NO. 4, SEQ ID NO. 5, and SEQ ID NO. 6, respectively:

HCDR1: DYFMN

HCDR2: DVNPKTGS PSYNQKFKG

HCDR3: RYGFLYSMDY

LCDR1: RASQDISNYLN

LCDR2: YTSRLHS

LCDR3: QQGNTHPFT

The amino acid sequences marked with underlines are the heavy chain/light chain variable region sequences, and the HV and LV are designated as SEQ ID NO. 7 and SEQ ID NO. 8, respectively.

HV:

QVQLVQSGAEVKKPGASVKVSCKASGYTFTDYFMNWVRQAPGQGLEWMGDVNPKTGSP
SYNQKFKGRVTMTRDTSTSTVYMELSSLRSEDTAVYYCARRYGFLYSMDYWGQGTSVTV
SS

LV:

DIQMTQSPSSLSASVGDRVTITCRASQDISNYLNWYQQKPGKAVKVLIYYTSRLHSGVPSRF
SGSGSGTDFTFTISSLQPEDIATYYCQQGNTHPFTFGGGTKVEIK

DP001 comprises 2 identical heavy chains and 2 identical light chains, wherein the amino acid sequence of the heavy chain is set forth in SEQ ID NO. 12, and the amino acid sequence of the light chain is set forth in SEQ ID NO. 13.

> DP001 heavy chain (SEQ ID NO. 12)

EVQLVESGGGLVQPGGSLRLSCAASGFNIKDTYIHWVRQAPGKGLEWVARIYPTNGYTRY
ADSVKGRFTISADTSKNTAYLQMNSLRAEDTAVYYCSRWGGDGFYAMDYWGQGTLVTV
SSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFFLYSKLTVDKSRWQQ
GNVFSCSVMHEALHNHYTQKSLSLSPGK

> DP001 light chain (SEQ ID NO. 13)

DIQMTQSPSSLSASVGDRVTITCRASQDVNTAVAWYQQKPGKAPKLLIYSASFLYSGVPSR
FSGSRSGTDFTLTISSLQPEDFATYYCQQHYTTPPTFGQGTKVEIKRTVAAPSVFIFPPSDEQL
KSGTASVVCLLNNFYPREAKVQWKVDNALQSGNSQESVTEQDSKDSTYSLSSTLTLSKAD
YEKHKVYACEVTHQGLSSPVTKSFNRGEC

sacituzumab comprises 2 identical heavy chains and 2 identical light chains, wherein the amino acid sequence of the heavy chain is set forth in SEQ ID NO. 14, and the amino acid sequence of the light chain is set forth in SEQ ID NO. 15.
> Sacituzumab heavy chain (SEQ ID NO. 14)

QVQLQQSGSELKKPGASVKVSCKASGYTFTNYGMNWVKQAPGQGLKWMGWINTYTGEP
TYTDDFKGRFAFSLDTSVSTAYLQISSLKADDTAVYFCARGGFGSSYWYFDVWGQGSLVT
VSSASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKRVEPKSCDKTHTCPPCPAPELLGGP
SVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFFLYSKLTVDKSRWQQ
GNVFSCSVMHEALHNHYTQKSLSLSPGK

> Sacituzumab light chain (SEQ ID NO. 15)

DIQLTQSPSSLSASVGDRVSITCKASQDVSIAVAWYQQKPGKAPKLLIYSASYRYTGVPDRF
SGSGSGTDFTLTISSLQPEDFAVYYCQQHYITPLTFGAGTKVEIKRTVAAPSVFIFPPSDEQL
KSGTASVVCLLNNFYPREAKVQWKVDNALQSGNSQESVTEQDSKDSTYSLSSTLTLSKAD
YEKHKVYACEVTHQGLSSPVTKSFNRGEC

Alsevalimab comprises 2 identical heavy chains and 2 identical light chains, wherein the amino acid sequence of the heavy chain is set forth in SEQ ID NO. 16, and the amino acid sequence of the light chain is set forth in SEQ ID NO. 17.

> Alsevalimab heavy chain (SEQ ID NO. 16)

QLQLQESGPGLVKPSETLSLTCTVSGGSIKSGSYYWGWIRQPPGKGLEWIGNIYYSGSTYY
NPSLRSRVTISVDTSKNQFSLKLSSVTAADTAVYYCAREGSYPNQFDPWGQGTLVTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFS
CSVMHEALHNHYTQKSLSLSPGK

> Alsevalimab light chain (SEQ ID NO. 17)

EIVMTQSPATLSVSPGERATLSCRASQSVSSNLAWYQQKPGQAPRLLIYGASTRATGIPARF
SGSGSGTEFTLTISSLQSEDFAVYYCQQYHSFPFTFGGGTKVEIKRTVAAPSVFIFPPSDEQL
KSGTASVVCLLNNFYPREAKVQWKVDNALQSGNSQESVTEQDSKDSTYSLSSTLTLSKAD
YEKHKVYACEVTHQGLSSPVTKSFNRGEC

Tisotumab comprises 2 identical heavy chains and 2 identical light chains, wherein the amino acid sequence of the heavy chain is set forth in SEQ ID NO. 18, and the amino acid sequence of the light chain is set forth in SEQ ID NO. 19.

> Tisotumab heavy chain (SEQ ID NO. 18)

EVQLLESGGGLVQPGGSLRLSCAASGFTFSNYAMSWVRQAPGKGLEWVSSISGSGDYTYY
TDSVKGRFTISRDNSKNTLYLQMNSLRAEDTAVYYCARSPWGYYLDSWGQGTLVTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQGNV
FSCSVMHEALHNHYTQKSLSLSPGK

> Tisotumab light chain (SEQ ID NO. 19)

DIQMTQSPPSLSASAGDRVTITCRASQGISSRLAWYQQKPEKAPKSLIYAASSLQSGVPSRFS
GSGSGTDFTLTISSLQPEDFATYYCQQYNSYPYTFGQGTKLEIKRTVAAPSVFIFPPSDEQLK
SGTASVVCLLNNFYPREAKVQWKVDNALQSGNSQESVTEQDSKDSTYSLSSTLTLSKADY
EKHKVYACEVTHQGLSSPVTKSFNRGEC

L19A comprises 2 identical heavy chains and 2 identical light chains, wherein the amino acid sequence of the heavy chain is set forth in SEQ ID NO. 20, and the amino acid sequence of the light chain is set forth in SEQ ID NO. 21.

> L19A heavy chain (SEQ ID NO. 20)

EVQLLESGGGLVQPGGSLRLSCAASGFTFSSFSMSWVRQAPGKGLEWVSSISGSSGTTYYA
DSVKGRFTISRDNSKNTLYLQMNSLRAEDTAVYYCARPFPYFDYWGQGTLVTVSSASTKG
PSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLT
CLVKGFYPSDIAVEWESNGQPENNYKTTPPVLDSDGSFFLYSKLTVDKSRWQQGNVFSCS
VMHEALHNHYTQKSLSLSPG

> L19A light chain (SEQ ID NO. 21)

EIVLTQSPGTLSLSPGERATLSCRASQSVSSSFLAWYQQKPGQAPRLLIYYASSRATGIPDRF
SGSGSGTDFTLTISRLEPEDFAVYYCQQTGRIPPTFGQGTKVEIKRTVAAPSVFIFPPSDEQLK
SGTASVVCLLNNFYPREAKVQWKVDNALQSGNSQESVTEQDSKDSTYSLSSTLTLSKADY
EKHKVYACEVTHQGLSSPVTKSFNRGEC

Zolbetuximab comprises 2 identical heavy chains and 2 identical light chains, wherein the amino acid sequence of the heavy chain is set forth in SEQ ID NO. 22, and the amino acid sequence of the light chain is set forth in SEQ ID NO. 23.

> Zolbetuximab heavy chain (SEQ ID NO. 22)

QVQLQQPGAELVRPGASVKLSCKASGYTFTSYWINWVKQRPGQGLEWIGNIYPSDSYTNY
NQKFKDKATLTVDKSSSTAYMQLSSPTSEDSAVYYCTRSWRGNSFDYWGQGTTLTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQGNV
FSCSVMHEALHNHYTQKSLSLSPGK

> Zolbetuximab light chain (SEQ ID NO. 23)

DIVMTQSPSSLTVTAGEKVTMSCKSSQSLLNSGNQKNYLTWYQQKPGQPPKLLIYWASTR
ESGVPDRFTGSGSGTDFTLTISSVQAEDLAVYYCQNDYSYPFTFGSGTKLEIKRTVAAPSVFI
FPPSDEQLKSGTASVVCLLNNFYPREAKVQWKVDNALQSGNSQESVTEQDSKDSTYSLSST
LTLSKADYEKHKVYACEVTHQGLSSPVTKSFNRGEC

The amino acid sequences of the variable regions of the present disclosure are all determined according to the Kabat numbering scheme.

In some embodiments, -L- is selected from $-L_1-L_2-L_3-L_4-L_5-$, wherein $L_1$ is a covalent linker unit covalently linked to A, $L_2$ is an extension unit, $L_3$ is selected from a bond and an amino acid residue optionally substituted with a polar hydrophilic group, $L_4$ is selected from a peptide residue consisting of 2-8 amino acids, and $L_5$ is a bond, a self-immolative fragment, or a self-immolative fragment substituted with a polar hydrophilic group.

In some embodiments, -L- is selected from $-L_1-L_2-L_3-L_4-L_5-$, wherein $L_1$ is a covalent linker unit covalently linked to A, $L_2$ is an extension unit, $L_3$ is selected from an amino acid residue optionally substituted with a polar hydrophilic group, $L_4$ is selected from a peptide residue consisting of 2-8 amino acids, and $L_5$ is a bond or a self-immolative fragment.

In some embodiments, $L_1$ is selected from the following groups:

| No. | $L_1$-1 | $L_1$-2 | $L_1$-3 | $L_1$-4 | $L_1$-5 |
|---|---|---|---|---|---|
| Structure | 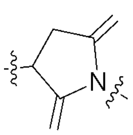 | 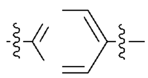 | 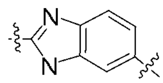 | 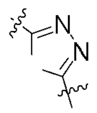 | 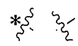 |
| wherein * indicates linkage to A. | | | | | |

In some embodiments, $L_2$ is selected from $-L_{2a}-$, $-L_{2a}-C(O)-$, $-C(O)-L_{2a}-C(O)-NH-L_{2b}-C(O)-$, and $-L_{2a}-NH-C(O)-L_{2b}-C(O)-$, wherein $L_{2a}$ and $L_{2b}$ are each independently selected from $-C_1-C_8$ alkylene-, $-C_1-C_8$ alkylene-$C_3-C_8$ cycloalkylene-, -alkynylene-$C_1-C_6$ alkylene-, -phenylene-, -phenylene-C1-3 alkylene-, and linear or branched heteroalkylene having 1-50 (preferably 9-30, and more preferably 9-26, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, wherein the alkylene, cycloalkylene, phenylene, and heteroalkylene are each optionally substituted with one or more substituents independently selected from $C_1-C_6$ alkyl, heteroalkyl having 1-6 atoms, $C_1-C_6$ alkoxy, hydroxyl, amino, carboxyl, and $C_3-C_8$ cycloalkyl, and the heteroalkylene contains 1-12 (preferably 1-8, and more preferably 3-8, e.g., 1, 2, 3, 4, 5, 6, 7, 8, 9, 10, 11, or 12) heteroatoms selected from one or more (two or three) of N, O, and S (preferably O).

In some embodiments, $L_2$ is selected from $-L_{2a}-$, $-L_{2a}-C(O)-$, $-C(O)-L_{2a}-C(O)-NH-L_{2b}-C(O)-$, and $-L_{2a}-NH-C(O)-L_{2b}-C(O)-$, wherein $L_{2a}$ and $L_{2b}$ are each independently selected from $-C_1-C_8$ alkylene-, $-C_1-C_8$ alkylene-$C_3-C_8$ cycloalkylene-, -alkynylene-$C_1-C_6$ alkylene-, and linear or branched heteroalkylene having 1-50 (preferably 9-30, and more preferably 9-26, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, wherein the alkylene, cycloalkylene, and heteroalkylene are each optionally substituted with one or more substituents independently selected from $C_1-C_6$ alkyl, heteroalkyl having 1-6 atoms, $C_1-C_6$ alkoxy, hydroxyl, amino, carboxyl, and $C_3-C_8$ cycloalkyl, and the heteroalkylene contains 1-12 (preferably 1-8, and more preferably 3-8, e.g., 1, 2, 3, 4, 5, 6, 7, 8, 9, 10, 11, or 12) heteroatoms selected from one or more (two or three) of N, O, and S (preferably O).

In some embodiments, $L_3$ is selected from an amino acid residue substituted with a polar hydrophilic group, wherein the polar hydrophilic group comprises a saccharide residue and a derivative thereof, a polyethylene glycol residue and a derivative thereof, a polysarcosine residue and a derivative thereof, or a combination thereof.

In some embodiments, $L_4$ is selected from a peptide residue consisting of 2-8 amino acids selected from phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine.

In some embodiments, $L_5$ is selected from a bond,

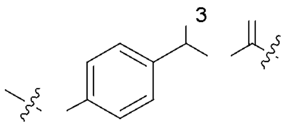, and 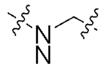,

wherein $R_3$ is selected from hydrogen and

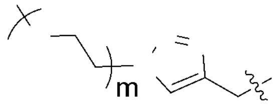,

wherein m is an integer selected from 3-50, preferably an integer selected from 8-24, and further preferably 8, 9, 10, 11, 12, 13, 14, 15, 16, 17, 18, 19, 20, 21, 22, 23, or 24; * indicates linkage to D.

In some embodiments, $L_5$ is selected from a bond,

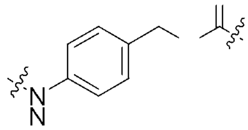, and 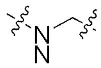,

wherein * indicates linkage to D.

In some embodiments, $L_5$ is selected from:

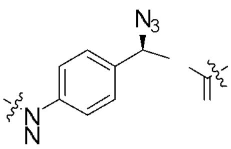 and 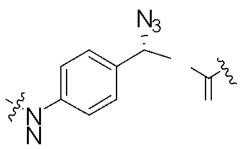.

In some embodiments, $L_2$ is selected from $-L_{2a}-$, $-L_{2a}-C(O)-$, $-C(O)-L_{2a}-C(O)-NH-L_{2b}-C(O)-$, and $-L_{2a}-NH-C(O)-L_{2b}-C(O)-$, wherein $L_{2a}$ and $L_{2b}$ are each independently selected from $-C_1-C_6$ alkylene-, $-C_1-C_3$ alkylene-$C_3-C_6$ cycloalkylene-, -ethynylene-$C_1-C_6$ alkylene-, $-(CH_2CH_2O)_s-$, $-(CH_2CH_2O)_sC_{1-3}$ alkylene-, and $-C_{1-3}$ alkylene$(CH_2CH_2O)_sC_{1-3}$ alkylene-, wherein s is an integer selected from 1-12 (preferably 3-8, e.g., 1, 2, 3, 4, 5, 6, 7, 8, 9, 10, 11, or 12).

In some embodiments, $L_2$ is selected from $-L_{2a}-$, $-L_{2a}-C(O)-$, $-C(O)-L_{2a}-C(O)-NH-L_{2b}-C(O)-$, and $-L_{2a}-NH-C(O)-L_{2b}-C(O)-$, wherein $L_{2a}$ and $L_{2b}$ are each independently selected from methylene, ethylene, *n*-propylene, isopropylene, *n*-pentylene, methylenecyclohexylene, ethynylenemethylene, ethynyleneethylene, ethynylenen-*n*-propylene, ethynylenen-*n*-butylene, ethynylenen-*n*-pentylene, ethynylenen-n-hexylene, phenylene, phenylenemethylene, $-(CH_2CH_2O)_sCH_2-$, $-(CH_2CH_2O)_sCH_2CH_2-$, $-CH_2(OCH_2CH_2)_s-$, $-CH_2CH_2(OCH_2CH_2)_s-$, $-CH_2CH_2(OCH_2CH_2)_sCH_2-$, $-CH_2CH_2(OCH_2CH_2)_sCH_2CH_2-$, and $-CH_2(OCH_2CH_2)_sCH_2-$, wherein s is an integer selected from 1-12 (preferably 3-8, e.g., 1, 2, 3, 4, 5, 6, 7, 8, 9, 10, 11, or 12).

In some embodiments, $L_2$ is selected from $-L_{2a}-$, $-L_{2a}-C(O)-$, $-C(O)-L_{2a}-C(O)-NH-L_{2b}-C(O)-$, and $-L_{2a}-NH-C(O)-L_{2b}-C(O)-$, wherein $L_{2a}$ and $L_{2b}$ are each independently selected from methylene, ethylene, n-propylene, isopropylene, n-pentylene, methylenecyclohexylene, ethynylenemethylene, ethynyleneethylene, ethynylenen-n-propylene, ethynylenen-n-butylene, ethynylenen-n-pentylene, ethynylenen-n-hexylene, $-(CH_2CH_2O)_sCH_2-$, $-(CH_2CH_2O)_sCH_2CH_2-$, $-CH_2(OCH_2CH_2)_s-$, $-CH_2CH_2(OCH_2CH_2)_s-$, $-CH_2CH_2(OCH_2CH_2)_sCH_2-$, $-CH_2CH_2(OCH_2CH_2)_sCH_2CH_2-$, and $-CH_2(OCH_2CH_2)_sCH_2-$, wherein s is an integer selected from 1-12 (preferably 3-8, e.g., 1, 2, 3, 4, 5, 6, 7, 8, 9, 10, 11, or 12).

In some embodiments, $L_2$ is selected from:

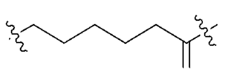, 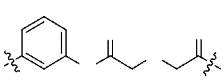,

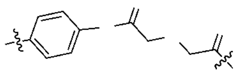 , 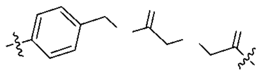 , 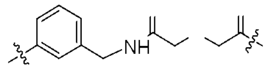 ,

$-(CH_2CH_2O)_sCH_2C(O)-$, $-(CH_2CH_2O)_sCH_2CH_2-$,

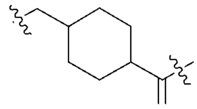 , and 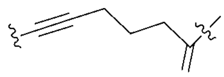 ,

wherein s is as defined above.

In some embodiments, $L_2$ is selected from:

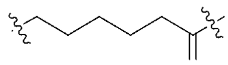 ,

$-(CH_2CH_2O)_sCH_2C(O)-$, $-(CH_2CH_2O)_sCH_2CH_2-$,

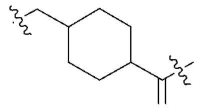 , and 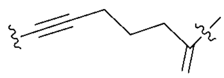 ,

wherein s is as defined above.

In some embodiments, $L_2$ is selected from:

| No. | $L_2$-1 | $L_2$-2 | $L_2$-3 |
|---|---|---|---|
| Structure | 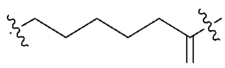 | 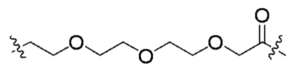 | 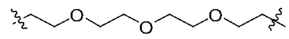 |
| No. | $L_2$-4 | $L_2$-5 | $L_2$-6 |
| Structure | 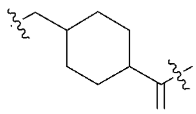 | 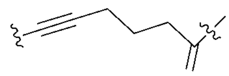 | 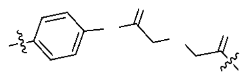 |
| No. | $L_2$-7 | $L_2$-8 | $L_2$-9 |
| Structure | 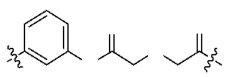 | 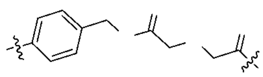 | 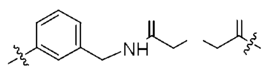 |
| wherein * indicates linkage to $L_1$. | | | |

In some embodiments, $L_3$ is selected from a bond, glutamic acid, and glutamic acid substituted with a polar hydrophilic group, wherein the polar hydrophilic group comprises a saccharide residue and a derivative thereof, a polyethylene glycol residue and a derivative thereof, a polysarcosine residue and a derivative thereof, or a combination thereof.

In some embodiments, $L_3$ is selected from glutamic acid substituted with a polar hydrophilic group, wherein the polar hydrophilic group comprises a saccharide residue and a derivative thereof, a polyethylene glycol residue and a derivative thereof, a polysarcosine residue and a derivative thereof, or a combination thereof.

In some embodiments, $L_3$ is selected from:

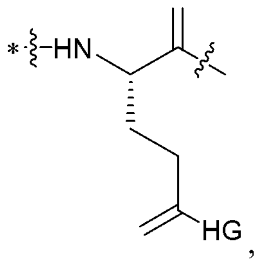,

wherein HG is a polar hydrophilic group, preferably selected from a group comprising a saccharide residue and a derivative thereof, a group comprising a polyethylene glycol residue and a derivative thereof, a group comprising a polysarcosine residue and a derivative thereof, and a combination thereof; * indicates linkage to $L_2$. In some embodiments, HG is selected from: $-NH(CH_2CH_2O)nCH_3$,

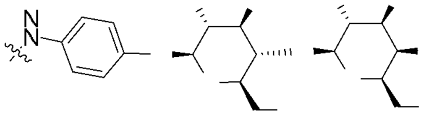, 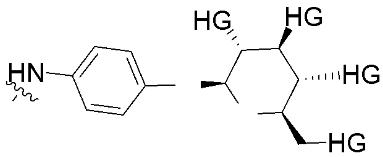, 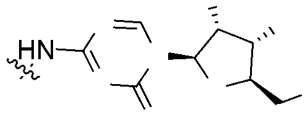,

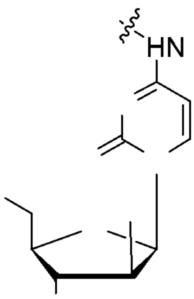, 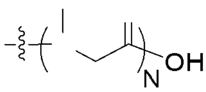, and 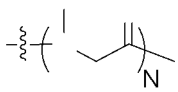,

wherein n and q are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50), preferably 1-24, and further preferably 3-12.

In some embodiments, $L_3$ is selected from:

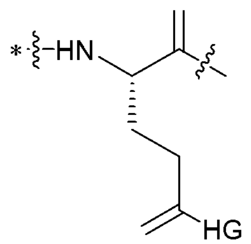

| | | |
|---|---|---|
| No. | $L_3$-1 | $L_3$-2 |
| HG structure | HG-1: $-NH(CH_2CH_2O)nCH_3$ | HG-2: 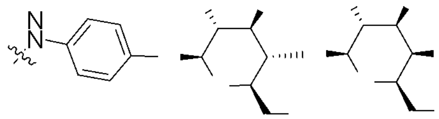 |
| No. | $L_3$-3 | $L_3$-4 |
| HG structure | HG-3: | HG-4: |

(continued)

| No. | $L_3$-3 | $L_3$-4 |
|---|---|---|
| | 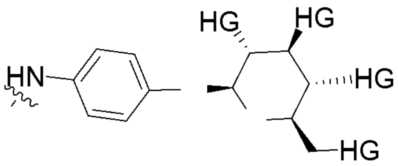 | 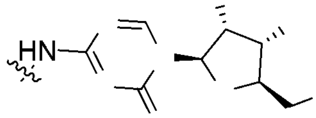 |
| No. | $L_3$-5 | $L_3$-6 |
| HG structure | HG-5: 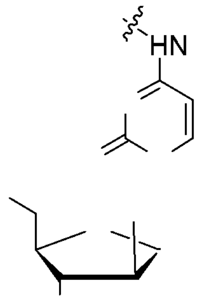 | HG-6: 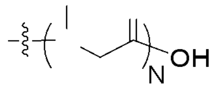 |
| No. | $L_3$-7 | |
| HG structure | HG-7: 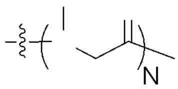 | |

wherein n and q are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50), preferably 1-24, and further preferably 3-12.

In some embodiments, $L_4$ is selected from:

| No. | $L_4$-1 | $L_4$-2 |
|---|---|---|
| Structure | 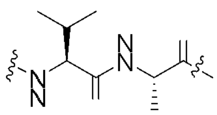 (VA) | 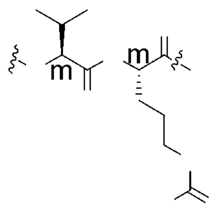 (VC) |
| No. | $L_4$-3 | $L_4$-4 |
| Structure | 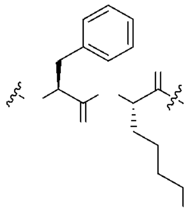 (FK) | 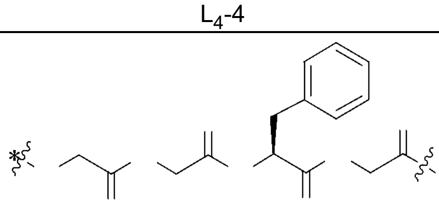 (GGFG) |
| wherein * indicates linkage to $L_3$. | | |

In some embodiments, $-L_1-L_2-$ is selected from:

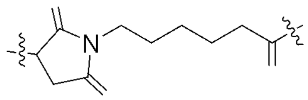

,

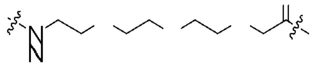

,

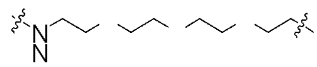

,

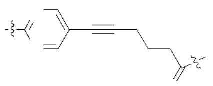

,

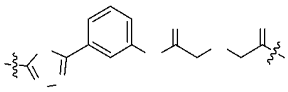

,

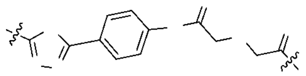

,

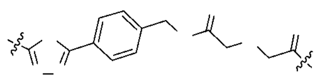

,

and

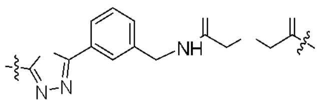

,

wherein * indicates linkage to A.

In some embodiments, $-L_1-L_2-$ is selected from:

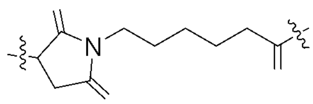

,

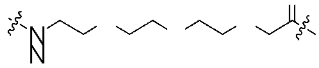

,

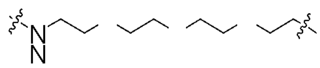

,

and

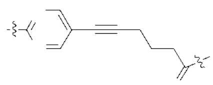

,

wherein * indicates linkage to A.

In some embodiments, $-L_3-L_4-$ is selected from:

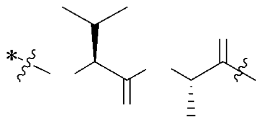

,

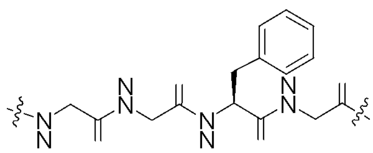

,

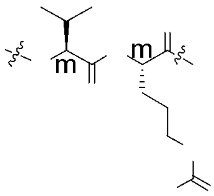

,

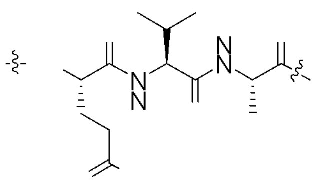

,

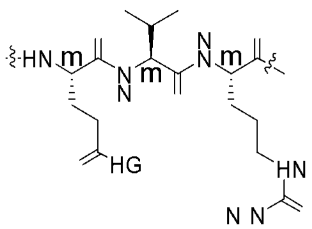

,

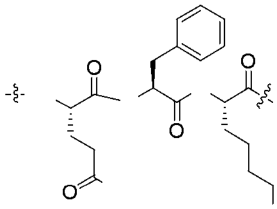

,

and

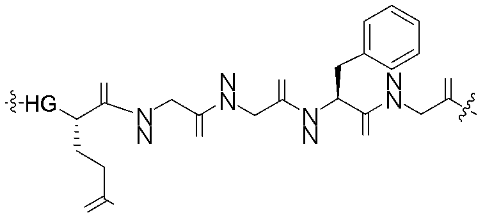

,

wherein HG is as defined above, and * indicates linkage to $L_2$.

In some embodiments, $-L_3-L_4-$ is selected from:

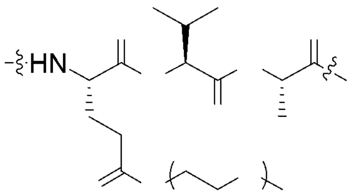

,

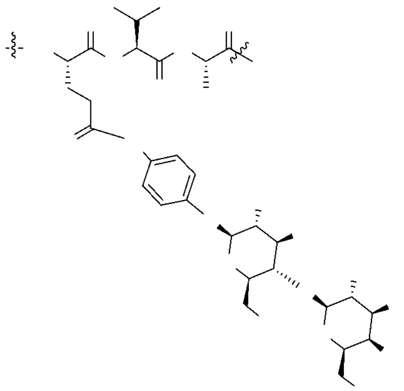

,

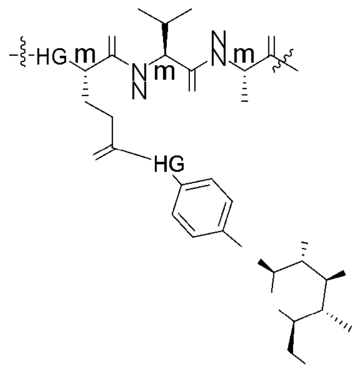

,

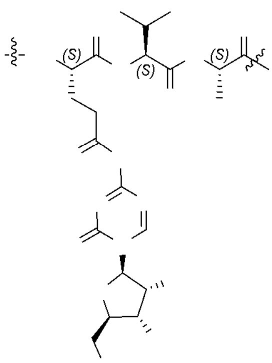

,

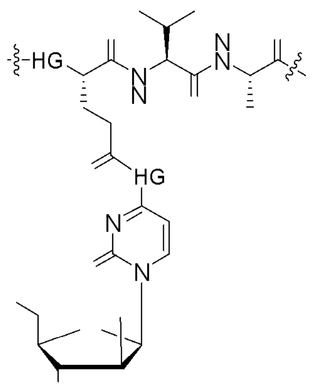

,

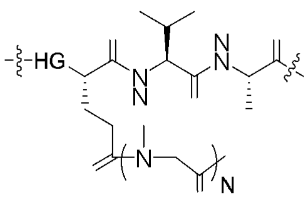

,

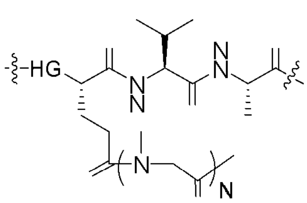

,

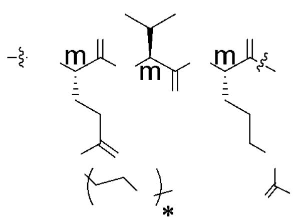

,

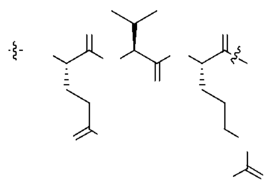

,

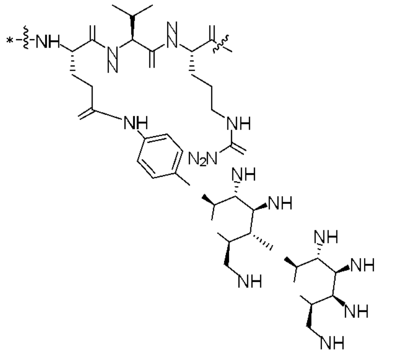
,
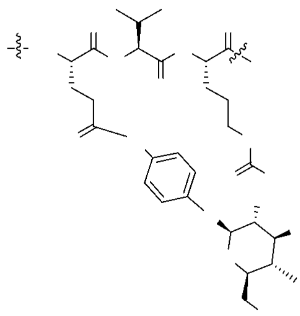
,
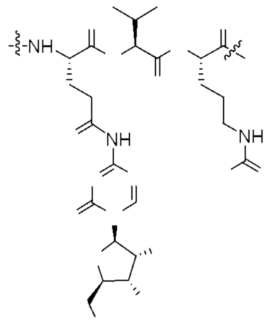
,
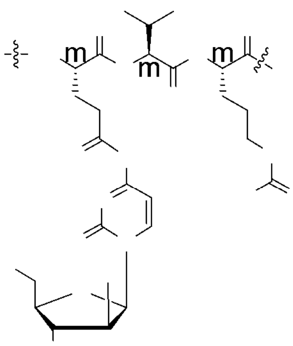
,
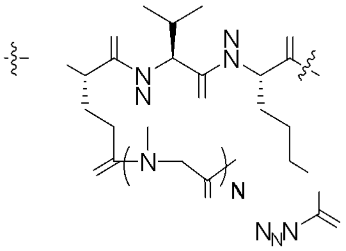
, and
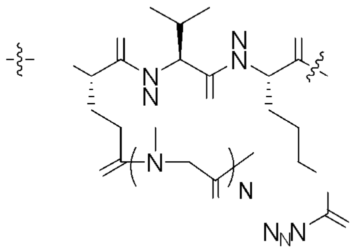
,

wherein * indicates linkage to $L_2$, and n and q are as defined above.

In some embodiments, -L- is selected from:

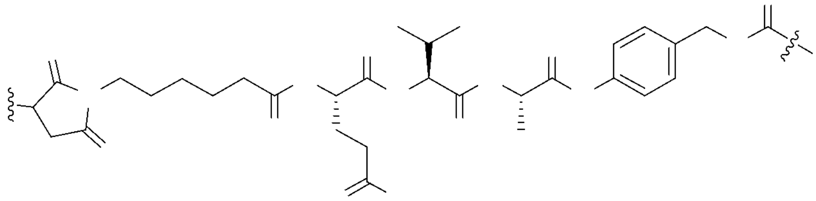

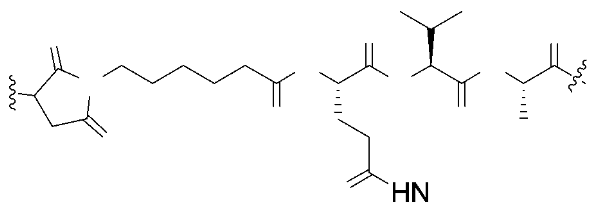

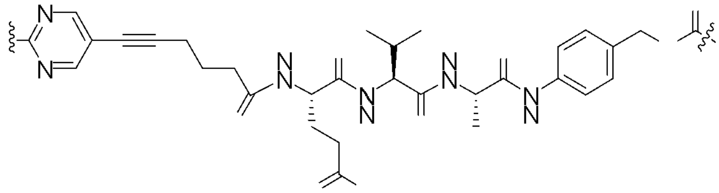

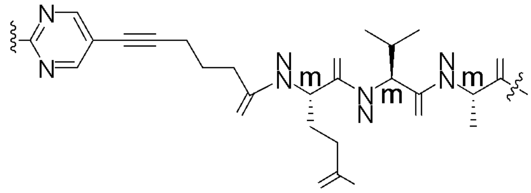

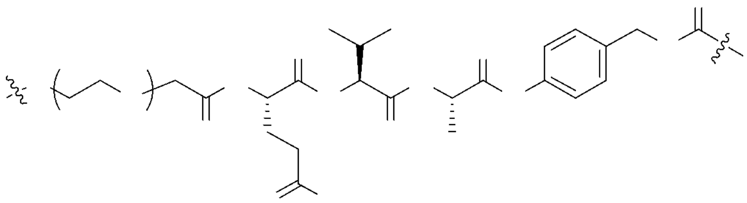

and

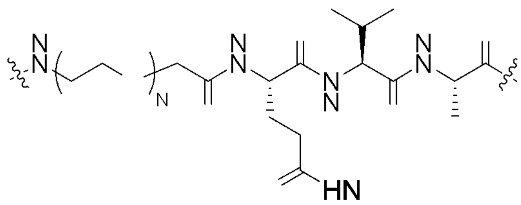

wherein HG and s are as defined above, and * indicates linkage to A.

In some embodiments, -L- is selected from:

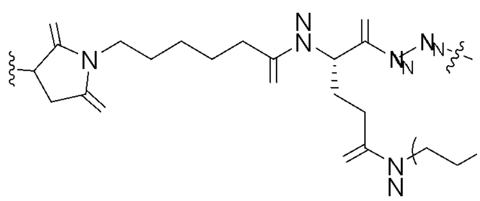
,
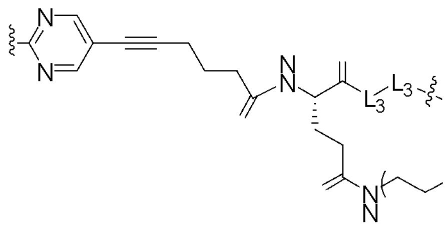
,

and

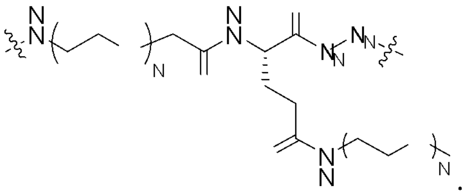

In some embodiments, -L- is selected from:

| 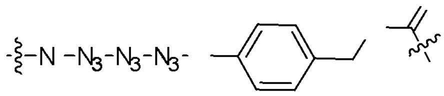 | | | | |
|---|---|---|---|---|
| No. | $L_1$ | $L_2$ | $L_3$ | $L_4$ |
| L-1 | $L_1$-1 | $L_2$-1 | $L_3$-1 | $L_4$-1 |
| L-2 | $L_1$-1 | $L_2$-1 | $L_3$-2 | $L_4$-1 |
| L-3 | $L_1$-1 | $L_2$-1 | $L_3$-3 | $L_4$-1 |
| L-4 | $L_1$-1 | $L_2$-1 | $L_3$-4 | $L_4$-1 |
| L-5 | $L_1$-1 | $L_2$-1 | $L_3$-5 | $L_4$-1 |
| L-6 | $L_1$-1 | $L_2$-1 | $L_3$-6 | $L_4$-1 |
| L-7 | $L_1$-1 | $L_2$-1 | $L_3$-7 | $L_4$-1 |
| L-8 | $L_1$-1 | $L_2$-1 | $L_3$-1 | $L_4$-2 |
| L-9 | $L_1$-1 | $L_2$-1 | $L_3$-2 | $L_4$-2 |

(continued)

| 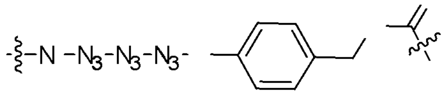 | | | | |
|---|---|---|---|---|
| No. | $L_1$ | $L_2$ | $L_3$ | $L_4$ |
| L-10 | $L_1$-1 | $L_2$-1 | $L_3$-3 | $L_4$-2 |
| L-11 | $L_1$-1 | $L_2$-1 | $L_3$-4 | $L_4$-2 |
| L-12 | $L_1$-1 | $L_2$-1 | $L_3$-5 | $L_4$-2 |
| L-13 | $L_1$-1 | $L_2$-1 | $L_3$-6 | $L_4$-2 |
| L-14 | $L_1$-1 | $L_2$-1 | $L_3$-7 | $L_4$-2 |
| L-15 | $L_1$-2 | $L_2$-5 | $L_3$-1 | $L_4$-1 |
| L-16 | $L_1$-2 | $L_2$-5 | $L_3$-2 | $L_4$-1 |
| L-17 | $L_1$-2 | $L_2$-5 | $L_3$-3 | $L_4$-1 |
| L-18 | $L_1$-2 | $L_2$-5 | $L_3$-4 | $L_4$-1 |
| L-19 | $L_1$-2 | $L_2$-5 | $L_3$-5 | $L_4$-1 |
| L-20 | $L_1$-2 | $L_2$-5 | $L_3$-6 | $L_4$-1 |
| L-21 | $L_1$-2 | $L_2$-5 | $L_3$-7 | $L_4$-1 |
| L-22 | $L_1$-2 | $L_2$-5 | $L_3$-1 | $L_4$-2 |
| L-23 | $L_1$-2 | $L_2$-5 | $L_3$-2 | $L_4$-2 |
| L-24 | $L_1$-2 | $L_2$-5 | $L_3$-3 | $L_4$-2 |
| L-25 | $L_1$-2 | $L_2$-5 | $L_3$-4 | $L_4$-2 |
| L-26 | $L_1$-2 | $L_2$-5 | $L_3$-5 | $L_4$-2 |
| L-27 | $L_1$-2 | $L_2$-5 | $L_3$-6 | $L_4$-2 |
| L-28 | $L_1$-2 | $L_2$-5 | $L_3$-7 | $L_4$-2 |
| L-29 | $L_1$-5 | $L_2$-2 | $L_3$-1 | $L_4$-1 |
| L-30 | $L_1$-5 | $L_2$-2 | $L_3$-2 | $L_4$-1 |
| L-31 | $L_1$-5 | $L_2$-2 | $L_3$-3 | $L_4$-1 |
| L-32 | $L_1$-5 | $L_2$-2 | $L_3$-4 | $L_4$-1 |
| L-33 | $L_1$-5 | $L_2$-2 | $L_3$-5 | $L_4$-1 |
| L-34 | $L_1$-5 | $L_2$-2 | $L_3$-6 | $L_4$-1 |
| L-35 | $L_1$-5 | $L_2$-2 | $L_3$-7 | $L_4$-1 |
| L-36 | $L_1$-5 | $L_2$-2 | $L_3$-1 | $L_4$-2 |
| L-37 | $L_1$-5 | $L_2$-2 | $L_3$-2 | $L_4$-2 |
| L-38 | $L_1$-5 | $L_2$-2 | $L_3$-3 | $L_4$-2 |
| L-39 | $L_1$-5 | $L_2$-2 | $L_3$-4 | $L_4$-2 |
| L-40 | $L_1$-5 | $L_2$-2 | $L_3$-5 | $L_4$-2 |
| L-41 | $L_1$-5 | $L_2$-2 | $L_3$-6 | $L_4$-2 |
| L-42 | $L_1$-5 | $L_2$-2 | $L_3$-7 | $L_4$-2 |
| L-43 | $L_1$-1 | $L_2$-1 | $L_3$-1 | $L_4$-3 |
| L-44 | $L_1$-1 | $L_2$-1 | $L_3$-1 | $L_4$-4 |
| L-45 | $L_1$-2 | $L_2$-5 | $L_3$-1 | $L_4$-3 |
| L-46 | $L_1$-2 | $L_2$-5 | $L_3$-1 | $L_4$-4 |

(continued)

| 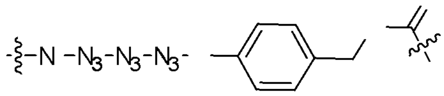 | | | | |
|---|---|---|---|---|
| No. | $L_1$ | $L_2$ | $L_3$ | $L_4$ |
| L-47 | $L_1$-5 | $L_2$-2 | $L_3$-1 | $L_4$-3 |
| L-48 | $L_1$-5 | $L_2$-2 | $L_3$-1 | $L_4$-4 |

In some embodiments of the present disclosure, -L- is selected from:

| -$L_1$-$L_2$-$L_3$-$L_4$- | | | | |
|---|---|---|---|---|
| No. | $L_1$ | $L_2$ | $L_3$ | $L_4$ |
| L-49 | $L_1$-1 | $L_2$-1 | $L_3$-1 | $L_4$-1 |
| L-50 | $L_1$-1 | $L_2$-1 | $L_3$-2 | $L_4$-1 |
| L-51 | $L_1$-1 | $L_2$-1 | $L_3$-3 | $L_4$-1 |
| L-52 | $L_1$-1 | $L_2$-1 | $L_3$-4 | $L_4$-1 |
| L-53 | $L_1$-1 | $L_2$-1 | $L_3$-5 | $L_4$-1 |
| L-54 | $L_1$-1 | $L_2$-1 | $L_3$-6 | $L_4$-1 |
| L-55 | $L_1$-1 | $L_2$-1 | $L_3$-7 | $L_4$-1 |
| L-56 | $L_1$-1 | $L_2$-1 | $L_3$-1 | $L_4$-2 |
| L-57 | $L_1$-1 | $L_2$-1 | $L_3$-2 | $L_4$-2 |
| L-58 | $L_1$-1 | $L_2$-1 | $L_3$-3 | $L_4$-2 |
| L-59 | $L_1$-1 | $L_2$-1 | $L_3$-4 | $L_4$-2 |
| L-60 | $L_1$-1 | $L_2$-1 | $L_3$-5 | $L_4$-2 |
| L-61 | $L_1$-1 | $L_2$-1 | $L_3$-6 | $L_4$-2 |
| L-62 | $L_1$-1 | $L_2$-1 | $L_3$-7 | $L_4$-2 |
| L-63 | $L_1$-2 | $L_2$-5 | $L_3$-1 | $L_4$-1 |
| L-64 | $L_1$-2 | $L_2$-5 | $L_3$-2 | $L_4$-1 |
| L-65 | $L_1$-2 | $L_2$-5 | $L_3$-3 | $L_4$-1 |
| L-66 | $L_1$-2 | $L_2$-5 | $L_3$-4 | $L_4$-1 |
| L-67 | $L_1$-2 | $L_2$-5 | $L_3$-5 | $L_4$-1 |
| L-68 | $L_1$-2 | $L_2$-5 | $L_3$-6 | $L_4$-1 |
| L-69 | $L_1$-2 | $L_2$-5 | $L_3$-7 | $L_4$-1 |
| L-70 | $L_1$-2 | $L_2$-5 | $L_3$-1 | $L_4$-2 |
| L-71 | $L_1$-2 | $L_2$-5 | $L_3$-2 | $L_4$-2 |
| L-72 | $L_1$-2 | $L_2$-5 | $L_3$-3 | $L_4$-2 |
| L-73 | $L_1$-2 | $L_2$-5 | $L_3$-4 | $L_4$-2 |
| L-74 | $L_1$-2 | $L_2$-5 | $L_3$-5 | $L_4$-2 |
| L-75 | $L_1$-2 | $L_2$-5 | $L_3$-6 | $L_4$-2 |
| L-76 | $L_1$-2 | $L_2$-5 | $L_3$-7 | $L_4$-2 |
| L-77 | $L_1$-5 | $L_2$-2 | $L_3$-1 | $L_4$-1 |
| L-78 | $L_1$-5 | $L_2$-2 | $L_3$-2 | $L_4$-1 |

(continued)

| -$L_1$-$L_2$-$L_3$-$L_4$- | | | | |
|---|---|---|---|---|
| No. | $L_1$ | $L_2$ | $L_3$ | $L_4$ |
| L-79 | $L_1$-5 | $L_2$-2 | $L_3$-3 | $L_4$-1 |
| L-80 | $L_1$-5 | $L_2$-2 | $L_3$-4 | $L_4$-1 |
| L-81 | $L_1$-5 | $L_2$-2 | $L_3$-5 | $L_4$-1 |
| L-82 | $L_1$-5 | $L_2$-2 | $L_3$-6 | $L_4$-1 |
| L-83 | $L_1$-5 | $L_2$-2 | $L_3$-7 | $L_4$-1 |
| L-84 | $L_1$-5 | $L_2$-2 | $L_3$-1 | $L_4$-2 |
| L-85 | $L_1$-5 | $L_2$-2 | $L_3$-2 | $L_4$-2 |
| L-86 | $L_1$-5 | $L_2$-2 | $L_3$-3 | $L_4$-2 |
| L-87 | $L_1$-5 | $L_2$-2 | $L_3$-4 | $L_4$-2 |
| L-88 | $L_1$-5 | $L_2$-2 | $L_3$-5 | $L_4$-2 |
| L-89 | $L_1$-5 | $L_2$-2 | $L_3$-6 | $L_4$-2 |
| L-90 | $L_1$-5 | $L_2$-2 | $L_3$-7 | $L_4$-2 |
| L-91 | $L_1$-1 | $L_2$-1 | $L_3$-1 | $L_4$-3 |
| L-92 | $L_1$-1 | $L_2$-1 | $L_3$-1 | $L_4$-4 |
| L-93 | $L_1$-2 | $L_2$-5 | $L_3$-1 | $L_4$-3 |
| L-94 | $L_1$-2 | $L_2$-5 | $L_3$-1 | $L_4$-4 |
| L-95 | $L_1$-5 | $L_2$-2 | $L_3$-1 | $L_4$-3 |
| L-96 | $L_1$-5 | $L_2$-2 | $L_3$-1 | $L_4$-4 |

In some embodiments of the present disclosure, -L- is selected from:

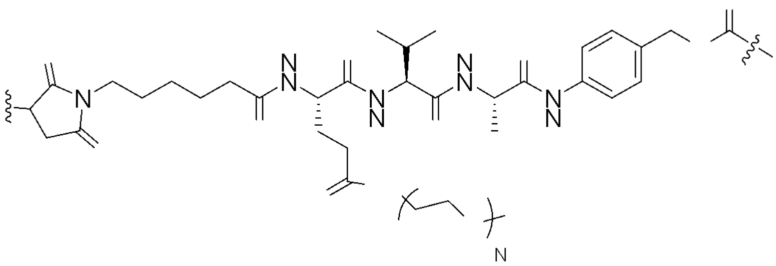

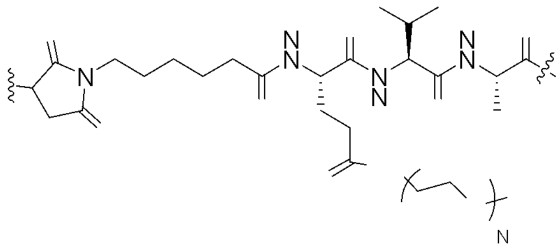

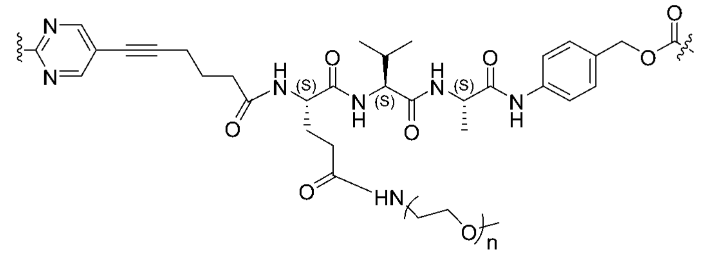

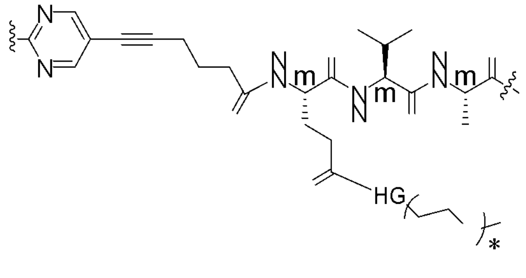

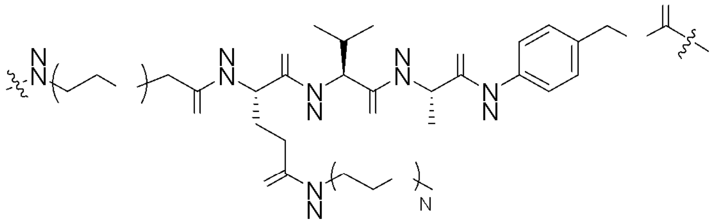

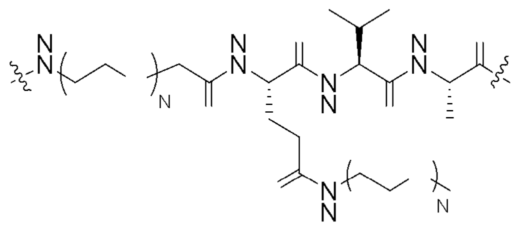

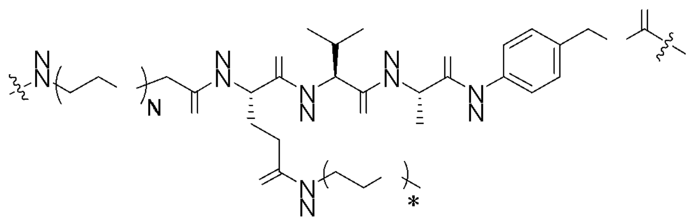

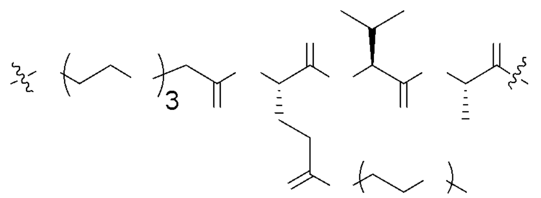

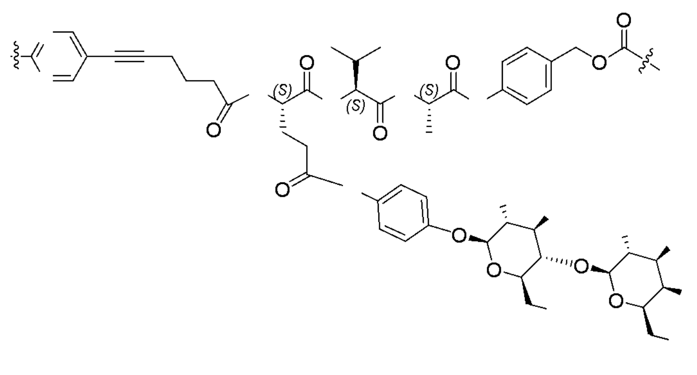

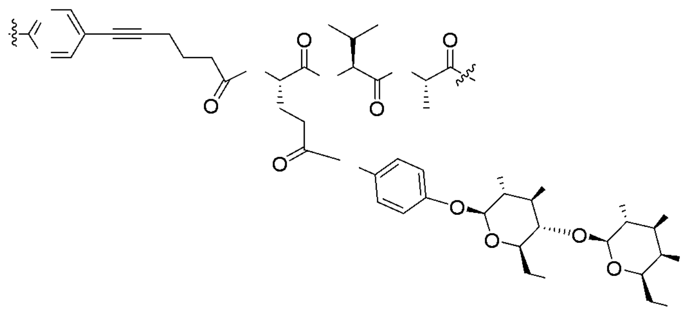

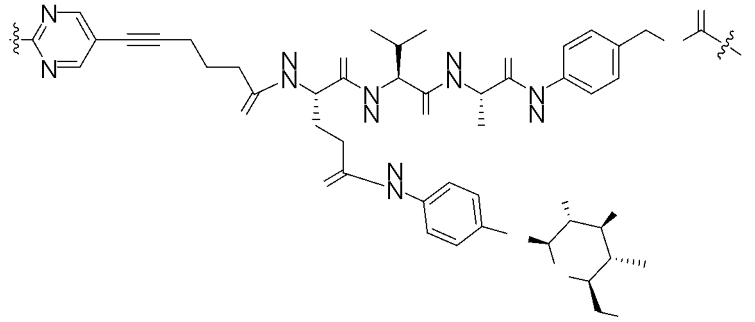

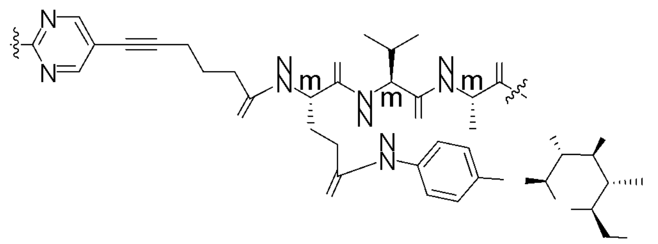

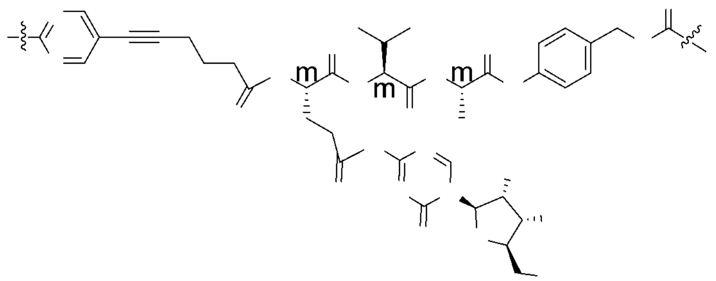

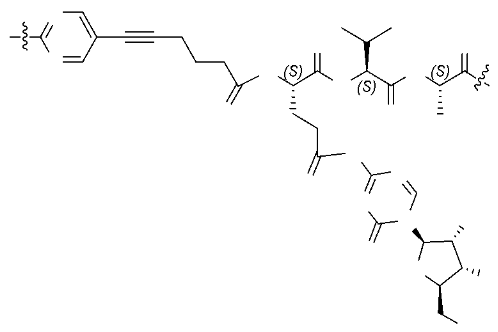

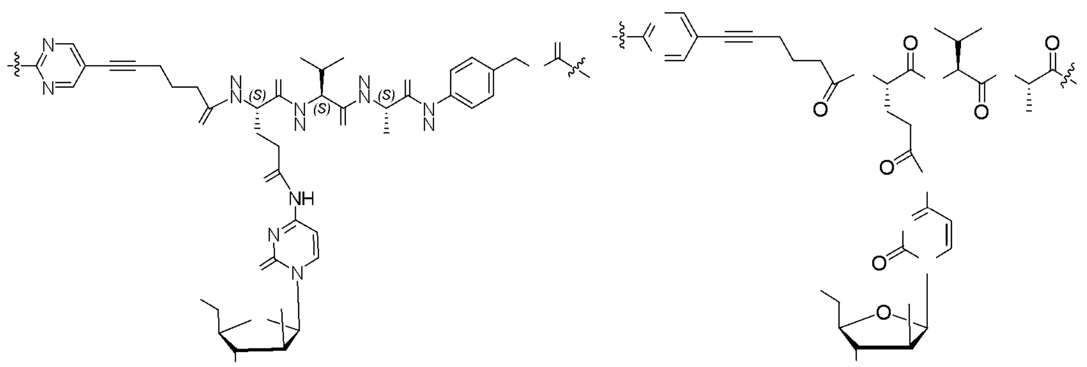

wherein n and s are as defined above.

In some embodiments, -L- is selected from -$L_1$-$L_2$-$L_4$-$L_5$-, wherein $L_1$, $L_2$, $L_4$, and $L_5$ are as defined above.

In some embodiments, -L- is selected from:

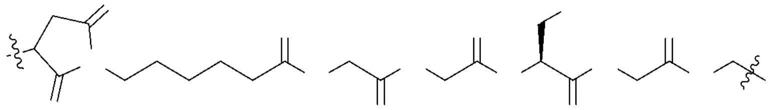

In some embodiments, -L- is selected from:

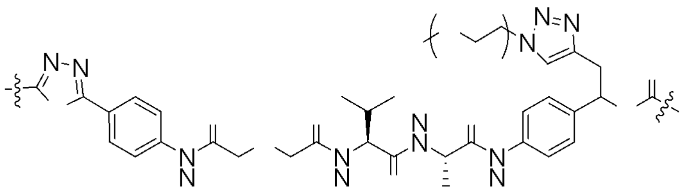

,

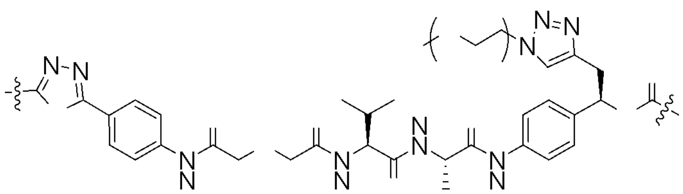

,

and

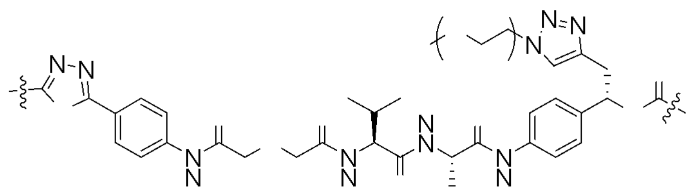

wherein m is an integer selected from 3-50, preferably an integer selected from 8-24, and further preferably 8, 9, 10, 11, 12, 13, 14, 15, 16, 17, 18, 19, 20, 21, 22, 23, or 24.

In some embodiments, D is selected from a topoisomerase I inhibitor, a tubulin inhibitor, and a DNA damaging agent.

In some embodiments, D is selected from a camptothecin derivative.

In some embodiments, D is selected from

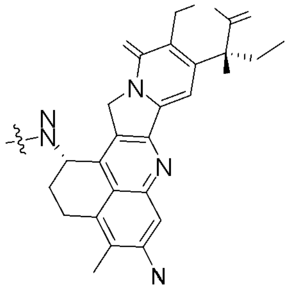 and 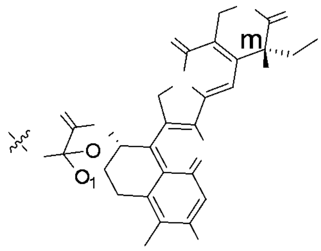,

wherein $R_1$ and $R_2$ are each independently selected from H, $C_{1-3}$ alkyl (preferably methyl, ethyl, n-propyl, or isopropyl), and 3- to 6-membered cycloalkyl (preferably cyclopropyl, cyclobutyl, cyclopentyl, or cyclohexyl); or $R_1$ and $R_2$, together with the carbon atom attached thereto, form 3-to 6-membered cycloalkyl (preferably cyclopropyl, cyclobutyl, cyclopentyl, or cyclohexyl).

In some embodiments, D is selected from MMAE, MMAF, eribulin, pyrrolobenzodiazepine,

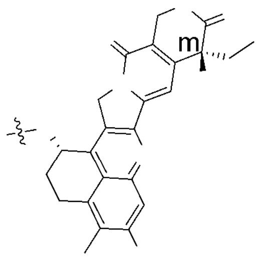, 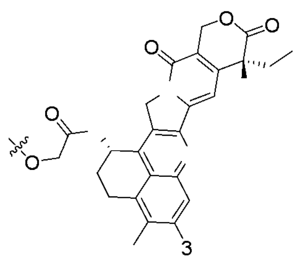, 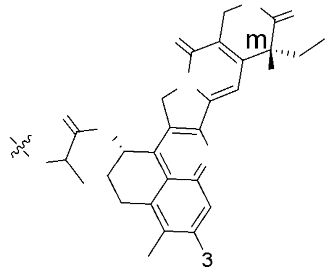,

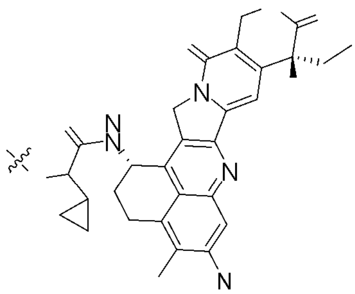, 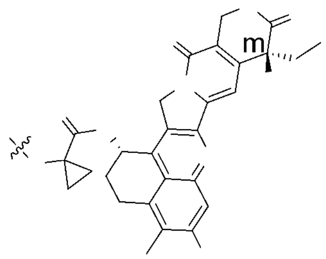, 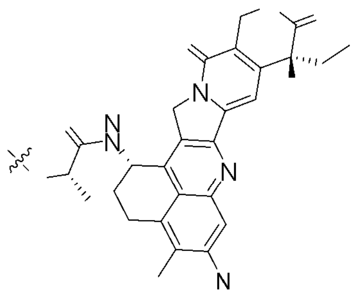,

and

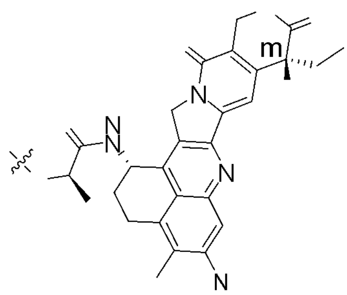.

In some embodiments, D is selected from:

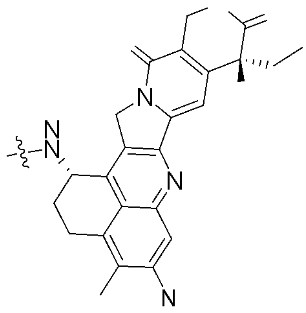

.

In some embodiments, the antibody-drug conjugate of formula (I) has the following structure:

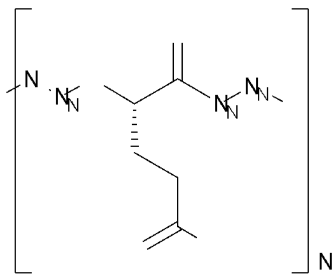

.

In some embodiments, the antibody-drug conjugate of formula (I) has the following structure:

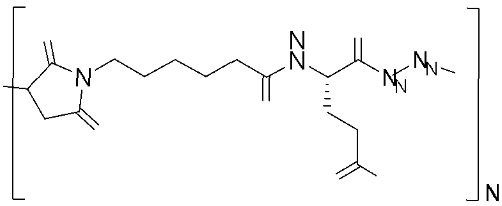

,

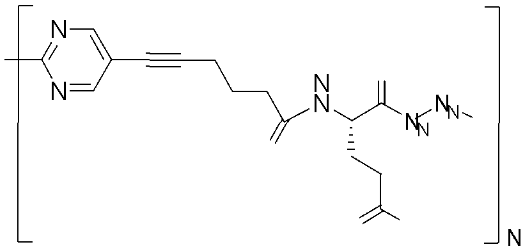

,

or

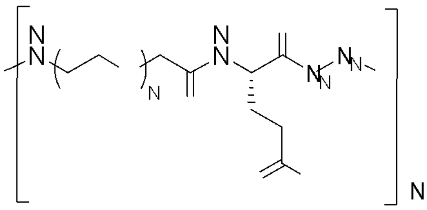

wherein d and s are as defined above.

In some embodiments, the antibody-drug conjugate of formula (I) has the following structure:

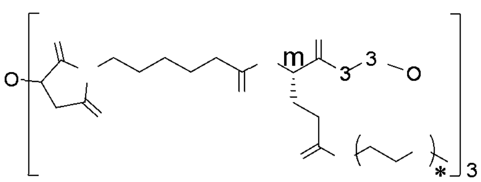

,

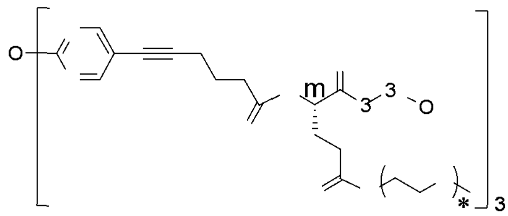

,

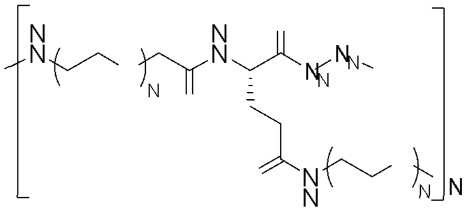, or 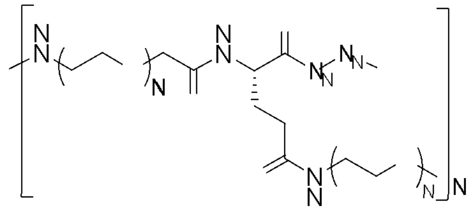,

wherein n, d, and s are as defined above.

In some embodiments, the antibody-drug conjugate of formula (I) has the following structure:

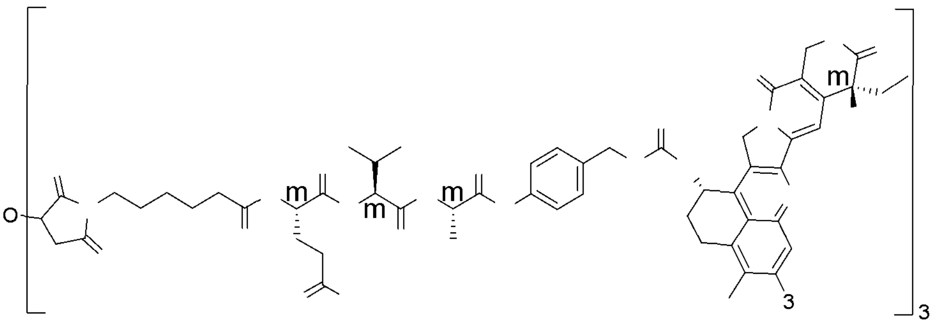

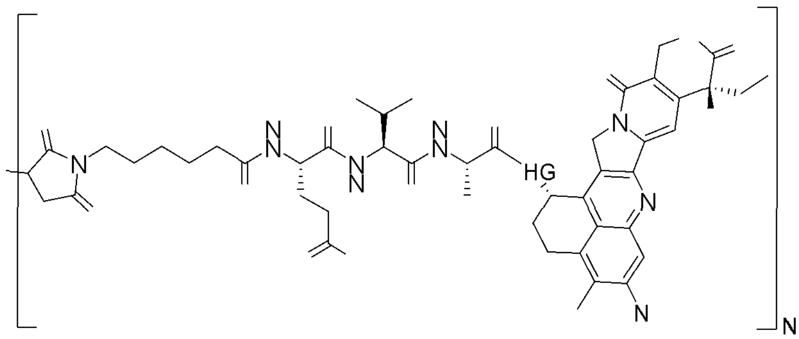

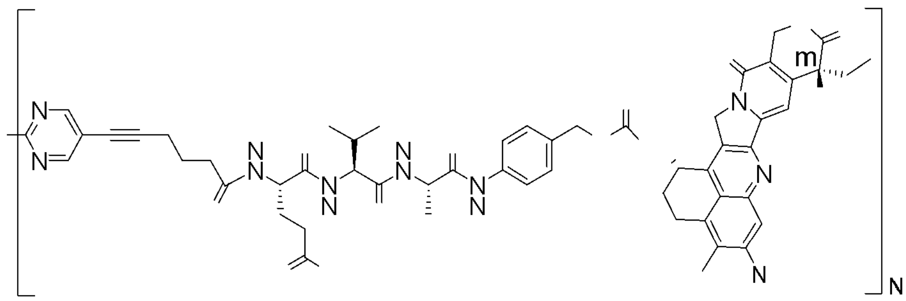

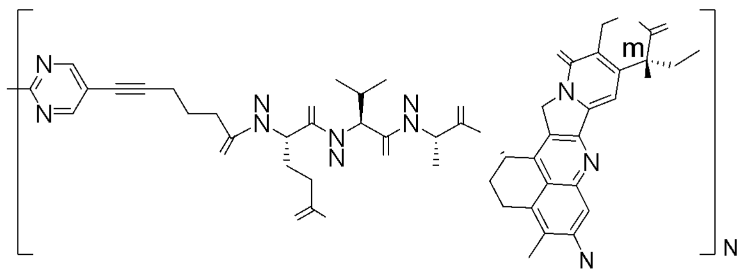

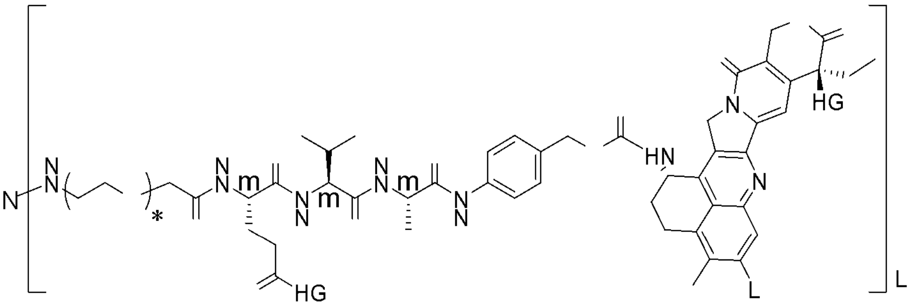

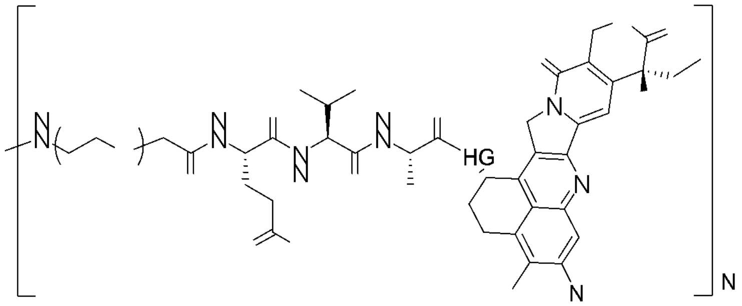

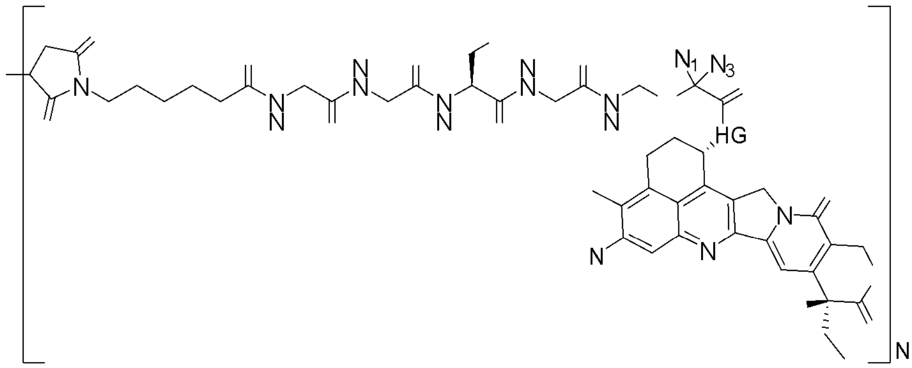

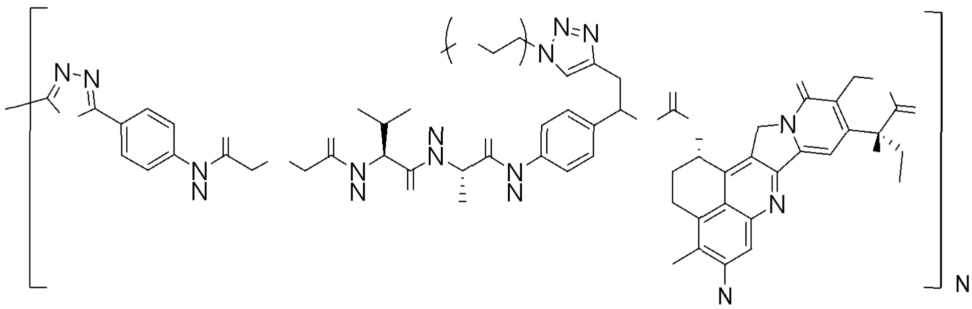

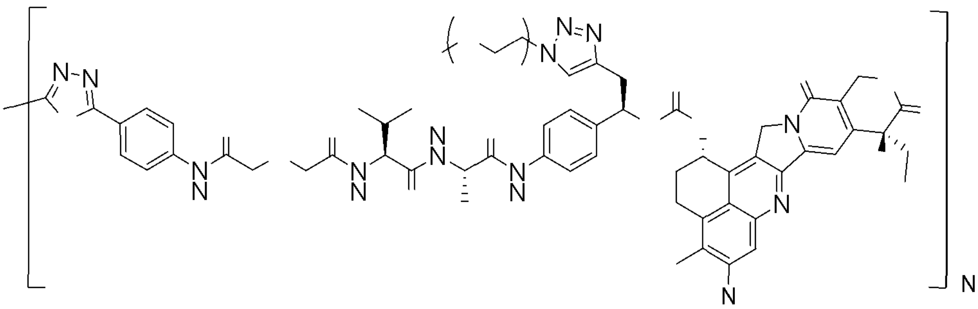

or

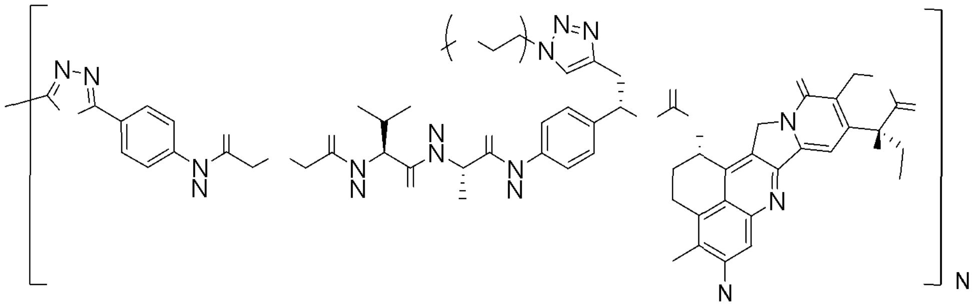

wherein HG, $R_1$, $R_2$, d, s, and m are as defined above.

In some embodiments, the antibody-drug conjugate of formula (I) has the following structure:

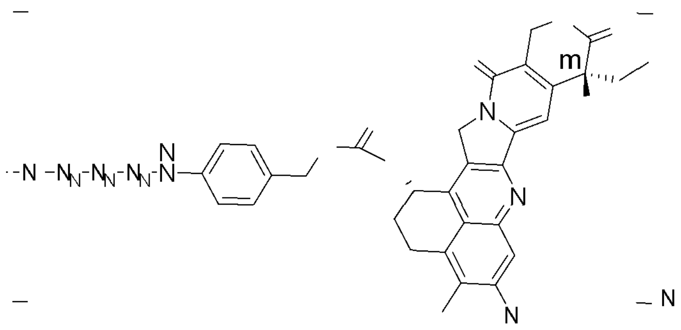

| No. | $L_1$ | $L_2$ | $L_3$ | $L_4$ |
|---|---|---|---|---|
| ADC-1 | $L_1$-1 | $L_2$-1 | $L_3$-1 | $L_4$-1 |
| ADC-2 | $L_1$-1 | $L_2$-1 | $L_3$-2 | $L_4$-1 |
| ADC-3 | $L_1$-1 | $L_2$-1 | $L_3$-3 | $L_4$-1 |
| ADC-4 | $L_1$-1 | $L_2$-1 | $L_3$-4 | $L_4$-1 |
| ADC-5 | $L_1$-1 | $L_2$-1 | $L_3$-5 | $L_4$-1 |
| ADC-6 | $L_1$-1 | $L_2$-1 | $L_3$-6 | $L_4$-1 |
| ADC-7 | $L_1$-1 | $L_2$-1 | $L_3$-7 | $L_4$-1 |
| ADC-8 | $L_1$-1 | $L_2$-1 | $L_3$-1 | $L_4$-2 |
| ADC-9 | $L_1$-1 | $L_2$-1 | $L_3$-2 | $L_4$-2 |
| ADC-10 | $L_1$-1 | $L_2$-1 | $L_3$-3 | $L_4$-2 |
| ADC-11 | $L_1$-1 | $L_2$-1 | $L_3$-4 | $L_4$-2 |
| ADC-12 | $L_1$-1 | $L_2$-1 | $L_3$-5 | $L_4$-2 |
| ADC-13 | $L_1$-1 | $L_2$-1 | $L_3$-6 | $L_4$-2 |
| ADC-14 | $L_1$-1 | $L_2$-1 | $L_3$-7 | $L_4$-2 |
| ADC-15 | $L_1$-2 | $L_2$-5 | $L_3$-1 | $L_4$-1 |
| ADC-16 | $L_1$-2 | $L_2$-5 | $L_3$-2 | $L_4$-1 |
| ADC-17 | $L_1$-2 | $L_2$-5 | $L_3$-3 | $L_4$-1 |
| ADC-18 | $L_1$-2 | $L_2$-5 | $L_3$-4 | $L_4$-1 |
| ADC-19 | $L_1$-2 | $L_2$-5 | $L_3$-5 | $L_4$-1 |
| ADC-20 | $L_1$-2 | $L_2$-5 | $L_3$-6 | $L_4$-1 |
| ADC-21 | $L_1$-2 | $L_2$-5 | $L_3$-7 | $L_4$-1 |
| ADC-22 | $L_1$-2 | $L_2$-5 | $L_3$-1 | $L_4$-2 |

(continued)

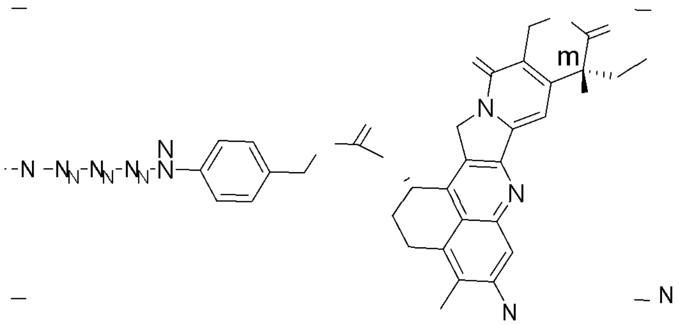

| No. | $L_1$ | $L_2$ | $L_3$ | $L_4$ |
|---|---|---|---|---|
| ADC-23 | $L_1$-2 | $L_2$-5 | $L_3$-2 | $L_4$-2 |
| ADC-24 | $L_1$-2 | $L_2$-5 | $L_3$-3 | $L_4$-2 |
| ADC-25 | $L_1$-2 | $L_2$-5 | $L_3$-4 | $L_4$-2 |
| ADC-26 | $L_1$-2 | $L_2$-5 | $L_3$-5 | $L_4$-2 |
| ADC-27 | $L_1$-2 | $L_2$-5 | $L_3$-6 | $L_4$-2 |
| ADC-28 | $L_1$-2 | $L_2$-5 | $L_3$-7 | $L_4$-2 |
| ADC -29 | $L_1$-5 | $L_2$-2 | $L_3$-1 | $L_4$-1 |
| ADC-30 | $L_1$-5 | $L_2$-2 | $L_3$-2 | $L_4$-1 |
| ADC-31 | $L_1$-5 | $L_2$-2 | $L_3$-3 | $L_4$-1 |
| ADC-32 | $L_1$-5 | $L_2$-2 | $L_3$-4 | $L_4$-1 |
| ADC-33 | $L_1$-5 | $L_2$-2 | $L_3$-5 | $L_4$-1 |
| ADC-34 | $L_1$-5 | $L_2$-2 | $L_3$-6 | $L_4$-1 |
| ADC-35 | $L_1$-5 | $L_2$-2 | $L_3$-7 | $L_4$-1 |
| ADC-36 | $L_1$-5 | $L_2$-2 | $L_3$-1 | $L_4$-2 |
| ADC-37 | $L_1$-5 | $L_2$-2 | $L_3$-2 | $L_4$-2 |
| ADC-38 | $L_1$-5 | $L_2$-2 | $L_3$-3 | $L_4$-2 |
| ADC-39 | $L_1$-5 | $L_2$-2 | $L_3$-4 | $L_4$-2 |
| ADC-40 | $L_1$-5 | $L_2$-2 | $L_3$-5 | $L_4$-2 |
| ADC-41 | $L_1$-5 | $L_2$-2 | $L_3$-6 | $L_4$-2 |
| ADC-42 | $L_1$-5 | $L_2$-2 | $L_3$-7 | $L_4$-2 |
| ADC-43 | $L_1$-1 | $L_2$-1 | $L_3$-1 | $L_4$-3 |
| ADC-44 | $L_1$-1 | $L_2$-1 | $L_3$-1 | $L_4$-4 |
| ADC-45 | $L_1$-2 | $L_2$-5 | $L_3$-1 | $L_4$-3 |
| ADC-46 | $L_1$-2 | $L_2$-5 | $L_3$-1 | $L_4$-4 |
| ADC-47 | $L_1$-5 | $L_2$-2 | $L_3$-1 | $L_4$-3 |
| ADC-48 | $L_1$-5 | $L_2$-2 | $L_3$-1 | $L_4$-4 |

In some embodiments, the antibody-drug conjugate of formula (I) has the following structure:

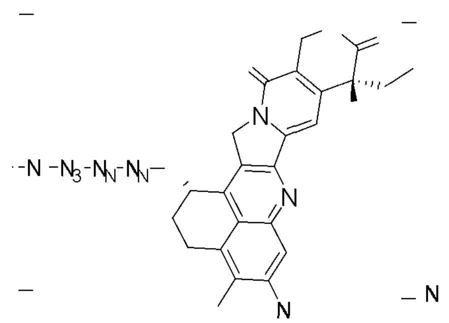

| No. | $L_1$ | $L_2$ | $L_3$ | $L_4$ |
|---|---|---|---|---|
| ADC-49 | $L_1$-1 | $L_2$-1 | $L_3$-1 | $L_4$-1 |
| ADC-50 | $L_1$-1 | $L_2$-1 | $L_3$-2 | $L_4$-1 |
| ADC-51 | $L_1$-1 | $L_2$-1 | $L_3$-3 | $L_4$-1 |
| ADC-52 | $L_1$-1 | $L_2$-1 | $L_3$-4 | $L_4$-1 |
| ADC-53 | $L_1$-1 | $L_2$-1 | $L_3$-5 | $L_4$-1 |
| ADC-54 | $L_1$-1 | $L_2$-1 | $L_3$-6 | $L_4$-1 |
| ADC-55 | $L_1$-1 | $L_2$-1 | $L_3$-7 | $L_4$-1 |
| ADC-56 | $L_1$-1 | $L_2$-1 | $L_3$-1 | $L_4$-2 |
| ADC-57 | $L_1$-1 | $L_2$-1 | $L_3$-2 | $L_4$-2 |
| ADC-58 | $L_1$-1 | $L_2$-1 | $L_3$-3 | $L_4$-2 |
| ADC-59 | $L_1$-1 | $L_2$-1 | $L_3$-4 | $L_4$-2 |
| ADC-60 | $L_1$-1 | $L_2$-1 | $L_3$-5 | $L_4$-2 |
| ADC-61 | $L_1$-1 | $L_2$-1 | $L_3$-6 | $L_4$-2 |
| ADC-62 | $L_1$-1 | $L_2$-1 | $L_3$-7 | $L_4$-2 |
| ADC-63 | $L_1$-2 | $L_2$-5 | $L_3$-1 | $L_4$-1 |
| ADC-64 | $L_1$-2 | $L_2$-5 | $L_3$-2 | $L_4$-1 |
| ADC-65 | $L_1$-2 | $L_2$-5 | $L_3$-3 | $L_4$-1 |
| ADC-66 | $L_1$-2 | $L_2$-5 | $L_3$-4 | $L_4$-1 |
| ADC-67 | $L_1$-2 | $L_2$-5 | $L_3$-5 | $L_4$-1 |
| ADC-68 | $L_1$-2 | $L_2$-5 | $L_3$-6 | $L_4$-1 |
| ADC-69 | $L_1$-2 | $L_2$-5 | $L_3$-7 | $L_4$-1 |
| ADC-70 | $L_1$-2 | $L_2$-5 | $L_3$-1 | $L_4$-2 |
| ADC-71 | $L_1$-2 | $L_2$-5 | $L_3$-2 | $L_4$-2 |
| ADC-72 | $L_1$-2 | $L_2$-5 | $L_3$-3 | $L_4$-2 |
| ADC-73 | $L_1$-2 | $L_2$-5 | $L_3$-4 | $L_4$-2 |
| ADC-74 | $L_1$-2 | $L_2$-5 | $L_3$-5 | $L_4$-2 |
| ADC-75 | $L_1$-2 | $L_2$-5 | $L_3$-6 | $L_4$-2 |
| ADC-76 | $L_1$-2 | $L_2$-5 | $L_3$-7 | $L_4$-2 |
| ADC-77 | $L_1$-5 | $L_2$-2 | $L_3$-1 | $L_4$-1 |
| ADC-78 | $L_1$-5 | $L_2$-2 | $L_3$-2 | $L_4$-1 |
| ADC-79 | $L_1$-5 | $L_2$-2 | $L_3$-3 | $L_4$-1 |
| ADC-80 | $L_1$-5 | $L_2$-2 | $L_3$-4 | $L_4$-1 |

(continued)

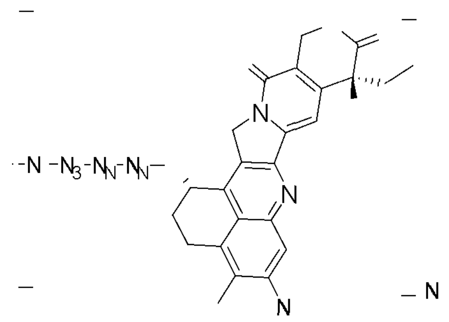

| No. | $L_1$ | $L_2$ | $L_3$ | $L_4$ |
|---|---|---|---|---|
| ADC-81 | $L_1$-5 | $L_2$-2 | $L_3$-5 | $L_4$-1 |
| ADC-82 | $L_1$-5 | $L_2$-2 | $L_3$-6 | $L_4$-1 |
| ADC-83 | $L_1$-5 | $L_2$-2 | $L_3$-7 | $L_4$-1 |
| ADC-84 | $L_1$-5 | $L_2$-2 | $L_3$-1 | $L_4$-2 |
| ADC-85 | $L_1$-5 | $L_2$-2 | $L_3$-2 | $L_4$-2 |
| ADC-86 | $L_1$-5 | $L_2$-2 | $L_3$-3 | $L_4$-2 |
| ADC-87 | $L_1$-5 | $L_2$-2 | $L_3$-4 | $L_4$-2 |
| ADC-88 | $L_1$-5 | $L_2$-2 | $L_3$-5 | $L_4$-2 |
| ADC-89 | $L_1$-5 | $L_2$-2 | $L_3$-6 | $L_4$-2 |
| ADC-90 | $L_1$-5 | $L_2$-2 | $L_3$-7 | $L_4$-2 |
| ADC-91 | $L_1$-1 | $L_2$-1 | $L_3$-1 | $L_4$-3 |
| ADC-92 | $L_1$-1 | $L_2$-1 | $L_3$-1 | $L_4$-4 |
| ADC-93 | $L_1$-2 | $L_2$-5 | $L_3$-1 | $L_4$-3 |
| ADC-94 | $L_1$-2 | $L_2$-5 | $L_3$-1 | $L_4$-4 |
| ADC-95 | $L_1$-5 | $L_2$-2 | $L_3$-1 | $L_4$-3 |
| ADC-96 | $L_1$-5 | $L_2$-2 | $L_3$-1 | $L_4$-4 |

In some embodiments, the antibody-drug conjugate of formula (I) has the following structure:

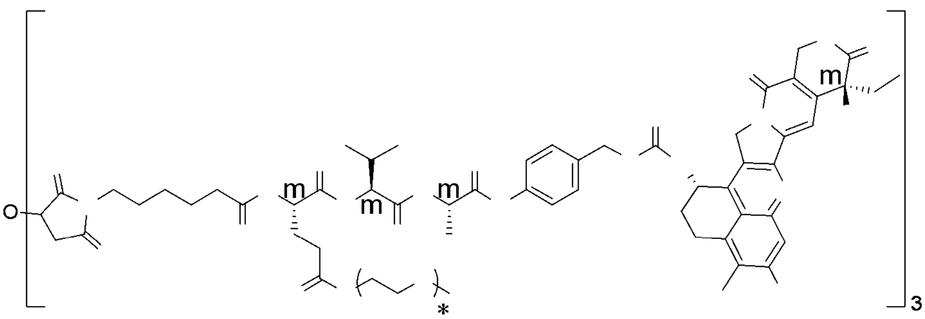

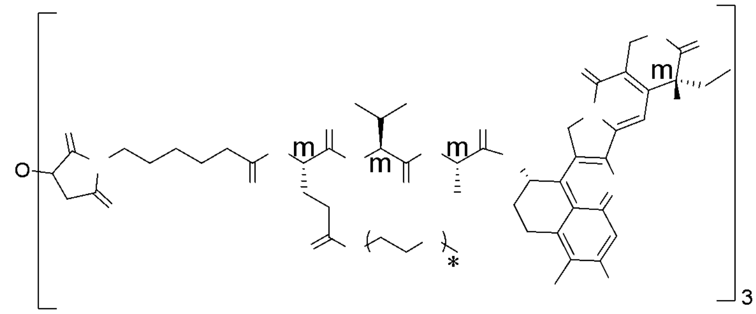

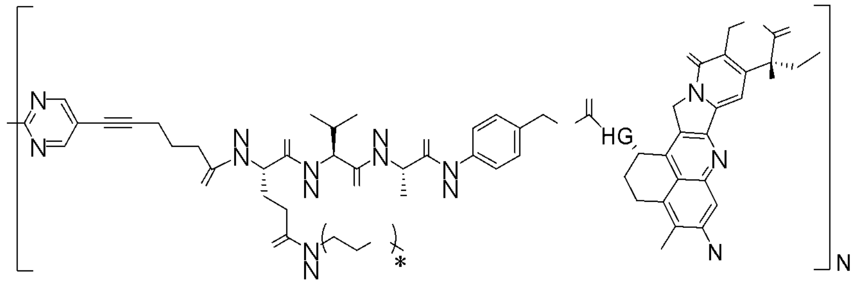

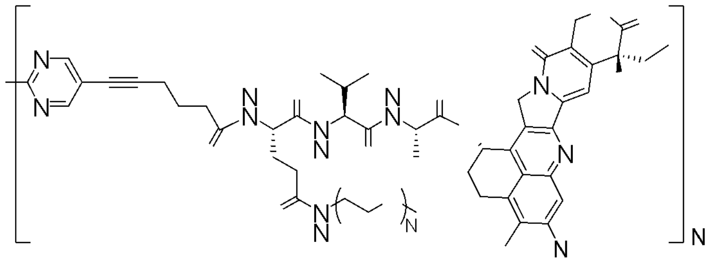

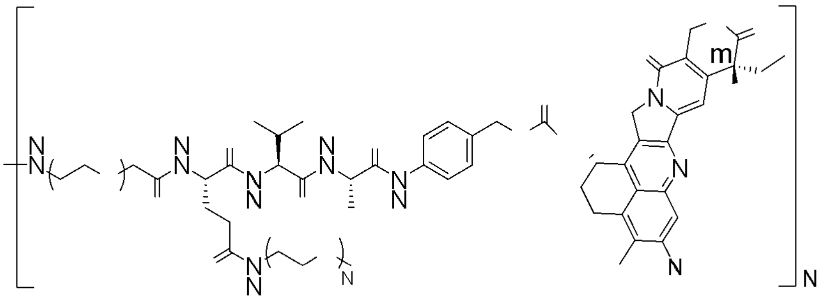

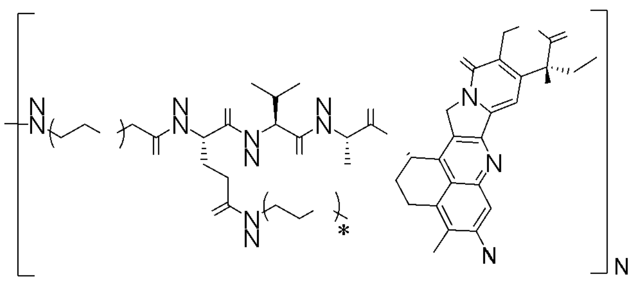

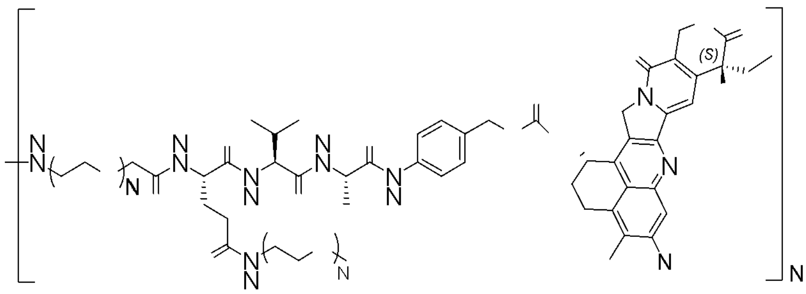

or

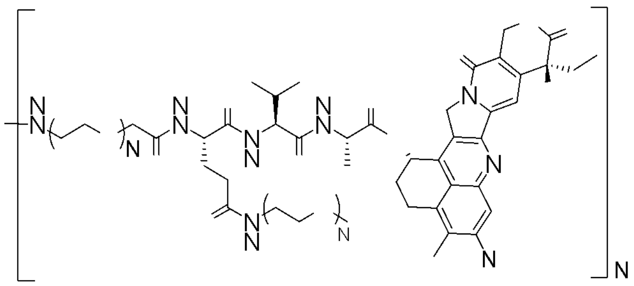

wherein n, d, and s are as defined above.

In some embodiments, in the antibody-drug conjugate of formula (I), A is 43A, 43B, or DP001. In some embodiments, the antibody-drug conjugate of formula (I) is selected from:

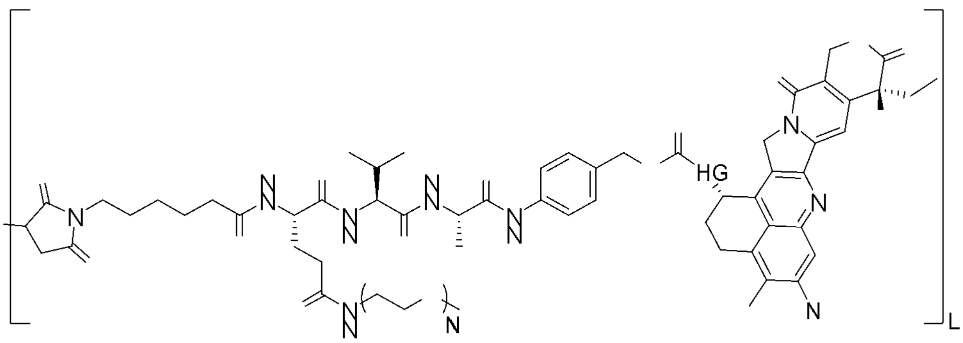

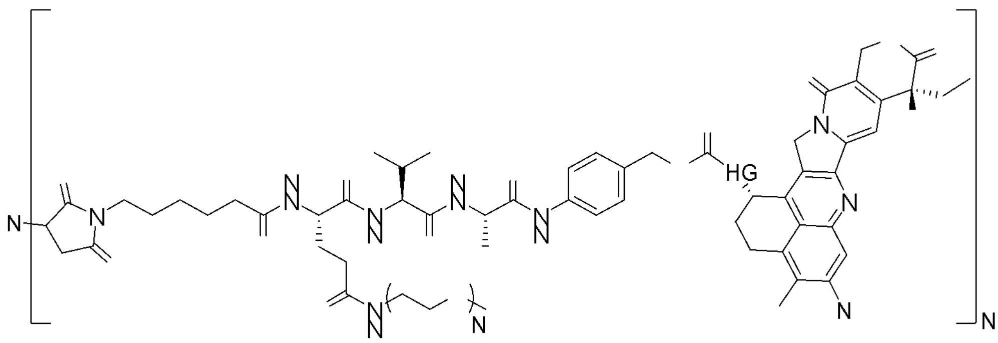

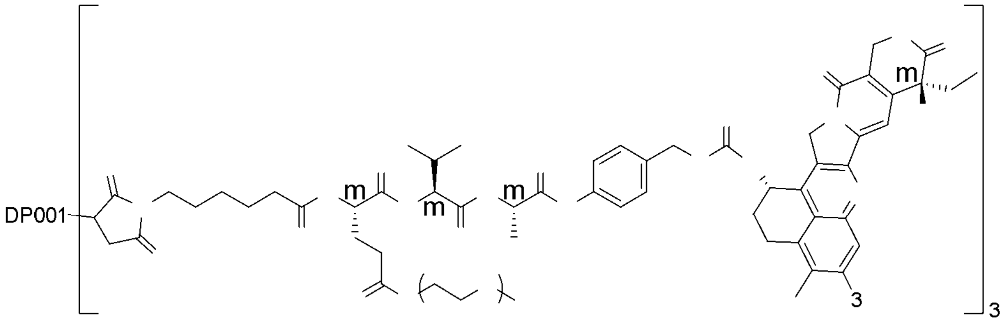

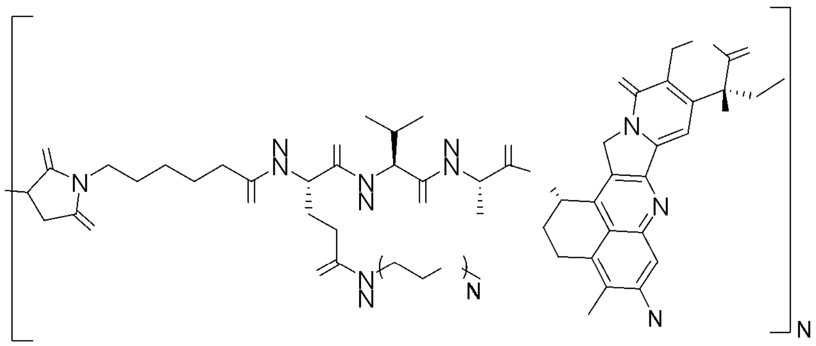

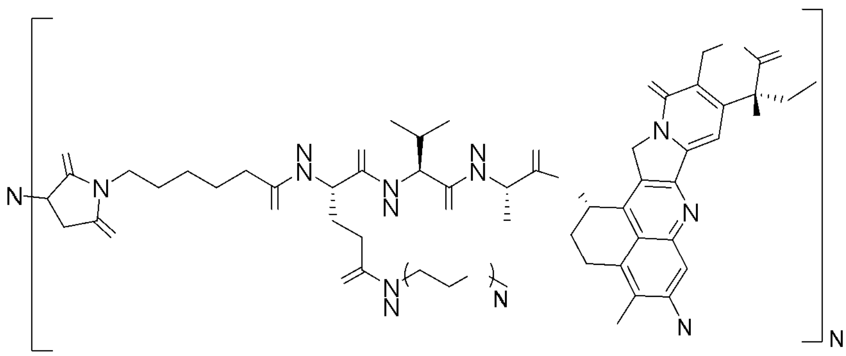

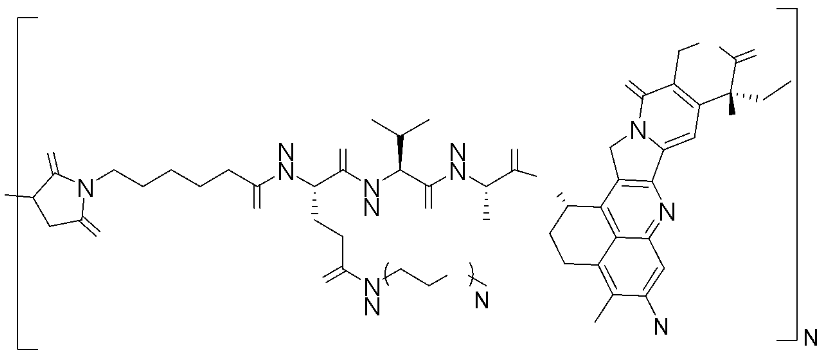

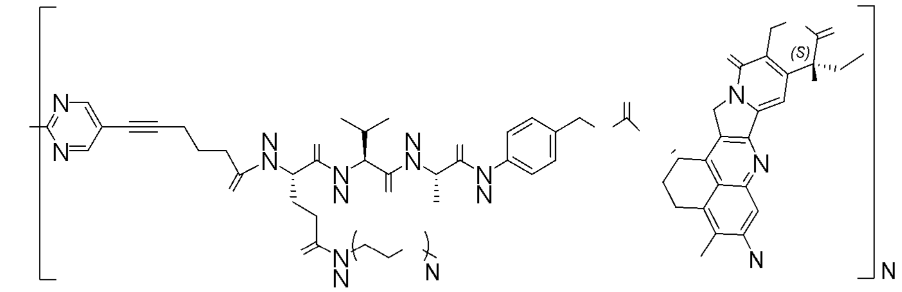

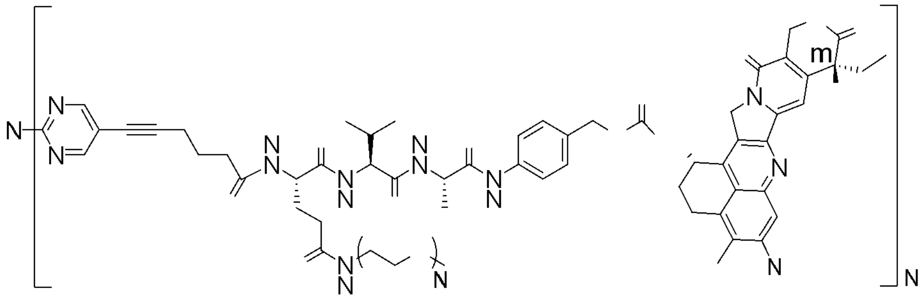

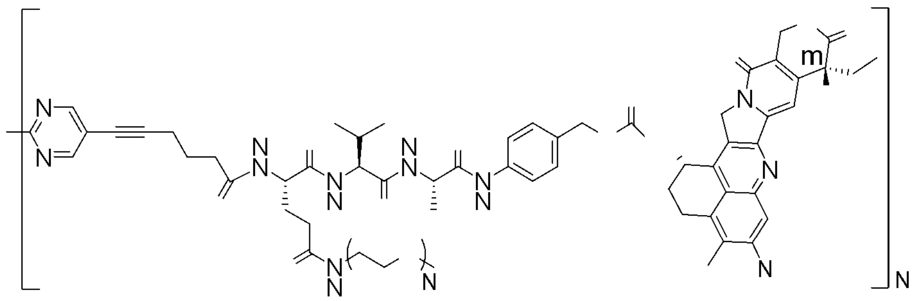

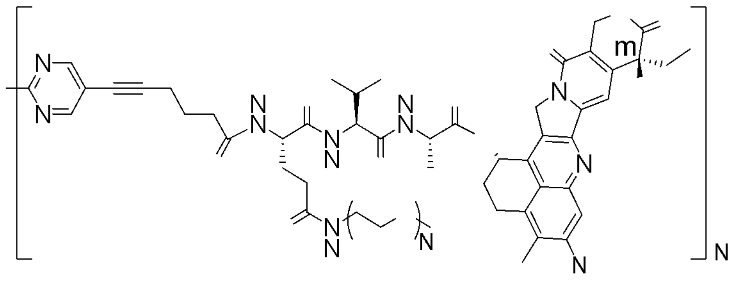

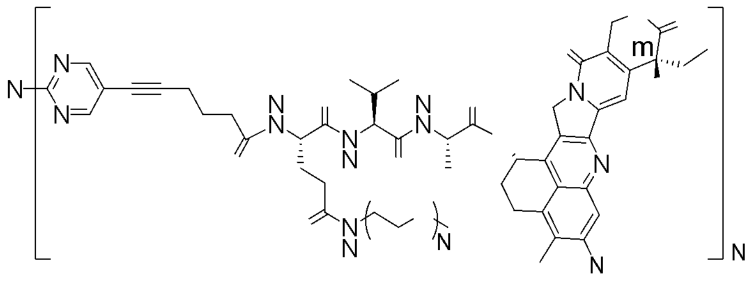

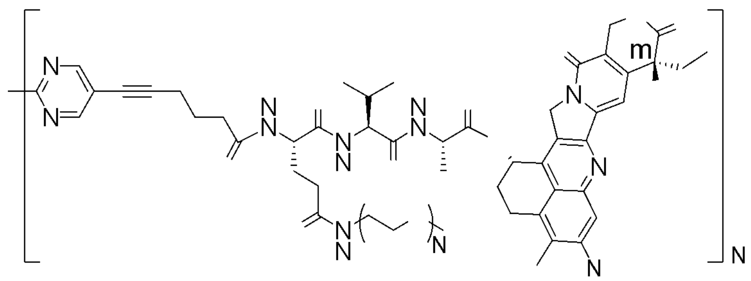

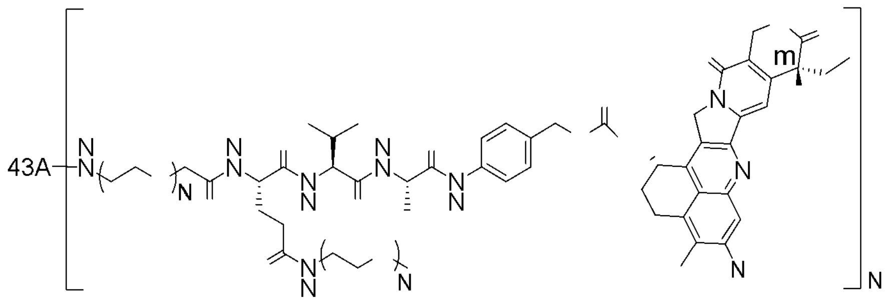

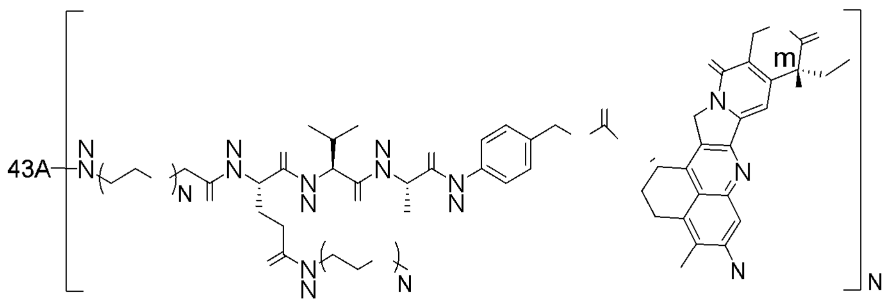

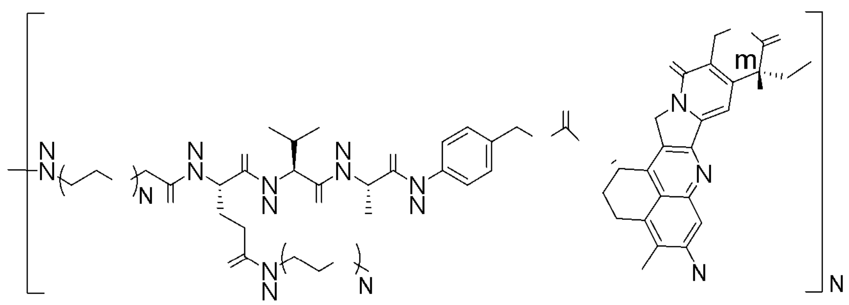

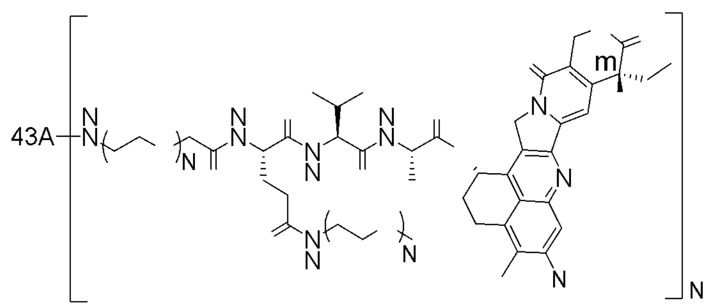

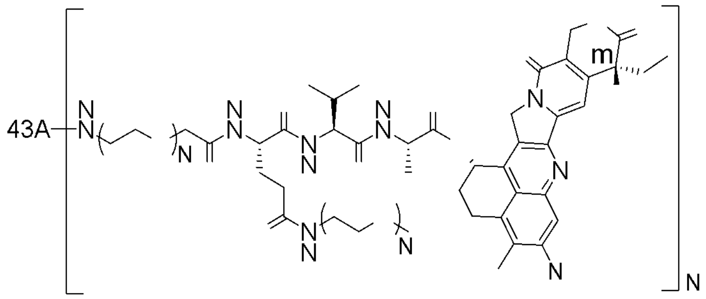

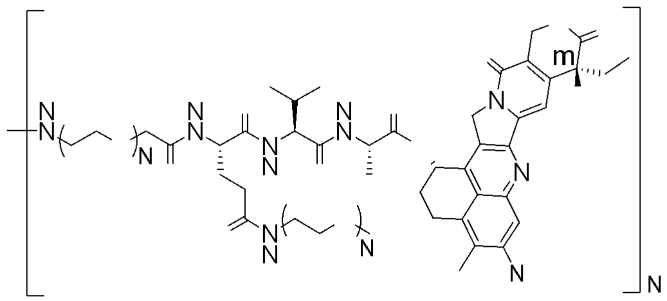

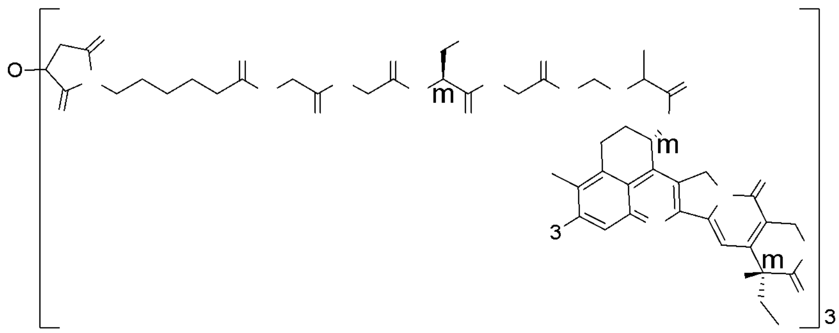

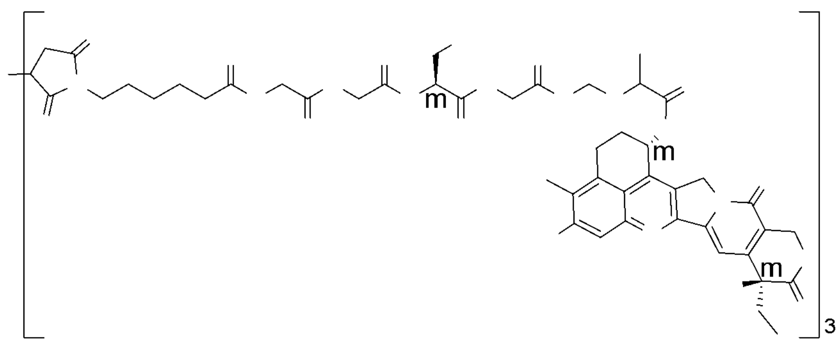

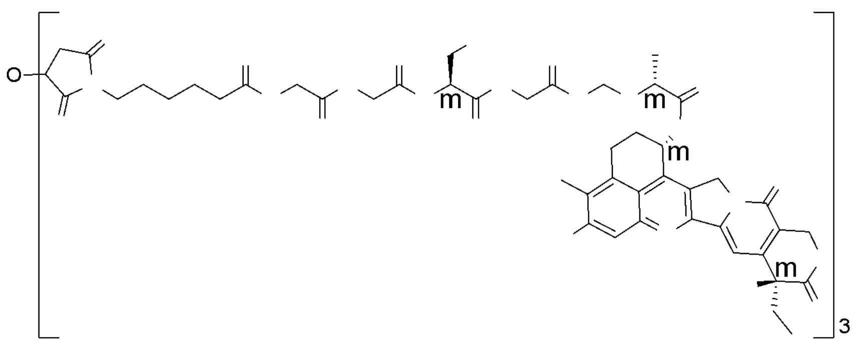

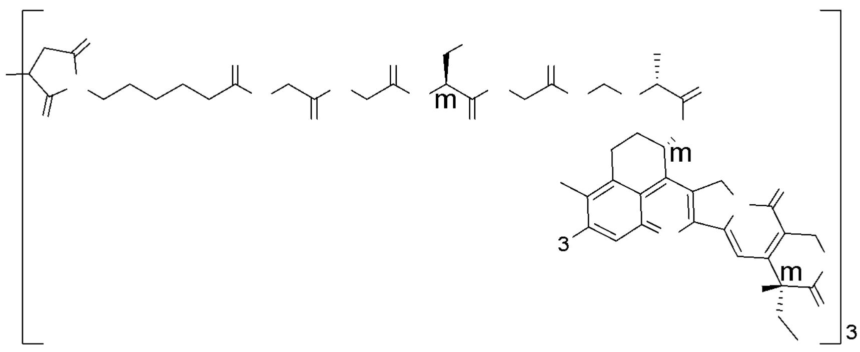

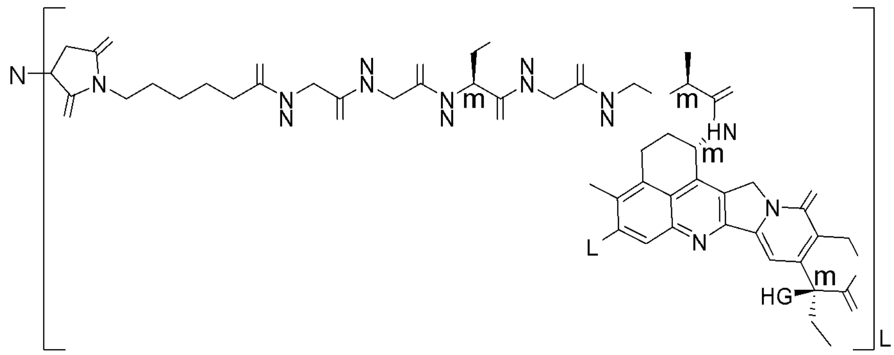

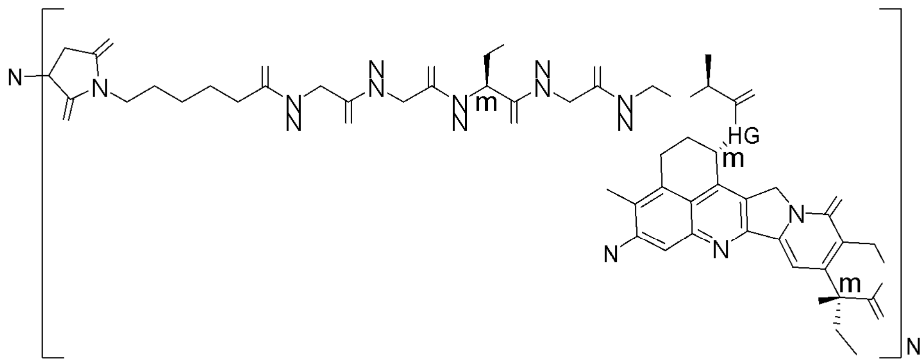

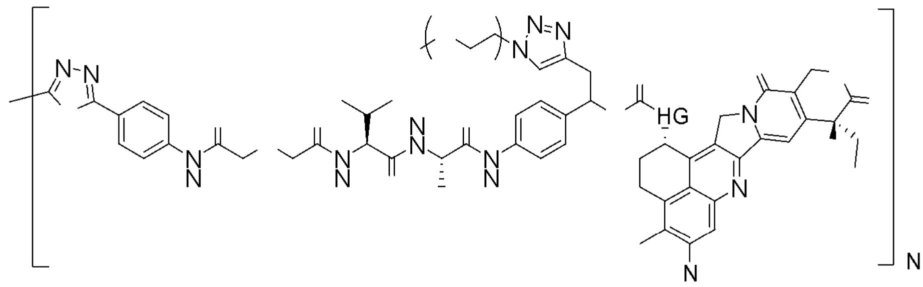

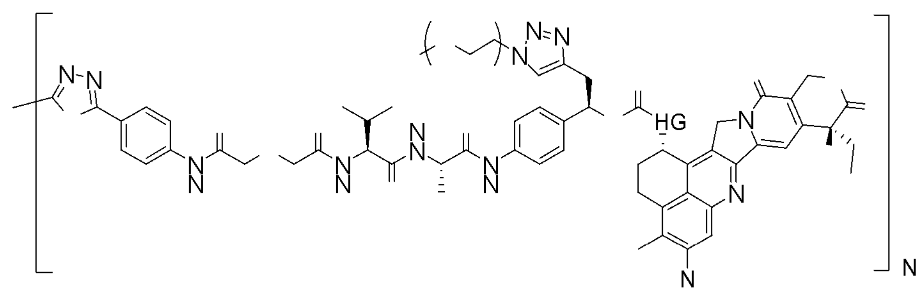

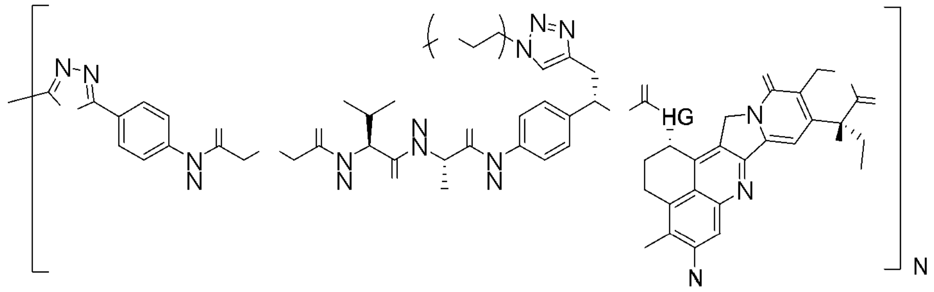

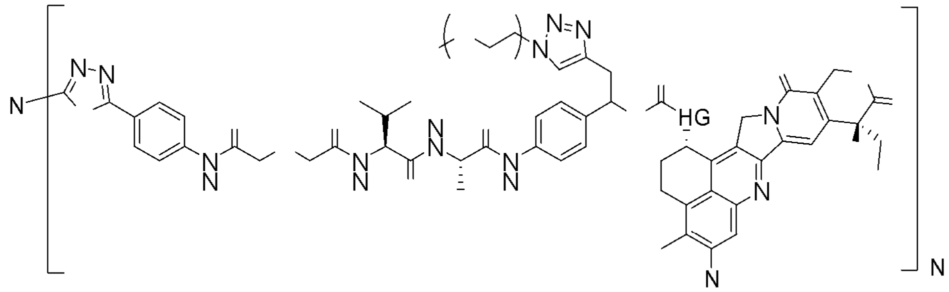

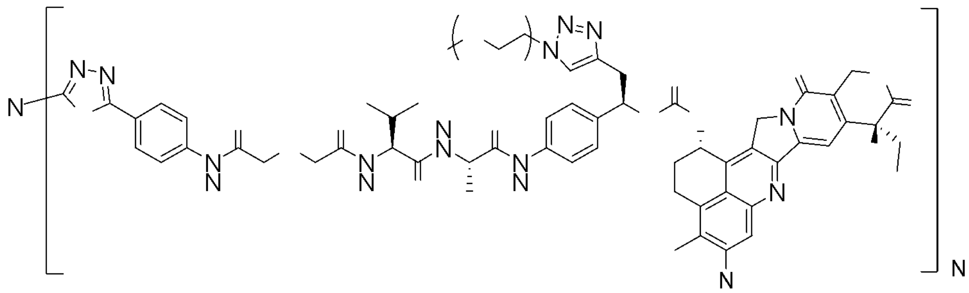

and

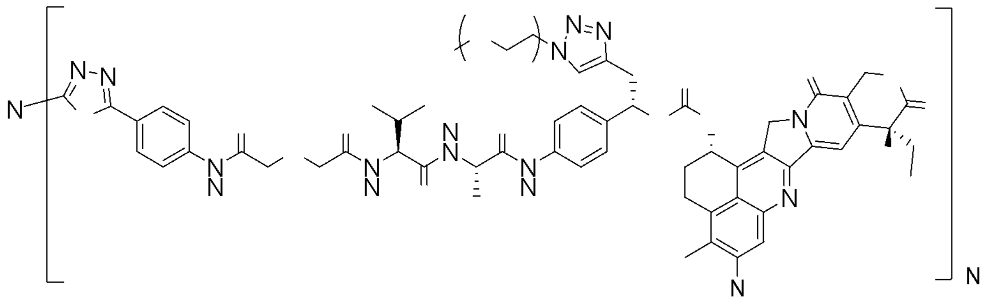

wherein d is an integer or a decimal selected from 1-8, preferably an integer or a decimal selected from 2-8, more preferably an integer or a decimal selected from 4-8, and most preferably 2, 4, 6, or 8.

The present disclosure further provides a linker-drug compound having a structure represented by formula (II), and a tautomer, a mesomer, a racemate, an enantiomer, a diastereoisomer, a pharmaceutically acceptable salt, a hydrate, a solvate or an isotopically labeled compound thereof:

$$L_1'\text{-}L_2\text{-}L_3\text{-}L_4\text{-}L_5\text{-}D \qquad \text{(II)}$$

wherein $L_1'$ is selected from

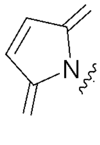, 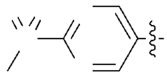, 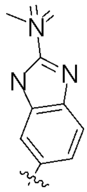, 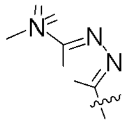, and 3 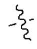;

$L_2$, $L_3$, $L_4$, $L_5$ and D are as defined in formula (I).

In some embodiments, the linker-drug compound having the structure represented by formula (II) is selected from:

| 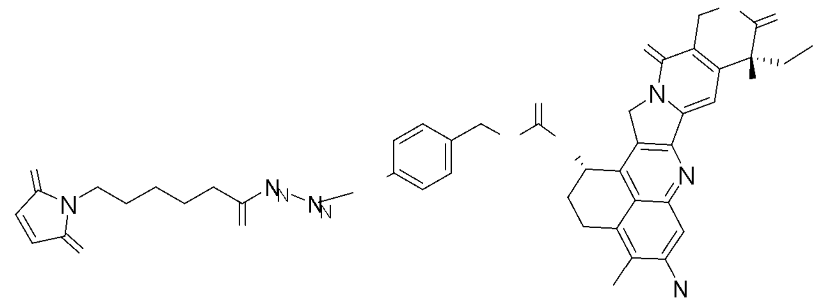 | | |
|---|---|---|
| No. | $L_3$ | $L_4$ |
| LD-1 | $L_3$-1 | $L_4$-1 |
| LD-2 | $L_3$-2 | $L_4$-1 |
| LD-3 | $L_3$-3 | $L_4$-1 |
| LD-4 | $L_3$-4 | $L_4$-1 |
| LD-5 | $L_3$-5 | $L_4$-1 |
| LD-6 | $L_3$-6 | $L_4$-1 |
| LD-7 | $L_3$-7 | $L_4$-1 |
| LD-8 | $L_3$-1 | $L_4$-2 |
| LD-9 | $L_3$-2 | $L_4$-2 |
| LD-10 | $L_3$-3 | $L_4$-2 |
| LD-11 | $L_3$-4 | $L_4$-2 |
| LD-12 | $L_3$-5 | $L_4$-2 |
| LD-13 | $L_3$-6 | $L_4$-2 |
| LD-14 | $L_3$-7 | $L_4$-2 |
| LD-43 | $L_3$-1 | $L_4$-3 |
| LD-44 | $L_3$-1 | $L_4$-4 |

(continued)

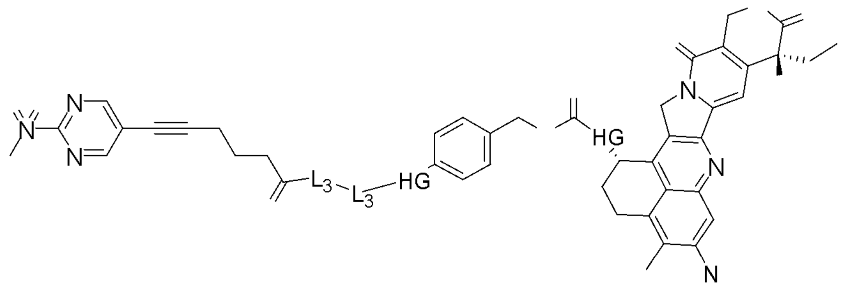

| No. | $L_3$ | $L_4$ |
|---|---|---|
| LD-15 | $L_3$-1 | $L_4$-1 |
| LD-16 | $L_3$-2 | $L_4$-1 |
| LD-17 | $L_3$-3 | $L_4$-1 |
| LD-18 | $L_3$-4 | $L_4$-1 |
| LD-19 | $L_3$-5 | $L_4$-1 |
| LD-20 | $L_3$-6 | $L_4$-1 |
| LD-21 | $L_3$-7 | $L_4$-1 |
| LD-22 | $L_3$-1 | $L_4$-2 |
| LD-23 | $L_3$-2 | $L_4$-2 |
| LD-24 | $L_3$-3 | $L_4$-2 |
| LD-25 | $L_3$-4 | $L_4$-2 |
| LD-26 | $L_3$-5 | $L_4$-2 |
| LD-27 | $L_3$-6 | $L_4$-2 |
| LD-28 | $L_3$-7 | $L_4$-2 |
| LD-45 | $L_3$-1 | $L_4$-3 |
| LD-46 | $L_3$-1 | $L_4$-4 |

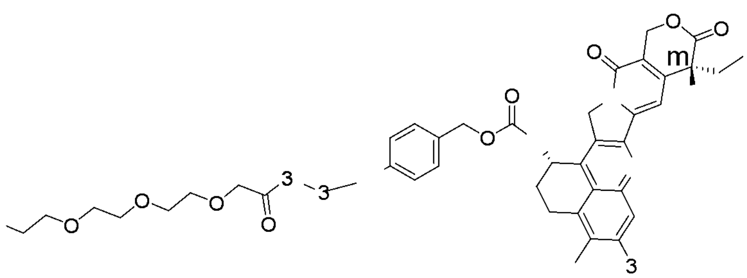

| No. | $L_3$ | $L_4$ |
|---|---|---|
| LD-29 | $L_3$-1 | $L_4$-1 |
| LD-30 | $L_3$-2 | $L_4$-1 |
| LD-31 | $L_3$-3 | $L_4$-1 |
| LD-32 | $L_3$-4 | $L_4$-1 |
| LD-33 | $L_3$-5 | $L_4$-1 |
| LD-34 | $L_3$-6 | $L_4$-1 |
| LD-35 | $L_3$-7 | $L_4$-1 |
| LD-36 | $L_3$-1 | $L_4$-2 |
| LD-37 | $L_3$-2 | $L_4$-2 |

(continued)

| 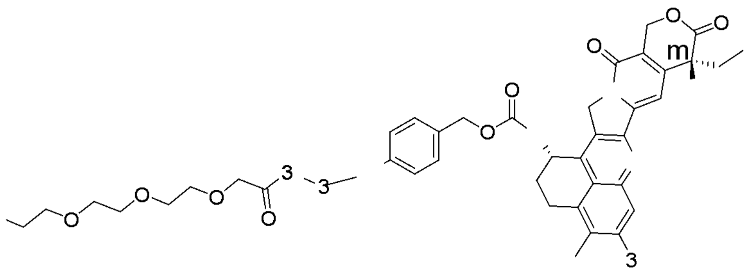 | | |
|---|---|---|
| No. | $L_3$ | $L_4$ |
| LD-38 | $L_3$-3 | $L_4$-2 |
| LD-39 | $L_3$-4 | $L_4$-2 |
| LD-40 | $L_3$-5 | $L_4$-2 |
| LD-41 | $L_3$-6 | $L_4$-2 |
| LD-42 | $L_3$-7 | $L_4$-2 |
| LD-47 | $L_3$-1 | $L_4$-3 |
| LD-48 | $L_3$-1 | $L_4$-4 |

In some embodiments, the linker-drug compound having the structure represented by formula (II) is selected from:

| 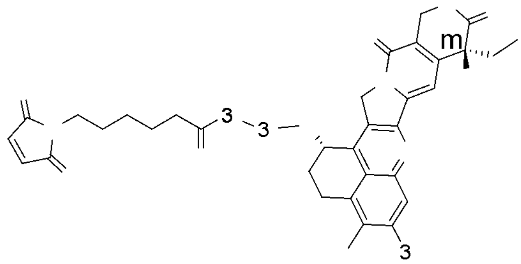 | | |
|---|---|---|
| No. | $L_3$ | $L_4$ |
| LD-49 | $L_3$-1 | $L_4$-1 |
| LD-50 | $L_3$-2 | $L_4$-1 |
| LD-51 | $L_3$-3 | $L_4$-1 |
| LD-52 | $L_3$-4 | $L_4$-1 |
| LD-53 | $L_3$-5 | $L_4$-1 |
| LD-54 | $L_3$-6 | $L_4$-1 |
| LD-55 | $L_3$-7 | $L_4$-1 |
| LD-56 | $L_3$-1 | $L_4$-2 |
| LD-57 | $L_3$-2 | $L_4$-2 |
| LD-58 | $L_3$-3 | $L_4$-2 |
| LD-59 | $L_3$-4 | $L_4$-2 |
| LD-60 | $L_3$-5 | $L_4$-2 |
| LD-61 | $L_3$-6 | $L_4$-2 |
| LD-62 | $L_3$-7 | $L_4$-2 |
| LD-91 | $L_3$-1 | $L_4$-3 |

(continued)

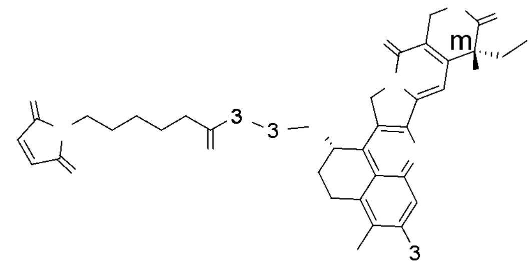

| No. | $L_3$ | $L_4$ |
|---|---|---|
| LD-92 | $L_3$-1 | $L_4$-4 |

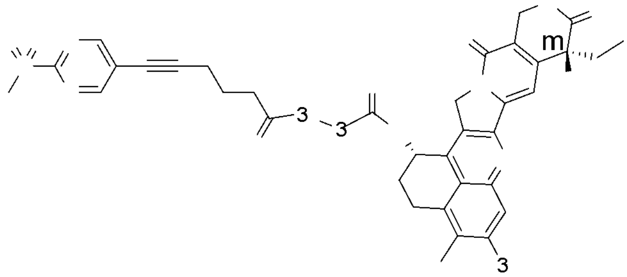

| No. | $L_3$ | $L_4$ |
|---|---|---|
| LD-63 | $L_3$-1 | $L_4$-1 |
| LD-64 | $L_3$-2 | $L_4$-1 |
| LD-65 | $L_3$-3 | $L_4$-1 |
| LD-66 | $L_3$-4 | $L_4$-1 |
| LD-67 | $L_3$-5 | $L_4$-1 |
| LD-68 | $L_3$-6 | $L_4$-1 |
| LD-69 | $L_3$-7 | $L_4$-1 |
| LD-70 | $L_3$-1 | $L_4$-2 |
| LD-71 | $L_3$-2 | $L_4$-2 |
| LD-72 | $L_3$-3 | $L_4$-2 |
| LD-73 | $L_3$-4 | $L_4$-2 |
| LD-74 | $L_3$-5 | $L_4$-2 |
| LD-75 | $L_3$-6 | $L_4$-2 |
| LD-76 | $L_3$-7 | $L_4$-2 |
| LD-93 | $L_3$-1 | $L_4$-3 |
| LD-94 | $L_3$-1 | $L_4$-4 |
| LD-77 | $L_3$-1 | $L_4$-1 |
| LD-78 | $L_3$-2 | $L_4$-1 |
| LD-79 | $L_3$-3 | $L_4$-1 |
| LD-80 | $L_3$-4 | $L_4$-1 |
| LD-81 | $L_3$-5 | $L_4$-1 |
| LD-82 | $L_3$-6 | $L_4$-1 |
| LD-83 | $L_3$-7 | $L_4$-1 |
| LD-84 | $L_3$-1 | $L_4$-2 |

(continued)

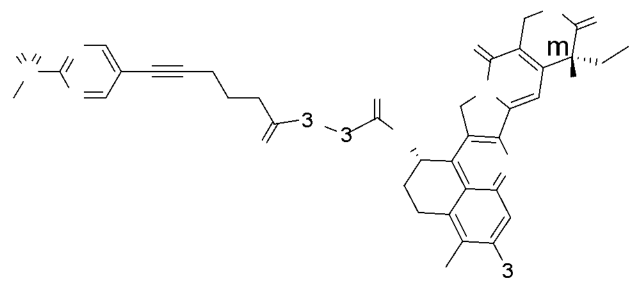

| No. | $L_3$ | $L_4$ |
|---|---|---|
| LD-85 | $L_3$-2 | $L_4$-2 |
| LD-86 | $L_3$-3 | $L_4$-2 |
| LD-87 | $L_3$-4 | $L_4$-2 |
| LD-88 | $L_3$-5 | $L_4$-2 |
| LD-89 | $L_3$-6 | $L_4$-2 |
| LD-90 | $L_3$-7 | $L_4$-2 |
| LD-95 | $L_3$-1 | $L_4$-3 |
| LD-96 | $L_3$-1 | $L_4$-4 |

In some embodiments, the linker-drug compound having the structure represented by formula (II) is selected from:

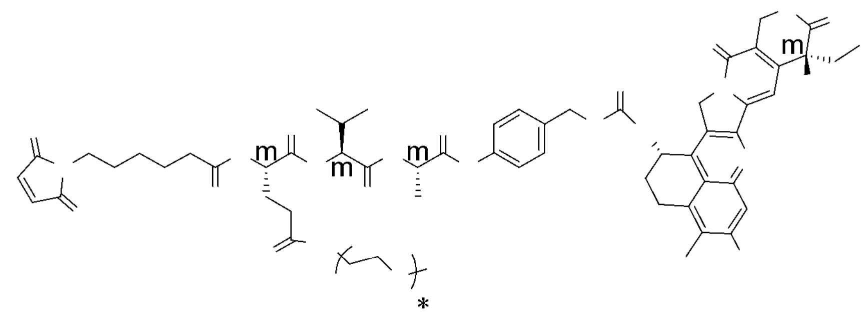

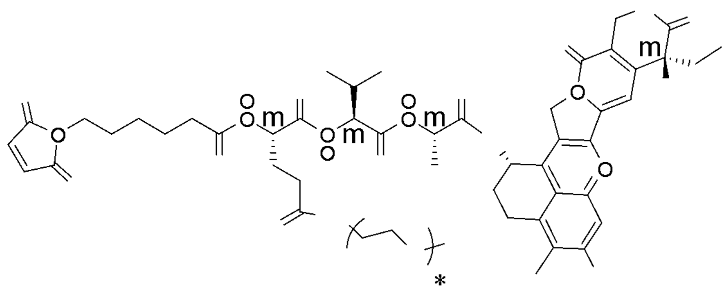

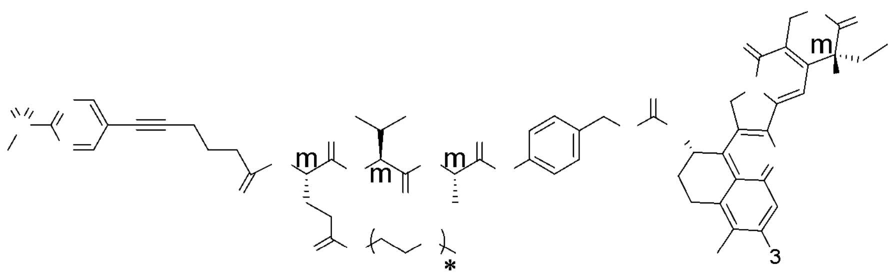

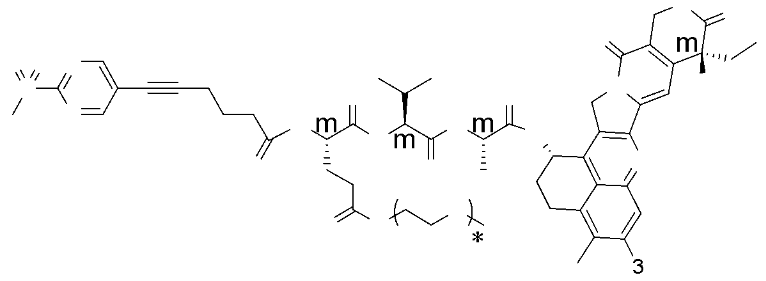

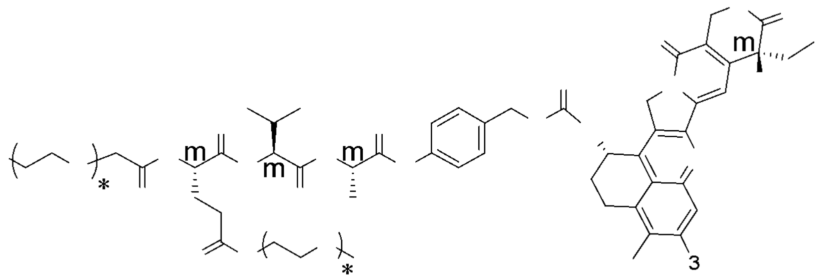

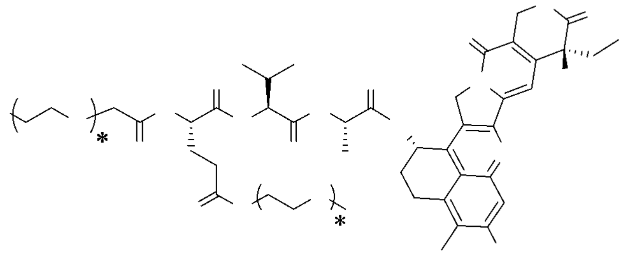

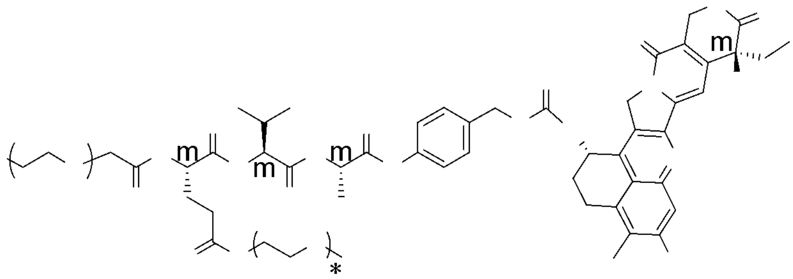

and

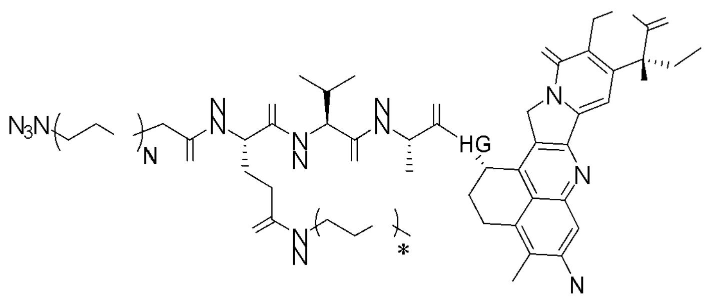

wherein n and s are as defined above.

In some embodiments, the linker-drug compound having the structure represented by formula (II) is selected from:

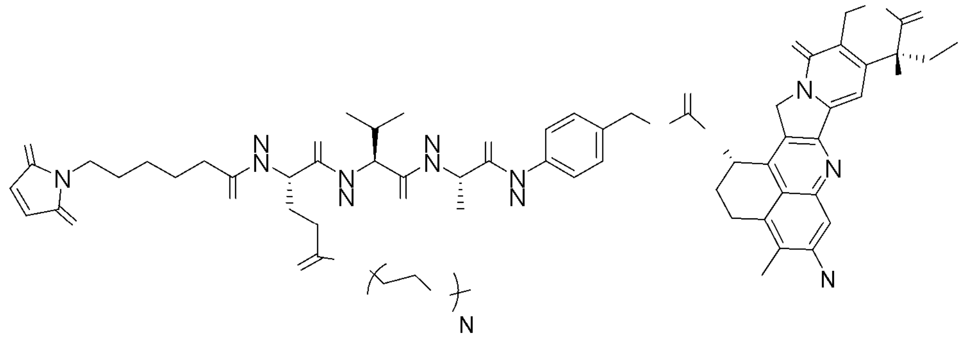

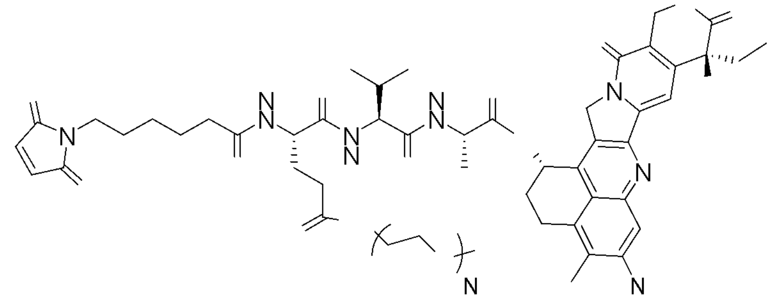

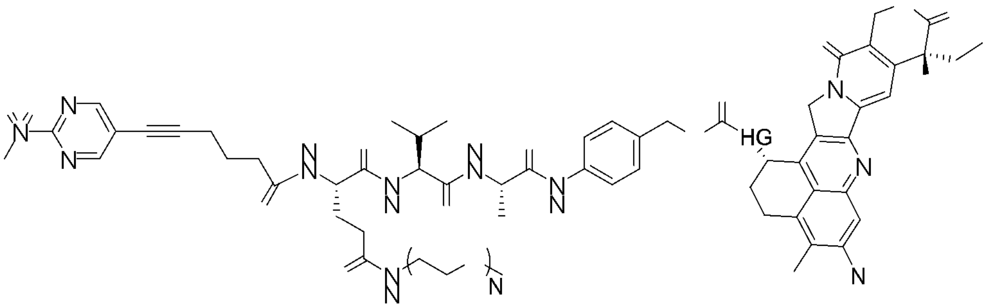

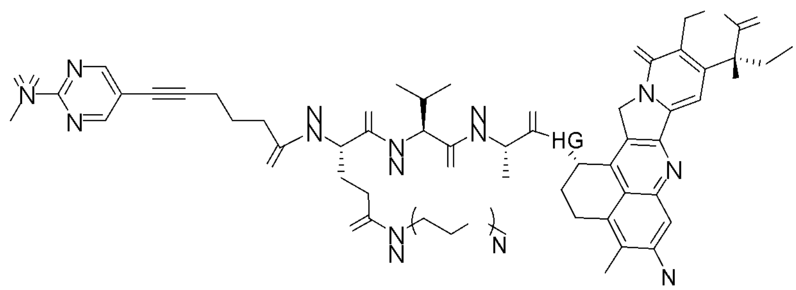

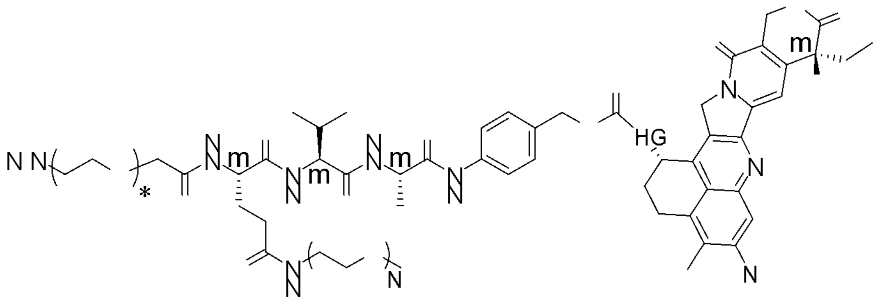

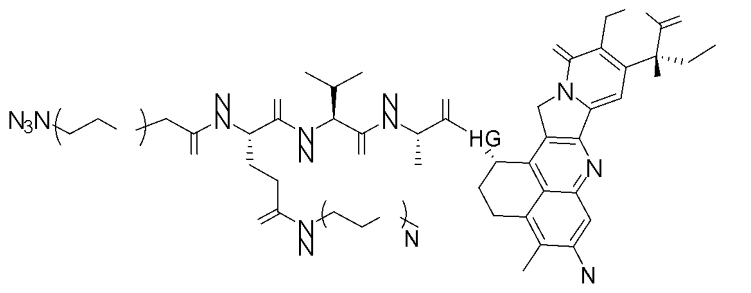

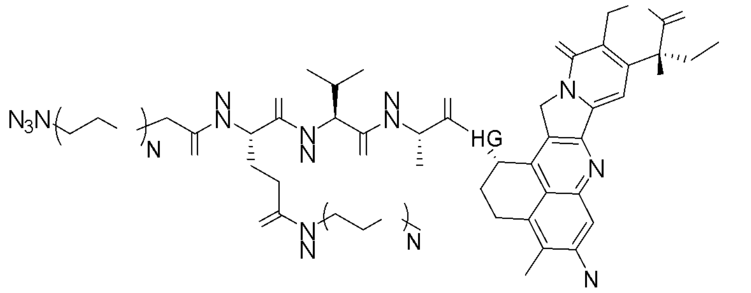

and

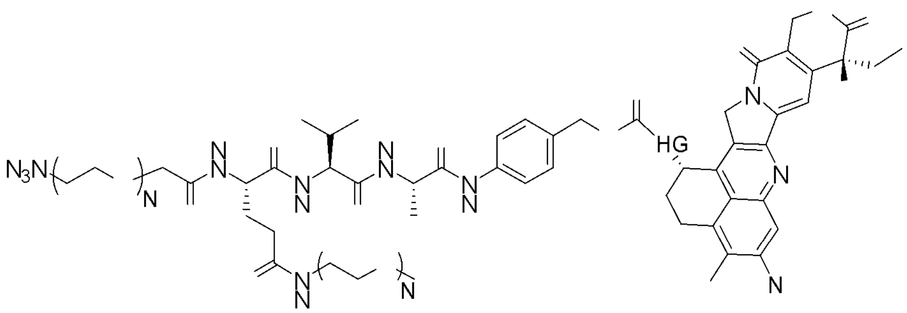

wherein s is as defined above.

In the antibody-drug conjugate, the partial structure consisting of the linker and the drug is referred to as a "linker-drug compound". The linker-drug compound is conjugated to the antibody either by reacting with thiol groups (sulfur atoms of cysteine residues) formed after reduction of one or more of interchain disulfide bond sites (two sites between the heavy chains and two sites between the heavy and light chains) in the antibody, or via microbial transglutaminase (mTGase).

The present disclosure provides a pharmaceutical composition, comprising the antibody-drug conjugate, and the tautomer, the mesomer, the racemate, the enantiomer, the diastereoisomer, the pharmaceutically acceptable salt, the hydrate, the solvate or the isotopically labeled compound thereof described herein. The pharmaceutical composition further comprises a pharmaceutically acceptable excipient and carrier.

The present disclosure provides use of the antibody-drug conjugate, and the tautomer, the mesomer, the racemate, the enantiomer, the diastereoisomer, the pharmaceutically acceptable salt, the hydrate, the solvate or the isotopically labeled compound thereof described herein for the manufacturing of a medicament for the treatment of a proliferative disease. The proliferative disease is preferably a disease associated with abnormal expression of HER2 or B7H3, including a cancer; the cancer is preferably breast cancer, ovarian cancer, cervical cancer, uterine cancer, prostate cancer, kidney cancer, urethral cancer, bladder cancer, liver cancer, gastric cancer, endometrial cancer, salivary gland cancer, esophageal cancer, melanoma, glioma, neuroblastoma, sarcoma, lung cancer (e.g., small cell lung cancer and non-small cell lung cancer), colon cancer, rectal cancer, colorectal cancer, leukemia (e.g., acute lymphoblastic leukemia, acute myeloid leukemia, acute promyelocytic leukemia, chronic myeloid leukemia, or chronic lymphocytic leukemia), bone cancer, skin cancer, thyroid cancer, pancreatic cancer, or lymphoma (e.g., Hodgkin's lymphoma, non-Hodgkin's lymphoma, or recurrent anaplastic large cell lymphoma).

The present disclosure provides a method for treating or preventing a proliferative disease, comprising administering to a patient in need thereof a therapeutically effective dose of the antibody-drug conjugate, and the tautomer, the mesomer, the racemate, the enantiomer, the diastereoisomer, the pharmaceutically acceptable salt, the hydrate, the solvate or the isotopically labeled compound thereof, or the pharmaceutical composition comprising the

same, wherein the proliferative disease is preferably a disease associated with abnormal expression of HER2 or B7H3, including a cancer. The cancer is preferably breast cancer, ovarian cancer, cervical cancer, uterine cancer, prostate cancer, kidney cancer, urethral cancer, bladder cancer, liver cancer, gastric cancer, endometrial cancer, salivary gland cancer, esophageal cancer, melanoma, glioma, neuroblastoma, sarcoma, lung cancer (e.g., small cell lung cancer and non-small cell lung cancer), colon cancer, rectal cancer, colorectal cancer, leukemia (e.g., acute lymphoblastic leukemia, acute myeloid leukemia, acute promyelocytic leukemia, chronic myeloid leukemia, or chronic lymphocytic leukemia), bone cancer, skin cancer, thyroid cancer, pancreatic cancer, or lymphoma (e.g., Hodgkin's lymphoma, non-Hodgkin's lymphoma, or recurrent anaplastic large cell lymphoma).

In some embodiments, the cancer is a tumor expressing HER2 (HER2+) or B7H3 (B7H3+) on the surface of tumor cells.

The present disclosure provides use of the linker-drug compound described herein in the preparation of an antibody-drug conjugate.

The antibody-drug conjugate of the present disclosure may be used alone or in combination with an additional anti-tumor agent.

The present disclosure provides a pharmaceutical composition, comprising the antibody-drug conjugate of the present disclosure and an additional anti-tumor agent.

The present disclosure provides a preparation method for the antibody-drug conjugate described herein, comprising the following steps:

S1, reduction of an antibody: The specific steps are as follows: The medium for the antibody is replaced with PBS 7.0/EDTA to give an antibody concentration of 10 mg/mL. According to the reduction level, appropriate amounts of a 10 mM aqueous TCEP solution and a 1 M aqueous dipotassium phosphate solution are added. After the pH of the resulting solution is confirmed to be correct, the solution is incubated at 37 °C for several hours, thereby reducing the disulfide bonds in the antibody.

S2, conjugation of the antibody with a linker-drug compound:

The specific steps are as follows: An appropriate amount of a 10 mM solution of the compound dissolved in dimethylsulfoxide is added to the above solution at room temperature. The mixture is well mixed and allowed to react at room temperature for 0.5-4 h, thereby conjugating the linker-drug compound to the antibody. Subsequently, a 100 mM aqueous N-ethylmaleimide solution is added, and the resulting mixture is further stirred at room temperature to terminate the reaction of the linker-drug compound.

S3, purification of the antibody-drug conjugate:

The specific steps are as follows: A 1 mL Mabselect SuRe pre-packed chromatographic column is equilibrated with PBS 7.0/EDTA solution (10 mM PB, 137 mM NaCl, 5 mM EDTA, pH 7.0) on an AKTA system, and the antibody-drug conjugate is loaded using a loading loop. After re-equilibration is performed with 10-20 mL of PBS 7.0/EDTA, elution is carried out using an acetate buffer at pH 3.5. The eluate is then neutralized by adding 1/10 volume of a sodium citrate neutralization solution to give the purified antibody-drug conjugate.

The present disclosure provides a preparation method for the antibody-drug conjugate described herein, comprising the following steps:

reacting a linker-drug compound with an antibody in the presence of a mTGase (preferably from *Streptoverticillium mobaraense*).

The specific procedures are as follows: Certain volumes of a linker-drug compound, a reaction buffer, an antibody, a mTGase (preferably from *Streptoverticillium mobaraense*), and $H_2O$ are added into an EP tube. ADC formation is then catalyzed by the mTGase. After being sealed and well mixed, the mixture is placed at room temperature and allowed to react for no more than 4 days. When the heavy chain conjugation ratio exceeds 95%, the conjugation reaction is considered complete, and purification should begin immediately. The purification method for the ADC is the same as that for the antibody. The reaction is monitored using RP-HPLC.

Using a sterilized AKTA® Ready system, the completed reaction mixture is loaded onto a sterilized CAPTIVA® Protein A column at a density of 30-35 g ADC per liter of resin, with a minimum residence time of 5 min to ensure complete binding of the ADC product. A depth filter is used to remove any particles from the reaction mixture and/or buffer during purification and to maintain sterility. The column is then washed with an excess volume of the binding and washing buffer to remove the mTGase, unreacted toxin molecules, and any undesirable buffer components prior to low-pH elution of the desired product. The eluted fraction is collected into a sterile collection bag pre-filled with a neutralization buffer.

The present disclosure further provides an anti-B7H3 antibody or an antigen-binding fragment thereof, comprising a heavy chain and/or a light chain, wherein the heavy chain comprises 3 complementarity determining regions (CDRs), wherein the amino acid sequence of the heavy chain complementarity determining region 1 (HCDR1) is set forth in SEQ ID NO. 1, the amino acid sequence of the heavy chain complementarity determining region 2 (HCDR2) is set forth in SEQ ID NO. 2, and the amino acid sequence of the heavy chain complementarity determining region 3 (HCDR3) is set forth in SEQ

ID NO. 3; the light chain comprises 3 complementarity determining regions (CDRs) of the light chain, wherein the amino acid sequence of the light chain complementarity determining region 1 (LCDR1) is set forth in SEQ ID NO. 4, the amino acid sequence of the light chain complementarity determining region 2 (LCDR2) is set forth in SEQ ID NO. 5, and the amino acid sequence of the light chain complementarity determining region 3 (LCDR3) is set forth in SEQ ID NO. 6; the CDRs are determined according to the Kabat numbering scheme.

In some embodiments, the anti-B7H3 antibody or the antigen-binding fragment thereof comprises a heavy chain and/or a light chain, wherein the sequence of a variable region of the heavy chain (HV) is set forth in SEQ ID NO. 7, and the sequence of a variable region of the light chain (LV) is set forth in SEQ ID NO. 8.

In some embodiments, the anti-B7H3 antibody further comprises a heavy chain constant region sequence or a variant thereof, and/or a light chain constant region sequence or a variant thereof.

In some embodiments, the antigen-binding fragment is selected from a Fab, a Fab', a Fab'-SH, an Fv, an scFv, and a $F(ab')_2$.

In some embodiments, the anti-B7H3 antibody or the antigen-binding fragment thereof is a humanized or fully human antibody or an antigen-binding fragment thereof.

In some embodiments, the anti-B7H3 antibody comprises a heavy chain and/or a light chain, wherein the amino acid sequence of the heavy chain is set forth in SEQ ID NO. 9 or SEQ ID NO. 10, and the amino acid sequence of the light chain is set forth in SEQ ID NO. 11.

The present disclosure provides a pharmaceutical composition, comprising the anti-B7H3 antibody or the antigen-binding fragment thereof described above.

The present disclosure provides a nucleotide sequence encoding the anti-B7H3 antibody or the antigen-binding fragment thereof described above.

The present disclosure provides an expression vector, comprising the nucleotide sequence encoding the anti-B7H3 antibody or the antigen-binding fragment thereof described above.

The present disclosure provides a host cell, comprising the expression vector described above.

The present disclosure provides a bispecific or multispecific antibody, comprising the anti-B7H3 antibody or the antigen-binding fragment thereof described above.

The present disclosure provides an antibody-drug conjugate formed by conjugation of the anti-B7H3 antibody or the antigen-binding fragment thereof described above.

The present disclosure provides an antibody-drug conjugate, comprising the anti-B7H3 antibody or the antigen-binding fragment thereof described above, and a cytotoxic agent.

Compared to antibody-drug conjugates known in the prior art, such as DS-8201, or currently available experimental samples with relatively good efficacy on different targets used as controls, the antibody-drug conjugate of the present disclosure shows a superior anti-cell proliferation effect, a significant tumor inhibition effect, enhanced stability (including plasma stability and buffer stability), lower toxicity, an improved bystander effect (i.e., after target gene-positive tumor cells die, the death of adjacent tumor cells can also be induced), and a wider therapeutic window. The toxin molecules discovered at present exhibit higher topoisomerase inhibitory activity at high concentrations.

Compared to anti-B7H3 antibodies known in the art, such as M30 (CN103687945B), the anti-B7H3 antibody of the present disclosure exhibits higher cell-binding activity and a faster internalization rate.

The sequences of the M30 antibody are as follows:

> M30-H1 (SEQ ID NO. 24)

QVQLVQSGAEVKKPGSSVKVSCKASGYTFTNYVMHWVRQAPGQGLEWMGYINPYNDDV
KYNEKFKGRVTITADESTSTAYMELSSLRSEDTAVYYCARWGYYGSPLYYFDYWGQGTL
VTVSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKRVEPKSCDKTHTCPPCPAPELL
GGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSRE
EMTKNQVSLTCLVKGFYPSDIAVEWESNGQPENNYKTTPPVLDSDGSFFLYSKLTVDKSR
WQQGNVFSCSVMHEALHNHYTQKSLSLSPGK

> M30-L4 (SEQ ID NO. 25)

EIVLTQSPATLSLSPGERATLSCRASSRLIYMHWYQQKPGQAPRPLIYATSNLASGIPARFSG
SGSGTDFTLTISSLEPEDFAVYYCQQWNSNPPTFGQGTKVEIKRTVAAPSVFIFPPSDEQLKS

GTASVVCLLNNFYPREAKVQWKVDNALQSGNSQESVTEQDSKDSTYSLSSTLTLSKADYE KHKVYACEVTHQGLSSPVTKSFNRGEC

Definitions

Unless otherwise specified, the term "antibody-drug conjugate" means that an antibody (e.g., a monoclonal antibody) or an antibody fragment is linked to a bioactive cytotoxic drug via a stable chemical linker compound.

Unless otherwise specified, the term "linker-drug compound" refers to a partial structure in the "antibody-drug conjugate" consisting of the linker compound and the drug compound.

In the present disclosure, the linker-drug compound is linked to the antibody by conventional conjugation methods in the art, including: lysine conjugation, reductive interchain (heavy-heavy and heavy-light) disulfide bond conjugation, enzymatic conjugation, and site-specific conjugation (Beck A, Reichert JM. Antibody-drug conjugates: Present and future. MAbs, 2014, 6: 15-17; McCombs J R, Owen S C. Antibody drug conjugates: design and selection of linker, payload and conjugation chemistry. The AAPS journal, 2015, 17: 339-351). The present disclosure preferably employs reductive interchain (heavy-heavy and heavy-light) disulfide bond conjugation, i.e., linkage via a reaction with thiol groups (sulfur atoms of cysteine residues) formed after reduction of one or more of interchain disulfide bond sites (two sites between the heavy chains and two sites between the heavy and light chains). Also preferred is enzymatic conjugation, i.e., conjugation of the linker-drug compound to the antibody via microbial transglutaminase (mTGase).

Unless otherwise specified, the term "pharmaceutically acceptable salt" refers to a salt that, within the scope of sound medical judgment, is suitable for use in contact with the tissues of mammals, particularly humans, without excessive toxicity, irritation, allergic response, etc., and is commensurate with a reasonable benefit/risk ratio. For example, pharmaceutically acceptable salts of amines, carboxylic acids, and other types of compounds are well known in the art. The salt may be prepared *in situ* during the final separation and purification of the compound of the present disclosure, or prepared separately by reacting a free base or free acid with a suitable reagent.

Unless otherwise specified, the term "isotopically labeled compound" means that the compound of the present disclosure can be present in an isotopically labeled or enriched form, containing one or more atoms whose atomic weight or mass number differs from that of the most abundant isotopic atom found in nature. The isotope may be a radioactive or non-radioactive isotope. Isotopes commonly used as isotopic labels are: hydrogen isotopes: $^{2}H$ and $^{3}H$; carbon isotopes: $^{13}C$ and $^{14}C$; chlorine isotopes: $^{35}Cl$ and $^{37}Cl$; fluorine isotope: $^{18}F$; iodine isotopes: $^{123}I$ and $^{125}I$; nitrogen isotopes: $^{13}N$ and $^{15}N$; oxygen isotopes: $^{15}O$, $^{17}O$, and $^{18}O$; and sulfur isotope: $^{35}S$. These isotopically labeled compounds can be used for research on the distribution of pharmaceutical molecules in tissues. Particularly, $^{2}H$ and $^{13}C$ are more widely used due to their ease of labeling and ease of detection. The substitution with certain heavy isotopes, such as deuterium ($^{2}H$), can enhance the metabolic stability and prolong the half-life to achieve the purpose of reducing the dose, thereby providing therapeutic advantages. Isotopically labeled compounds are generally synthesized from pre-labeled starting materials using known synthetic techniques, analogous to the synthesis of their non-isotopically labeled counterparts.

Unless otherwise specified, the term "solvate" means a physical association of the compound of the present disclosure with one or more solvent molecules (whether organic or inorganic). The physical association includes hydrogen bonding. In certain cases, the solvate can be isolated, for example, when one or more solvent molecules are incorporated into the crystal lattice of a crystalline solid. The solvent molecules in the solvate may be present in a regular arrangement and/or a disordered arrangement. The solvate may contain a stoichiometric or non-stoichiometric amount of solvent molecules. The "solvate" encompasses both solution phase and isolable solvates. Exemplary solvates include, but are not limited to, hydrates, ethanolates, methanolates, and isopropanolates. Solvation methods are well known in the art.

Unless otherwise specified, the term "stereoisomer" refers to compounds that have the same chemical constitution but differ in spatial arrangements of the atoms or groups. Stereoisomers include enantiomers, diastereoisomers, conformational isomers (rotamers), geometric isomers (cis/trans isomers), atropisomers, etc. Any resulting mixture of stereoisomers may be separated into pure or substantially pure geometric isomers, enantiomers, or diastereoisomers depending on differences in the physicochemical properties of the components, for example, by chromatography and/or fractional crystallization.

Unless otherwise specified, the term "heteroatom" refers to a nitrogen, oxygen, sulfur, or halogen atom.

Unless otherwise specified, the term "alkyl" refers to a monovalent saturated aliphatic hydrocarbyl group, i.e., a linear or branched group containing 1-20 carbon atoms, preferably containing 1-10 carbon atoms (i.e., $C_{1-10}$ alkyl), further preferably containing 1-8 carbon atoms ($C_{1-8}$ alkyl), and more preferably containing 1-6 carbon atoms (i.e., $C_{1-6}$ alkyl). For example, "$C_{1-6}$ alkyl" means that the group is alkyl and the number of carbon atoms on the carbon chain is between 1 and 6 (specifically 1, 2, 3, 4, 5, or 6), and examples thereof include, but are not limited to, methyl, ethyl, *n*-propyl, isopropyl, *n*-butyl, isobutyl, *tert*-butyl, *sec*-butyl, *n*-pentyl, neopentyl, 1,1-dimethylpropyl, 1,2-dimethylpropyl, 2,2-dimethylpropyl, 1-ethylpropyl, 2-methylbutyl, 3-methylbutyl, *n*-hexyl, etc. Unless otherwise specified, the term "-alkyl-" or "alkylene" refers to saturated linear or branched divalent hydrocarbyl. For example, $C_1$-$C_8$ alkylene refers to linear or branched alkylene

having 1-8 carbon atoms.

Unless otherwise specified, the term "heteroalkyl" refers to alkyl in which one or more carbon atoms are replaced by a heteroatom.

Unless otherwise specified, the term "heteroalkylene" refers to divalent "heteroalkyl" attached at both ends to other groups.

Unless otherwise specified, the term "phenylene" refers to divalent "phenyl" attached at both ends to other groups.

Unless otherwise specified, the term "cycloalkyl" refers to a fully saturated carbocyclic ring that may exist as a monocyclic, bridged cyclic, or spiro cyclic structure. Preferably, the cycloalkyl contains 3-12 carbon atoms (i.e., C3-12 cycloalkyl), more preferably 3-10 carbon atoms (C3-10 cycloalkyl), and further preferably 3-7 carbon atoms (C3-7 cycloalkyl), 4-6 carbon atoms (C4-6 cycloalkyl), or 5-6 carbon atoms (C5-6 cycloalkyl). Examples of the cycloalkyl include, but are not limited to, cyclopropyl, cyclobutyl, cyclopentyl, cyclohexyl, cycloheptyl, methylcyclopropyl, 2-ethyl-cyclopentyl, dimethylcyclobutyl, norbornyl (bicyclo[2.2.1]heptyl), bicyclo[2.2.2]octyl, adamantyl, etc.

Unless otherwise specified, the term "cycloalkylene" refers to divalent "cycloalkyl" attached at both ends to other groups.

Unless otherwise specified, the term "amino acid residue" refers to the corresponding residue formed after a hydrogen atom is removed from the amine of an amino acid and/or a hydroxyl group is removed from the carboxyl terminus.

Unless otherwise specified, the term "peptide" refers to a short chain of amino acid monomers linked by peptide (amide) bonds.

## BRIEF DESCRIPTION OF THE DRAWINGS

FIG. 1 shows the analytical results of the DAR value of DP001-042.
FIG. 2 shows the analytical results of the DAR value of DP001-043.
FIG. 3 shows the analytical results of the DAR value of 43A-DXd.
FIG. 4 shows the analytical results of the DAR value of 43A-042.
FIG. 5 shows the analytical results of the DAR value of 43B-042.
FIG. 6 shows the analytical results of the DAR value of 43A-043.
FIG. 7 shows the analytical results of the DAR value of 43B-043.
FIG. 8 shows the analytical results of the hydrophobicity of antibody-drug conjugates.
FIG. 9 shows the results of the affinity of the 43A antibody.
FIG. 10 shows the results of the affinity of the 43B antibody.
FIG. 11 shows the results of the affinity of the 43A and 43B antibodies as determined by flow cytometry.
FIGs. 12 and 13 show the results of the inhibitory activity of antibody-drug conjugates against SK-OV-3 and SK-BR-3 cells.
FIGs. 14 and 15 show the results of the inhibitory activity of antibody-drug conjugates against human melanoma A375 cells.
FIG. 16 shows the effects of antibody-drug conjugates on the growth of human lung cancer Calu-6 cell xenograft tumors.
FIG. 17 shows a single crystal diffraction pattern of compound 12.
FIG. 18 shows the effects of antibody-drug conjugates on the growth of human melanoma A375 cell xenograft tumors in mice.
FIG. 19 shows the effects of antibody-drug conjugates on the growth of human lung cancer Calu-6 cell xenograft tumors in mice.
FIG. 20 shows the effect of an antibody-drug conjugate on the growth of human melanoma A375 cell xenograft tumors in mice.
FIG. 21 shows the effects of antibody-drug conjugates on the growth of human esophageal squamous cell carcinoma KYSE-150 xenograft tumors in mice.
FIG. 22 shows the effect of antibody-drug conjugates on the growth of human non-small cell lung cancer SK-MES-1 xenograft tumors in mice.
FIG. 23 shows the effects of antibody-drug conjugates on the growth of human liver cancer Hep3B xenograft tumors in mice.
FIG. 24 shows the effects of antibody-drug conjugates on the growth of human lung cancer Calu-6 cell xenograft tumors in mice.

## DETAILED DESCRIPTION

The present disclosure will be further illustrated with reference to the following specific examples. It should be understood that these examples are merely intended to illustrate the present disclosure rather than limit the scope of the present disclosure. Experimental procedures without specified conditions in the following examples are generally conducted under conventional conditions or conditions recommended by the manufacturers. Unless otherwise defined, all professional and scientific terms used herein have the same meanings as those commonly understood by those of ordinary skill in the art. In addition, any methods and materials similar or equivalent to those described herein can be used in the methods of the present disclosure. The preferred embodiments and materials described herein are for illustrative purposes only.

The compound structures of the present disclosure were determined by nuclear magnetic resonance (NMR) and/or liquid chromatography-mass spectrometry (LC-MS) and/or high performance liquid chromatography (HPLC). The instrument used for NMR determination was a Bruker Avance III 400 MHz nuclear magnetic resonance spectrometer; the instrument used for LC-MS was a SHIMADZU LC-20AD-PDA-LCMS-2020; the instrument used for HPLC was a SHIMADZU LC-20AD-PDA high performance liquid chromatograph.

Starting materials in the examples of the present disclosure are known and commercially available, or may be synthesized by using or following methods known in the art.

Explanation of abbreviations

| | | | |
|---|---|---|---|
| DMF | *N,N*-Dimethylformamide | TBAF | Tetrabutylammonium fluoride |
| TEA | Triethylamine | DMSO | Dimethylsulfoxide |
| THF | Tetrahydrofuran | DIBAL-H | Diisobutylaluminium hydride |
| DIPEA | *N,N*-Diisopropylethylamine | TFAA | Trifluoroacetic anhydride |
| DBU | 1,8-Diazabicyclo[5.4.0]undec-7-ene | Boc | *tert*-Butoxycarbonyl |
| EEDQ | 2-Ethoxy-1-ethoxycarbonyl-1,2-dihydroquinoline | Fmoc | 9-Fluorenylmethyloxycarbonyl |
| PPTS | Pyridinium *p*-toluenesulfonate | TECP | Tris(2-carboxyethyl)phosphine |
| THPTA | Tris(3-hydroxypropyltriazolylmethyl)amine | DCA | Dichloroacetic acid |
| EDCI | 1-(3-Dimethylaminopropyl)-3-ethylcarbodiimide hydrochloride | Py | Pyridine |
| 9-BBN | 9-Borabicyclo[3.3.1]nonane | HOBT | 1-Hydroxybenzotriazole |
| tBuOH | *tert*-Butanol | $NH_4FA$ | Ammonium formate |
| NPCCl | *p*-Nitrobenzoyl chloride | | |

Preparation Examples

I. Preparation of Linker-Drug Compounds

Example 1

4-((30*S*,33*S*,36*S*)-30-(6-(2,5-Dioxo-2,5-dihydro-1*H*-pyrrol-1-yl)hexanamido)-33-isopropyl-36-meth yl-27,31,34-trioxo-2,5,8,11,14,17,20,23-octaoxa-26,32,35-triazaheptatriacontan-37-amido)benzyl ((1*S*,9*S*)-9-ethyl-5-fluoro-9-hydroxy-4-methyl-10,13-dioxo-2,3,9,10,13,15-hexahydro-1*H*,12*H*-benz o[*de*]pyrano[3',4':6,7]indolizino[1,2-*b*]quinolin-1-yl)carbamate (A_10)

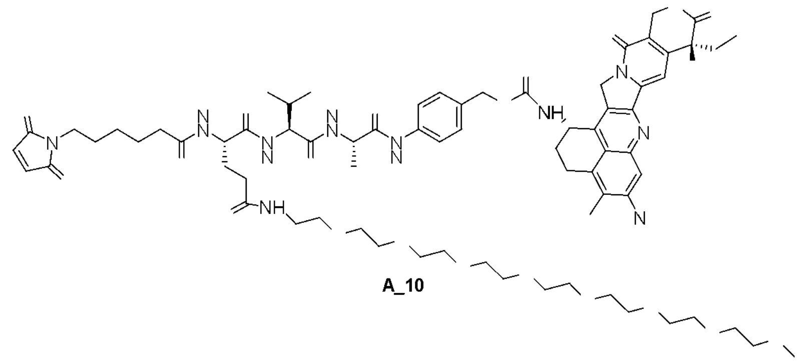

**A_10**

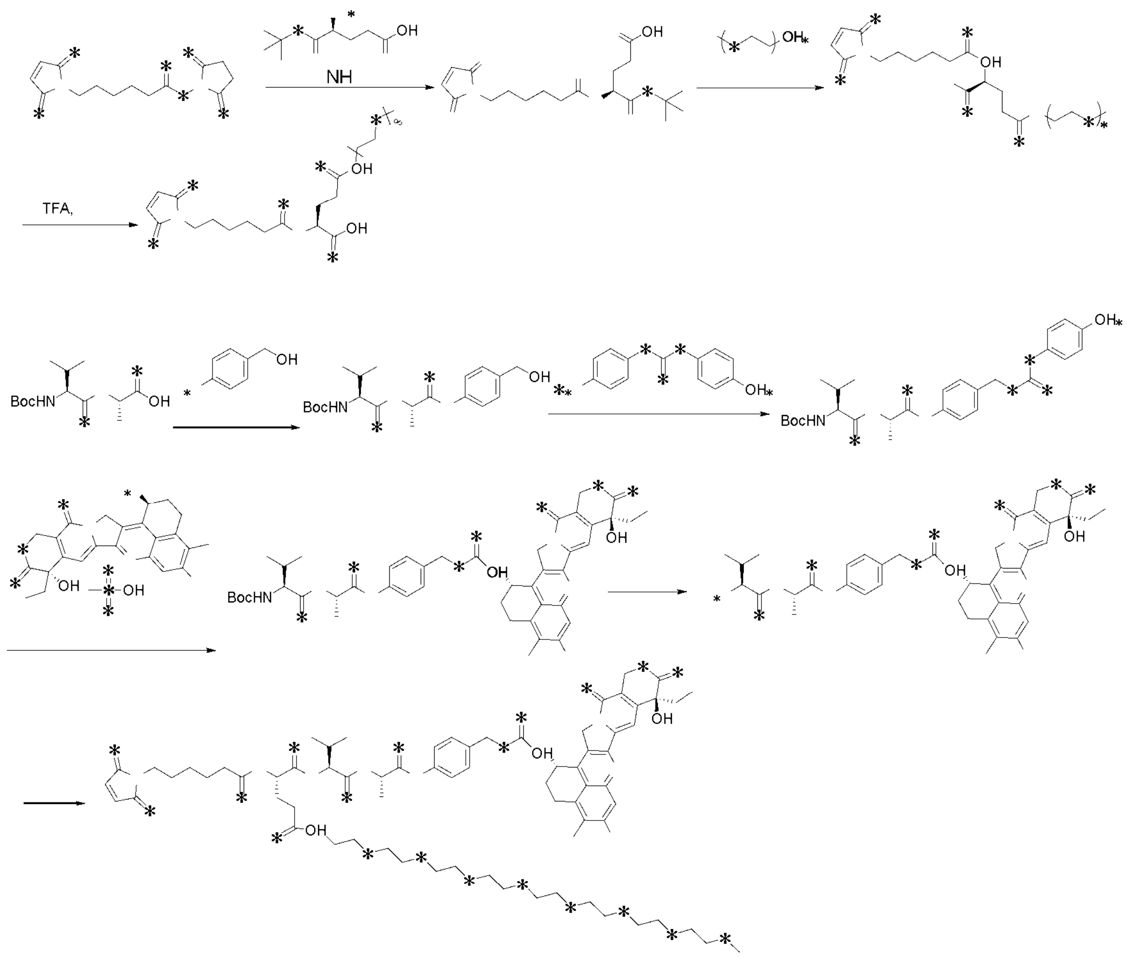


Step 1: Preparation of compound **A_2:**

(1) Q_6 (10 g, 32.5 mmol) and A_1 (6.59 g, 32.5 mmol) were dissolved in DMF (120 mL), and DIEA (12.56 g, 97.4 mmol) was added. The mixture was allowed to react at room temperature for 2 h.

(2) After the reaction was completed as monitored by LC-MS, the reaction liquid was added to 1 mol/L HCl. The resulting mixture was extracted twice with ethyl acetate. The organic phases were combined, washed twice with saturated brine, dried over anhydrous sodium sulfate, and then concentrated to give a crude product (30 g) as a light yellow oil. The above crude product was then mixed with silica gel and subjected to column chromatography (DCM:MeOH = 50:1) to give **A_2** (12 g) as a light yellow oil. LC-MS$[M+H]^+$: m/z 397.1.

Step 2: Preparation of compound **A_4:**

(1) A_2 (1.2 g, 3 mmol) and A_3 (1 g, 3 mmol) were dissolved in DMF (15 mL), and DIEA (1.16 g, 9 mmol) and HATU (1.14 g, 3 mmol) were added. The mixture was allowed to react at room temperature for 2 h.
(2) After the reaction was completed as monitored by LC-MS, the reaction liquid was subjected to reversed-phase column chromatography (water:acetonitrile = 65%:35%) to give a product, which was then lyophilized to give A_4 (1 g) as a light yellow oil. LC-MS$[M+H]^+$: m/z 762.4.

Step 3: Preparation of compound **A_5:**

(1) A_4 (1 g, 3 mmol) was dissolved in DCM:TFA (1:1, 10 mL). The resulting solution was allowed to react at room temperature for 17 h.
(2) After the reaction was completed as monitored by LCMS, the reaction liquid was concentrated to give a crude product (1.2 g) as a yellow oil. LC-MS$[M+H]^+$: m/z 706.4.

Step 4: Preparation of compound **A_12:**

(1) B_4 (1 g, 3.5 mmol) and A_11 (427 mg, 3.5 mmol) were dissolved in DMF (20 mL), and 2,4,6-trimethylpyridine (1.1 g, 10.4 mmol) and (1-cyano-2-ethoxy-2-oxoethylidenaminooxy)dimethylamino-morpholino-carbenium hexafluorophosphate (COMU) (1.49 g, 3.5 mmol) were added. The mixture was allowed to react at room temperature for 2 h.
(2) After the reaction was completed as monitored by LC-MS, the reaction liquid was subjected to reversed-phase column chromatography (water:acetonitrile = 65%:35%) to give a product, which was then lyophilized to give a white solid powder (1.2 g).

Step 5: Preparation of compound **A_14:**

(1) A_12 (500 mg, 1.27 mmol) and A_13 (774 mg, 2.55 mmol) were dissolved in DMF (120 mL), and DIEA (492 mg, 3.81 mmol) was added. The mixture was allowed to react at room temperature for 3 h.
(2) After the reaction was completed as monitored by LC-MS, the reaction liquid was added to 1 mol/L HCl. The resulting mixture was extracted twice with ethyl acetate. The organic phases were combined, washed twice with saturated brine, dried over anhydrous sodium sulfate, then concentrated, mixed with silica gel, and subjected to column chromatography (PE:EA = 1:1) to give a product, which was then concentrated to give a white solid (590 mg).

Step 6: Preparation of compound **A_15:**

(1) Exatecan mesylate (169869-90-3) (300 mg, 0.56 mmol), A_14 (315 mg, 0.56 mmol), and HOBt (153 mg, 1.13 mmol) were dissolved in DMF (9 mL), and DIEA (219 mg, 1.69 mmol) was added. The mixture was allowed to react at room temperature for 2 h.
(2) After the reaction was completed as monitored by LC-MS, the reaction liquid was subjected to reversed-phase column chromatography (water:acetonitrile = 40%:60%) to give a product, which was then lyophilized to give a yellow solid (510 mg). LC-MS$[M+H]^+$: m/z 855.4.

Step 7: Preparation of compound **A_16:**

(1) A_15 (300 mg, 0.56 mmol) was dissolved in DCM:TFA (1:1, 6 mL). The resulting solution was allowed to react at room temperature for 2 h.

(2) After the reaction was completed as monitored by LC-MS, the reaction liquid was poured into diethyl ether, resulting in the precipitation of a yellow solid. The mixture was then centrifuged to give a crude product (150 mg) as a yellow solid. LC-MS$[M+H]^+$: m/z 755.2.

Step 8: Preparation of compound **A_10:**

(1) A_16 (100 mg, 0.14 mmol) and A_5 (220 mg, 0.28 mmol) were dissolved in DMF (4 mL), and 2,4,6-trimethylpyridine (54 mg, 0.5 mmol) and HATU (85 mg, 0.27 mmol) were added. The mixture was allowed to react at room temperature for 2 h.
(2) After the reaction was completed as monitored by LC-MS, the reaction liquid was subjected to preparative chromatography. The prepared liquid was then lyophilized to give A_10 (22 mg) as a yellow solid. LC-MS[M+H]+: m/z 1443.6.

Example 2

(*S*)-2-(6-(2,5-Dioxo-2,5-dihydro-1*H*-pyrrol-1-yl)hexanamido)-*N*1-((*S*)-1-(((*S*)-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6 ,7]indolizino[1,2-*b*]quinolin-1-yl)amino)-1-oxopropan-2-yl)amino)-3-methyl-1-oxobutan-2-yl)-*N*5-( 2,5,8,11,14,17,20,23-octaoxapentacosan-25-yl)pentanediamide (B_3)

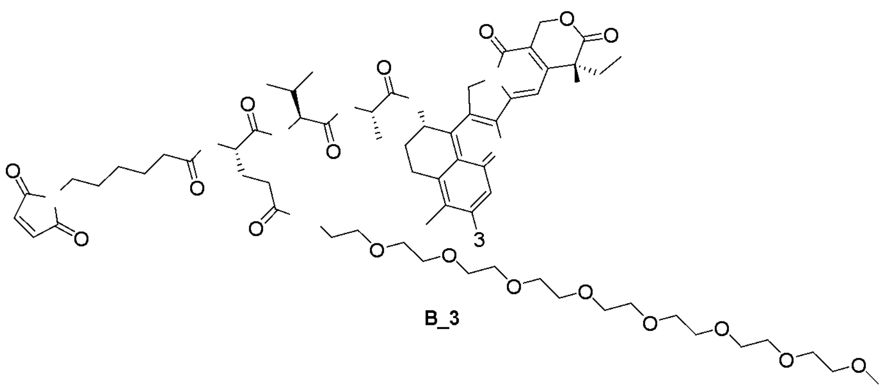

B_3

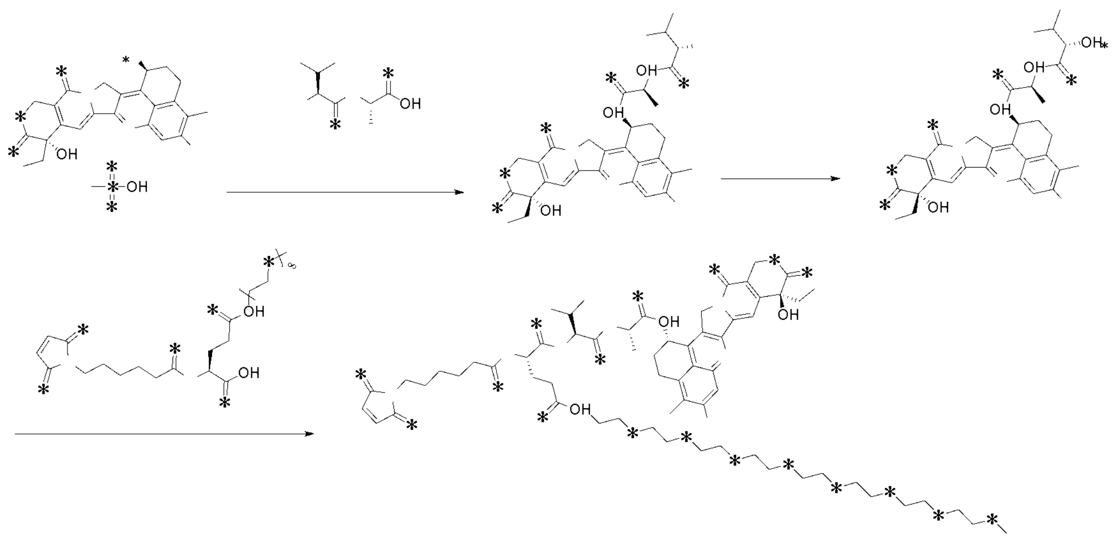

Step 1: Preparation of compound **B_5:** (1) Exatecan mesylate (169869-90-3) (300 mg, 0.56 mmol) and B_4 (163 mg, 0.56 mmol) were dissolved in DMF (3 mL), and 2,4,6-trimethylpyridine (205 mg, 1.69 mmol) and COMU (242 mg, 0.56

mmol) were added. The mixture was allowed to react at room temperature for 0.5 h.
(2) After the reaction was completed as monitored by LC-MS, the reaction liquid was added to water (40 mL), resulting in the precipitation of a blue-gray solid. The mixture was then filtered, and the filter cake was washed with clear water. The solid was then lyophilized to give a crude product (330 mg) as a blue-gray solid. LC-MS[M+H]+: m/z 706.2.

Step 2: Preparation of compound **B_6:**

(1) B_5 (330 mg, 0.47 mmol) was dissolved in DCM:TFA (1:1, 10 mL). The resulting solution was allowed to react for 20 min in an ice bath.
(2) After the reaction was completed as monitored by LC-MS, the reaction liquid was poured into diethyl ether, resulting in the precipitation of a blue-gray solid. The solid was isolated by centrifugation and subjected to preparative chromatography. The prepared liquid was then lyophilized to give a yellow solid (150 mg). LC-MS[M+H]+: m/z 606.2.

Step 3: Preparation of compound **B_3:**

(1) **B_6** (50 mg, 0.08 mmol) and A_5 (115 mg, 0.16 mmol) were dissolved in DMF (3 mL), and 2,4,6-trimethylpyridine (26.5 mg, 0.25 mmol) and HATU (47 mg, 0.125 mmol) were added. The mixture was allowed to react at room temperature for 3 h.
(2) After the reaction was completed as monitored by LC-MS, the reaction liquid was subjected to preparative chromatography. The prepared liquid was then lyophilized to give a yellow solid powder (25 mg).

Example 3: Preparation of Compound 045

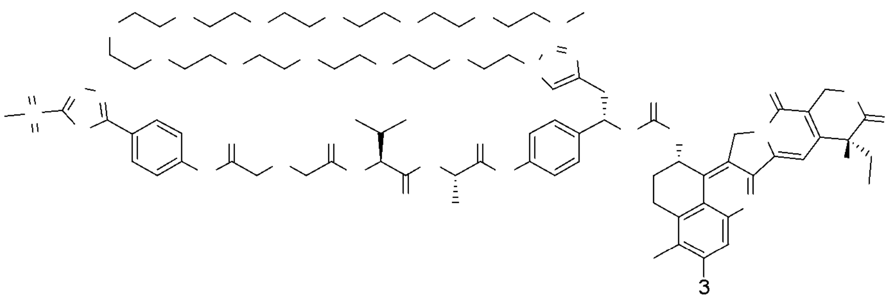

3

1. Preparation of intermediate compound 8

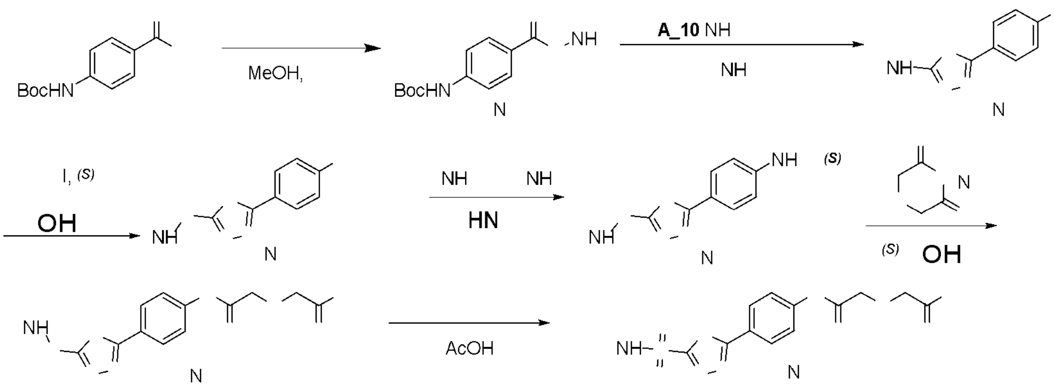


Compound **1** (47.5 g, 189 mmol) and methanol (250 mL) were added to a reaction flask, and 80% hydrazine

hydrate (35.4 g, 567 mmol) was slowly added at room temperature. The mixture was warmed to 70 °C and refluxed for 6 h. After cooling, a white crystal was precipitated, and the mixture was subjected to suction filtration. The remaining solid was washed with methanol (20 mL × 3) to give **2** (47.47 g, yield: 100%) as a white solid. MS (ESI): $(M+H)^+$, calculated 252.1, found 252.2.

Compound **2** (47.4 g, 189 mmol), potassium hydroxide (12.7 g, 227 mmol), and ethanol (400 mL) were added to a reaction flask, and the mixture was stirred for dissolution at room temperature. Carbon disulfide (17 g, 283 mmol) was slowly added, and the mixture was warmed to 100 °C and refluxed for 5 h. The solvent was removed by concentration under reduced pressure, and then water (50 mL) was added. The mixture was adjusted to pH 6 with diluted hydrochloric acid and extracted with dichloromethane (100 mL × 3). The organic phases were combined, washed with saturated brine, dried over anhydrous sodium sulfate, and filtered. The filtrate was then concentrated under reduced pressure, and the residue was purified by silica gel chromatography to give compound **3** (49.9 g, yield: 90%). MS (ESI): $(M+H)^+$, calculated 294.1, found 294.3.

Compound **3** (5.86 g, 20 mmol), triethylamine (2.42 g, 24 mmol), and tetrahydrofuran (36 mL) were added to a reaction flask, and iodomethane (3.12 g, 22 mmol) was added to the above reaction liquid. The reaction was completed after the mixture was stirred for reaction at 25 °C for 1.5 h. The solvent was removed by concentration under reduced pressure, and the residue was purified by silica gel chromatography to give compound **4** (4.05 g, yield: 66%). MS (ESI): $(M+H)^+$, calculated 308.1, found 308.2.

Compound **4** (3.3 g, 10.6 mmol) and ethyl acetate (20 mL) were added to a reaction flask, and a solution of hydrogen chloride in ethyl acetate (2.7 mL, 4 M) was added dropwise at 25 °C. The mixture was then allowed to react for 6 h, and the solvent was removed by concentration under reduced pressure to give a crude product of **5,** which was directly used in the next step.

The crude product of **5,** diglycolic anhydride **6** (1.35 g, 11.7 mmol), triethylamine (2.14 g, 21.2 mmol), and tetrahydrofuran (30 mL) were added to a reaction flask. The mixture was allowed to react at 25 °C for 1.5 h, and the solvent was removed by concentration under reduced pressure. Diethyl ether was added to the residue, and the resulting mixture was subjected to suction filtration and then washed with water and diethyl ether to give compound 7 (3.4 g, yield: 99%). MS (ESI): $(M+H)^+$, calculated 324.1, found 324.2.

Compound **7** (3.4 g, 10.5 mmol) and glacial acetic acid (20 mL) were added to a reaction flask. After dissolution, potassium permanganate (2.48 g, 15.7 mmol) was added at 0 °C. The mixture was warmed to 25 °C and allowed to react for 1 h. A saturated sodium sulfite solution was added until the solution became colorless, and then the solution was extracted with ethyl acetate (100 mL × 3). The organic phases were combined, washed with saturated brine, dried over anhydrous sodium sulfate, and filtered. The filtrate was then concentrated under reduced pressure, and the residue was purified by silica gel chromatography to give compound **8** (2.4 g, yield: 65%). MS (ESI): $(M+H)^+$, calculated 356.0, found 356.0.

2. Preparation of compound 045

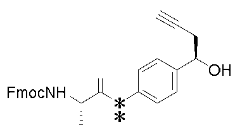

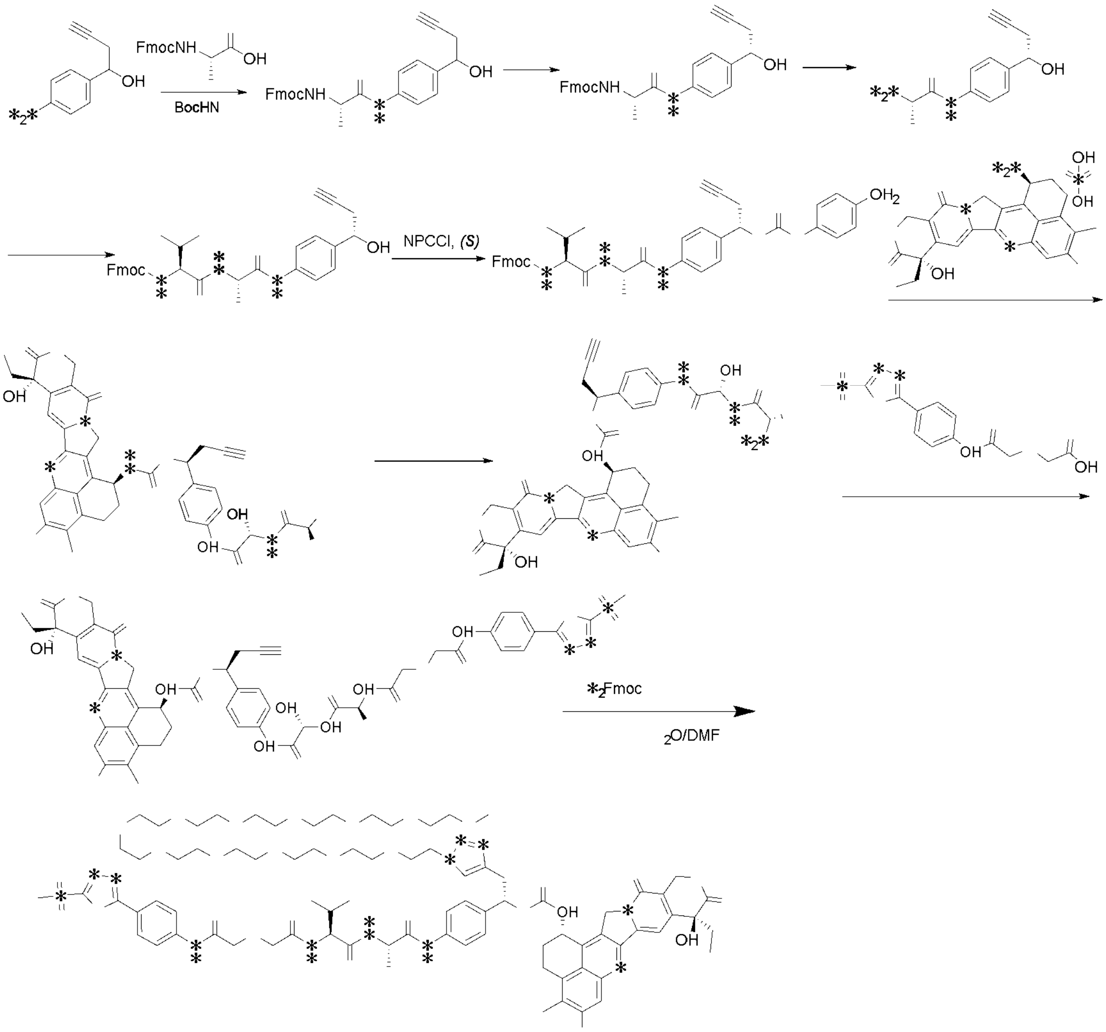


1-(4-Aminophenyl)-3-butyn-1-ol (4997 mg, 31 mmol), Fmoc-Ala-OH (9651 mg, 31 mmol), EEDQ (11498 mg, 46.5 mmol), and extra-dry DCM (90 mL) were added to a reaction flask at 0 °C. After 3 h of reaction, the solvent was removed by concentration under reduced pressure, and the residue was added with MTBE (500 mL) for slurrying to give compound **10** (9007 mg, yield: 64%, *dr* = 1:1). MS (ESI): $(M+H)^+$, calculated 455.2, found 455.2.

Chiral resolution of intermediate **10:** Compound **10** (50 g) was resolved by SFC to give **11** (19 g, retention time: 13.55 min) and **12** (20 g, retention time: 16.29 min). SFC resolution method: column model: DAICEL CHIRALCEL OD (250 mm-50 mm, 10 μm); mobile phase: A: $CO_2$, B: $CO_2$-ACN/i-PrOH (0.1% $NH_3H_2O$); isocratic elution: B in A for 50%; flow rate: 200 mL/min; detector: PDA; column temperature: 25 °C; back pressure: 100 Bar.

HPLC method: instrument information: Thermo liquid chromatograph (ADC-U3000-01); chromatographic column: CHIRALPAK®ID (4.6 × 150 mm, 5 μm); column temperature: 25 °C; sample tray temperature: 25 °C; mobile phase: A: 10 mM $NH_4FA$; B: ACN; flow rate: 0.8 mL/min; detection wavelength: 254 nm; sample injection volume: 2 μL.

Gradient conditions:

| Time (min) | A% | B% |
|---|---|---|
| 0 | 40.0 | 60.0 |
| 30.000 | 40.0 | 60.0 |

The structure and configuration of compound **12,** confirmed by X-RAY, are as follows, and its single crystal diffraction pattern is shown in FIG. 17.

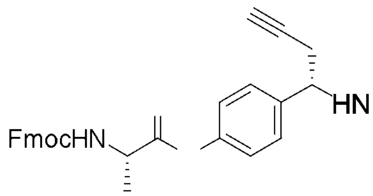

**12** (3632 mg, 8 mmol) and THF (commercially available, 90 mL) were added to a reaction flask at 0 °C. The mixture was stirred while DBU (1215 mg, 8 mmol) was slowly added. After half an hour of reaction, the mixture was warmed to room temperature. After the starting materials disappeared as monitored by TLC, the solvent was removed by concentration under reduced pressure, and the residue was purified by silica gel chromatography (DCM/MeOH 95:5) to give compound **13** (1707 mg, yield: 92%). MS (ESI): $(M+H)^+$, calculated 233.1, found 233.2.

Compound **13** (1624 mg, 7 mmol), Fmoc-Val-OSu (362 mg, 8.4 mmol), and *N*,*N*-dimethylformamide (150 mL) were added to a reaction flask. The mixture was stirred while DIEA (1158 μL, 7 mmol) was slowly added dropwise. The mixture was allowed to react at 25 °C for 12 h. The solvent was removed by concentration under reduced pressure. EA (50 mL) and PE (50 mL) were added for slurrying, resulting in the precipitation of a white solid. This procedure was repeated three times to give compound **14.** MS (ESI): $(M+H)^+$, calculated 554.3, found 554.4. Compound **14** (1661 mg, 3 mmol), 4-nitrophenyl chloroformate (1206 mg, 6 mmol), and THF (150 mL) were added to a reaction flask, and the mixture was stirred while Py (474 μL, 6 mmol) was added dropwise. The mixture was allowed to react at 65 °C for 1 h. The solvent was removed by concentration under reduced pressure, and the residue was purified by silica gel chromatography (DCM/MeOH 98:2) to give compound **15** (1831 mg, yield: 85%). MS (ESI): $(M+H)^+$, calculated 719.8, found 719.9.

Compound **15** (1436 mg, 2 mmol), exatecan mesylate **16** (1168.2 mg, 2.2 mmol), and N,N-dimethylformamide (100 mL) were added to a reaction flask, and the mixture was stirred while DIEA (695 μL, 4 mmol) was slowly added dropwise. The mixture was allowed to react at 25 °C for 20 h. The solvent was removed by concentration under reduced pressure, and the residue was purified by silica gel chromatography (DCM/MeOH 95:5) to give compound **17** (1664 mg, yield: 82%). MS (ESI): $(M+H)^+$, calculated 1015.4, found 1015.1.

**17** (1522 mg, 1.5 mmol) and THF (commercially available, 40 mL) were added to a reaction flask at 0 °C. The mixture was stirred while DBU (228 mg, 1.5 mmol) was slowly added. After half an hour of reaction, the mixture was warmed to room temperature and then allowed to react for 40 min until the starting materials disappeared as detected by TLC. The reaction liquid was concentrated under vacuum to remove THF, and DCM (15 mL) and PE (300 mL) were added, yielding a large amount of yellowish-green solid. The solid **18** (1177 mg, yield: 99%) was then obtained by filtration through a Buchner funnel. MS (ESI): $(M+H)^+$, calculated 793.3, found 793.6.

Compound **18** (1031 mg, 1.3 mmol), **8** (554 mg, 1.56 mmol), EDCI (498 mg, 2.6 mmol), HOBT (263 mg, 1.95 mmol), and N,N-dimethylformamide (20 mL) were added to a reaction flask. The mixture was allowed to react at 25 °C for 1 h. The solvent was removed by concentration under reduced pressure, and the residue was purified by C18 (60% ACN/0.05% formic acid in $H_2O$) and lyophilized to give compound **19** (1322 mg, yield: 90%). MS (ESI): $(M+H)^+$, calculated 1130.4, found 1130.7.

Compound **19** (903 mg, 0.8 mmol), compound **20** (702 mg, 1.2 mmol), tris(3-hydroxypropyltriazolylmethyl)amine (34.7 mg, 0.08 mmol), and copper(I) bromide (11.4 mg, 0.08 mmol) were added to a reaction flask. The mixture was purged three times with nitrogen, and a mixture of THF/DMF/$H_2O$ (3.5 mL:0.6 mL:0.4 mL) was added. The resulting mixture was allowed to react at 25 °C for 0.5 h. The residue was purified by preparative C18 (62% ACN/0.05% formic acid in $H_2O$) to give compound **045** (1276 mg, yield: 93%). MS (ESI): $(M+H)^+$, calculated 1715.7, found 1716.2. $^1H$ NMR (600 MHz, DMSO) δ 10.71 (s, 1H), 10.08 (s, 1H), 8.48 (d, *J* = 6.7 Hz, 1H), 8.17 (d, *J* = 8.6 Hz, 3H), 8.09 (d, *J* = 8.8 Hz, 1H), 8.03 (d, *J* = 8.7 Hz, 2H), 7.89 (s, 1H), 7.81 (d, *J* = 10.8 Hz, 1H), 7.66 (d, *J* = 8.4 Hz, 2H), 7.42 - 7.36 (m, 3H), 6.61 (s, 1H), 6.02 (t, *J* = 6.8 Hz, 1H), 5.55 (s, 2H), 5.38 -5.26 (m, 3H), 4.55 - 4.47 (m, 3H), 4.46 - 4.41 (m, 1H), 4.36 (q, *J* = 15.6 Hz, 2H), 4.28 (s, 2H), 3.80 (s, 5H), 3.62 - 3.55 (m, 36H), 3.52 - 3.47 (m, 9H), 3.44 - 3.36 (m, 2H), 3.30 -2.29 (m, 1H), 3.19 - 3.09 (m, 1H), 2.42 (s, 3H), 2.28 - 2.19 (m, 1H), 2.17 (s, 3H), 2.17 - 2.10 (m,1H), 2.04 - 1.89 (m, 2H), 1.42 (d, *J* = 7.0 Hz, 3H), 1.02 (d, *J* = 6.7 Hz, 3H), 0.98 (t, *J* = 7.2 Hz, 6H).

II. Preparation of Antibody-Drug Conjugate (ADC)

Common procedure A: Purification of antibody-drug conjugate

A 1 mL Mabselect SuRe pre-packed chromatographic column was equilibrated with PBS 7.0/EDTA solution (10 mM PB, 137 mM NaCl, 5 mM EDTA, pH 7.0) on an AKTA system, and the antibody-drug conjugate was loaded using a loading loop. After re-equilibration with 10-20 mL of PBS 7.0/EDTA, elution was carried out using an acetate buffer at pH 3.5. The eluate was then neutralized by adding 1/10 volume of a sodium citrate neutralization solution to obtain the purified antibody-drug conjugate.

Common procedure B: Determination of drug-to-antibody ratio (DAR) of antibody-drug conjugate

Dithiothreitol at a final concentration of 20 mM was added to the antibody-drug conjugate, and the mixture was placed in a water bath at 37 °C for 30 min. This process cleaved the interchain disulfide bonds of the antibody-drug conjugate. The resulting sample was then used for HPLC analysis. The HPLC system used was an Agilent Technologies 1260 Infinity HPLC; the chromatographic column used was PLRP-S (particle size: 5 μm; 2.1 mm × 50 mm; Agilent Technologies); the column temperature was 80 °C; mobile phase A was 0.1% trifluoroacetic acid (TFA) in water, and mobile phase B was 0.1% trifluoroacetic acid (TFA) in acetonitrile. The loading amount was 10 μL, and the gradient program was as follows: 27%-27% for 0-3 min, 27%-35% for 3-8 min, 35%-43% for 8-25 min, 43%-95% for 25-26 min, 95%-95% for 26-31 min, 95%-27% for 31-31.5 min, 27%-27% for 31.5-40 min. Relative to the unconjugated light chain (L0) and heavy chain (H0), the drug-conjugated light chain (light chain conjugated with one drug, L1) and heavy chains (heavy chain conjugated with one drug, H1; heavy chain conjugated with two drugs, H2; heavy chain conjugated with three drugs, H3) exhibited increased hydrophobicity as the number of conjugated drugs increased. Therefore, elution occurred in the following order: L0, L1, H0, H1, H2, H3. The DAR value was calculated based on the peak area at 280 nm.

$$\mathrm{DAR} = \left(\frac{\mathrm{L1}}{\mathrm{L0} + \mathrm{L1}} \times 1 + \frac{\mathrm{H1}}{\mathrm{H0} + \mathrm{H1} + \mathrm{H2} + \mathrm{H3}} \times 1 + \frac{\mathrm{H2}}{\mathrm{H0} + \mathrm{H1} + \mathrm{H2} + \mathrm{H3}} \times 2 + \frac{\mathrm{H3}}{\mathrm{H0} + \mathrm{H1} + \mathrm{H2} + \mathrm{H3}} \times 3\right) \times 2$$

Example 4: Preparation of Antibody-Drug Conjugate DP001-042

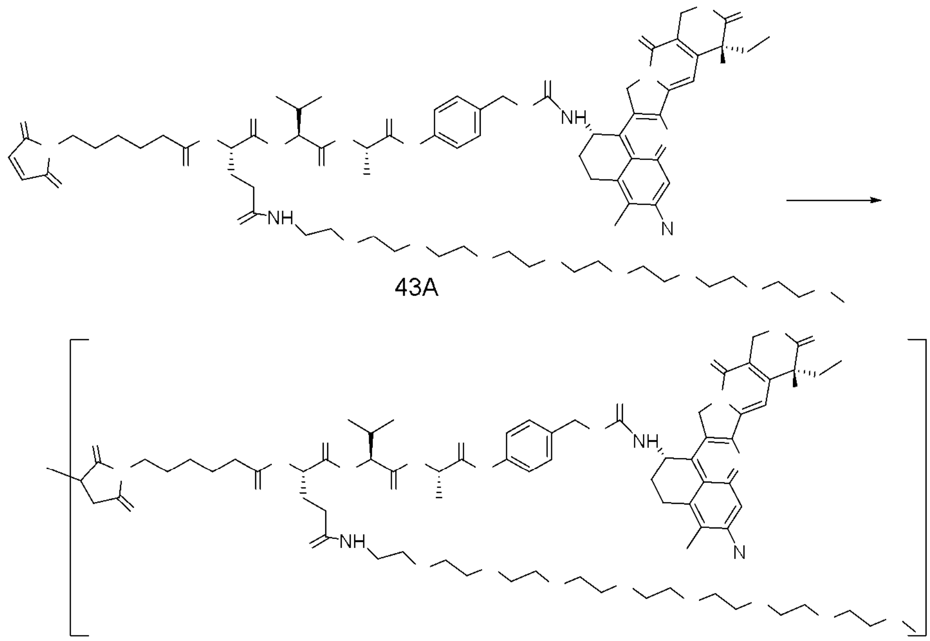

43A

**Engineering procedure 1:**

Reduction of antibody:

The buffer for the DP001 antibody prepared in the laboratory was replaced with PBS 7.0/EDTA to give an antibody concentration of 10 mg/mL. The resulting solution (1.0 mL) was placed into a 1.5 mL EP tube, and then a 100 mM aqueous TCEP (J&K Scientific) solution (8.16 μL; 12 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of **A_10** in DMSO (68.0 μL; 10 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous N-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to the purification described in common procedure A to give a solution containing compound DP001-042.

Determination of drug-to-antibody ratio (DAR) of antibody-drug conjugate:

DAR determination was performed using common procedure B. The results, as shown in FIG. 1, indicate that the DAR value of DP001-042 was 8.0.

Example 5: Preparation of Antibody-Drug Conjugate DP001-043

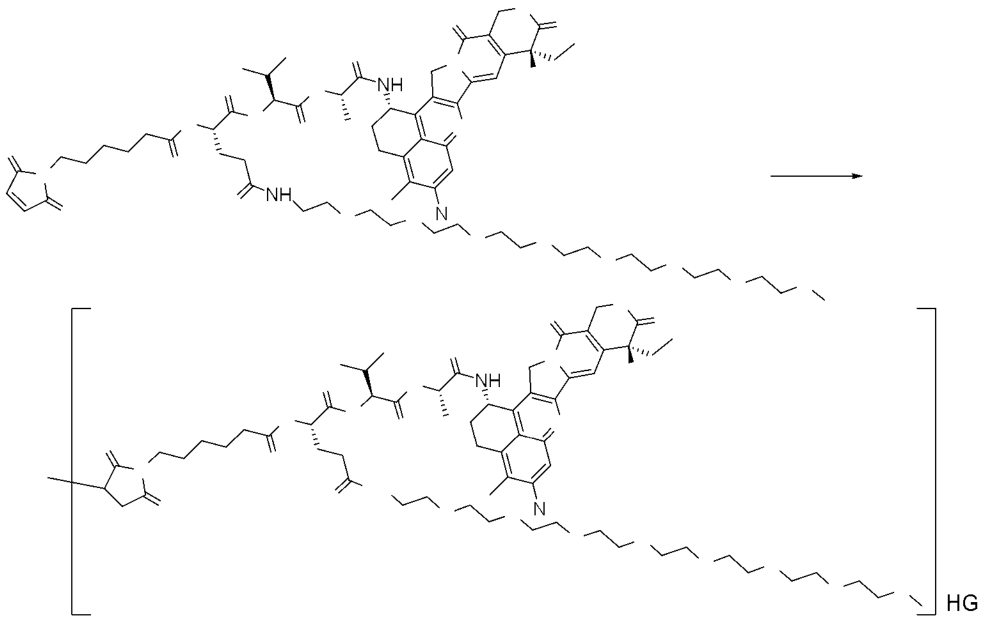

**Engineering procedure 1:**

Reduction of antibody:

The buffer for the DP001 antibody prepared in the laboratory was replaced with PBS 7.0/EDTA to give an antibody concentration of 10 mg/mL. The resulting solution (1.0 mL) was placed into a 1.5 mL EP tube, and then a 100 mM aqueous TCEP (J&K Scientific) solution (8.16 μL; 12 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of **B_3** in DMSO (68.0 μL; 10 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous *N*-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to the purification described in common procedure A to give a solution containing compound DP001-043.

Determination of drug-to-antibody ratio (DAR) of antibody-drug conjugate:

DAR determination was performed using common procedure B. The results, as shown in FIG. 2, indicate that the DAR value of DP001-043 was 8.0.

Example 6: Preparation of Antibody-Drug Conjugate 43A-DXd

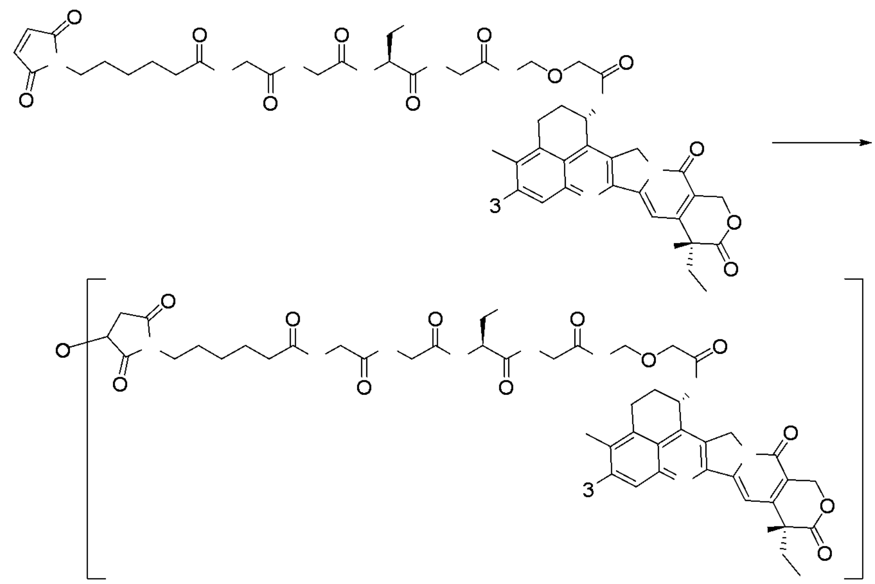

**Engineering procedure 1:**

Reduction of antibody:

The buffer for the 43A antibody prepared in the laboratory was replaced with PBS 7.0/EDTA to give an antibody concentration of 10 mg/mL. The resulting solution (1.0 mL) was placed into a 1.5 mL EP tube, and then a 100 mM aqueous TCEP (J&K Scientific) solution (8.16 μL; 12 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of deruxtecan (Shanghai Haoyuan Chemexpress Co., Ltd.) in DMSO (68.0 μL; 10 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous N-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to the purification described in common procedure A to give a solution containing compound 43A-DXd.

Determination of drug-to-antibody ratio (DAR) of antibody-drug conjugate:

DAR determination was performed using common procedure B. The results, as shown in FIG. 3, indicate that the DAR value of 43A-DXd was 7.4.

Example 7: Preparation of Antibody-Drug Conjugate 43A-042

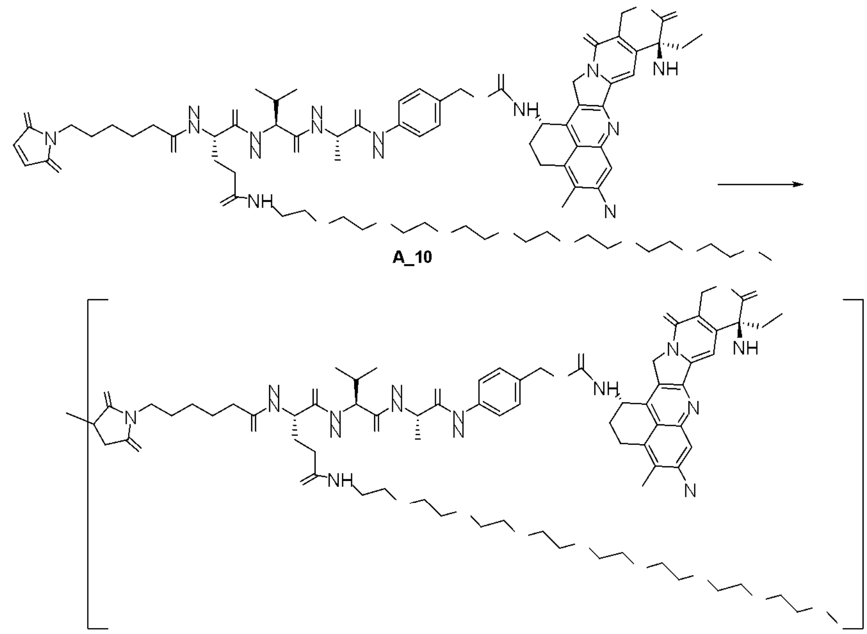

A_10

**Engineering procedure 1:**

Reduction of antibody:

The buffer for the 43A antibody prepared in the laboratory was replaced with PBS 7.0/EDTA to give an antibody concentration of 10 mg/mL. The resulting solution (1.0 mL) was placed into a 1.5 mL EP tube, and then a 100 mM aqueous TCEP (J&K Scientific) solution (8.16 μL; 12 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of **A_10** in DMSO (68.0 μL; 10 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous N-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to the purification described in common procedure A to give a solution containing compound 43A-042.

Determination of drug-to-antibody ratio (DAR) of antibody-drug conjugate:

DAR determination was performed using common procedure B. The results, as shown in FIG. 4, indicate that the DAR value of 43A-042 was 7.6.

Example 8: Preparation of Antibody-Drug Conjugate 43B-042

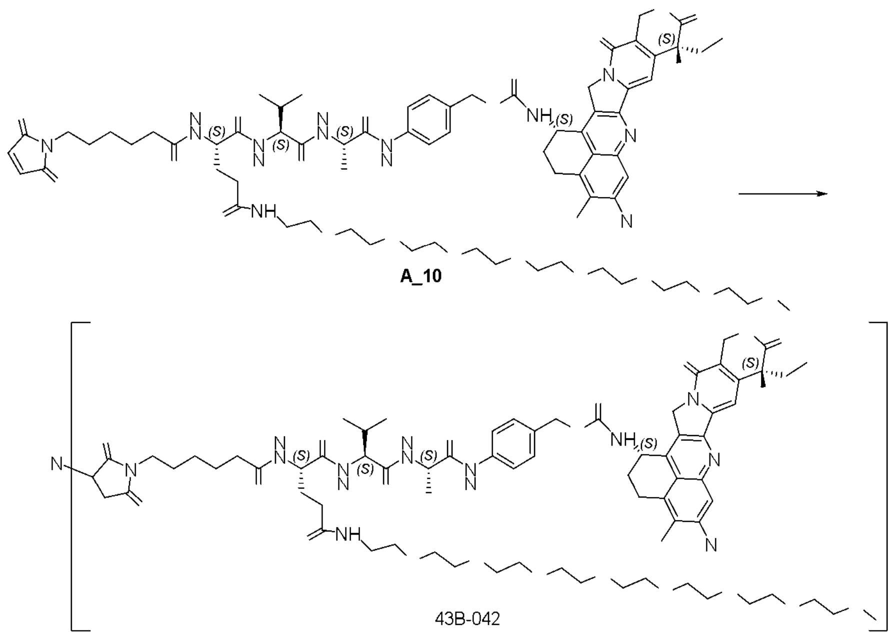

**A_10**

43B-042

**Engineering procedure 1:**

Reduction of antibody:

The buffer for the 43B antibody prepared in the laboratory was replaced with PBS 7.0/EDTA to give an antibody concentration of 10 mg/mL. The resulting solution (1.0 mL) was placed into a 1.5 mL EP tube, and then a 100 mM aqueous TCEP (J&K Scientific) solution (8.16 μL; 12 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of **A_10** in DMSO (68.0 μL; 10 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous N-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to the purification described in common procedure A to give a solution containing compound 43B-042.

Determination of drug-to-antibody ratio (DAR) of antibody-drug conjugate:

DAR determination was performed using common procedure B. The results, as shown in FIG. 5, indicate that the DAR value of 43B-042 was 5.9.

Example 9: Preparation of Antibody-Drug Conjugate 43A-043

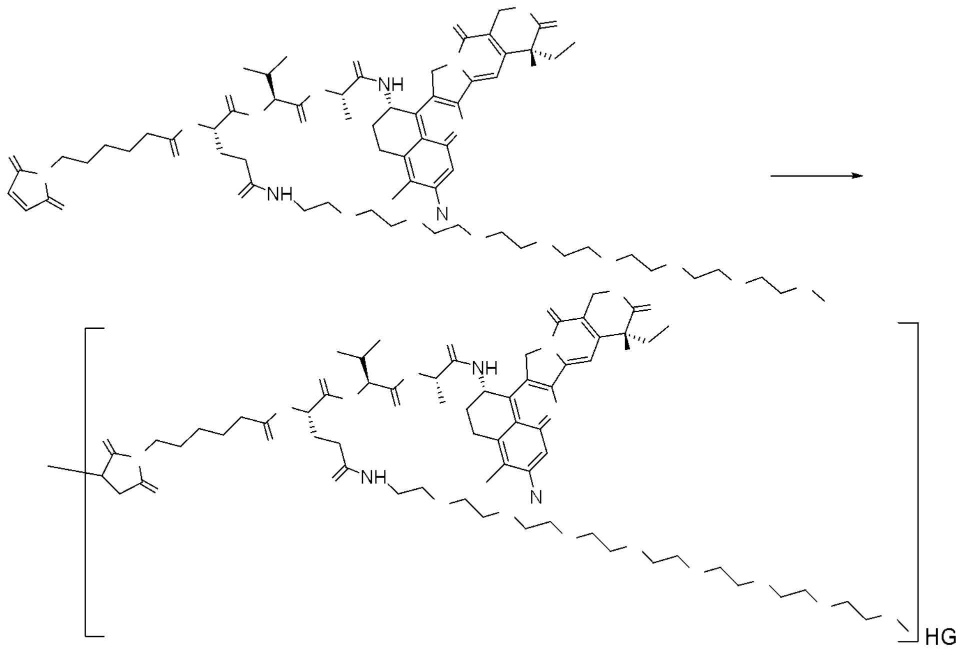


**Engineering procedure 1:**

Reduction of antibody:

The buffer for the 43A antibody prepared in the laboratory was replaced with PBS 7.0/EDTA to give an antibody concentration of 10 mg/mL. The resulting solution (1.0 mL) was placed into a 1.5 mL EP tube, and then a 100 mM aqueous TCEP (J&K Scientific) solution (8.16 μL; 12 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of B_3 in DMSO (68.0 μL; 10 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous N-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to the purification described in common procedure A to give a solution containing compound 43A-043.

Determination of drug-to-antibody ratio (DAR) of antibody-drug conjugate:

DAR determination was performed using common procedure B. The results, as shown in FIG. 6, indicate that the DAR value of 43A-043 was 7.8.

Example 10: Preparation of Antibody-Drug Conjugate 43B-043

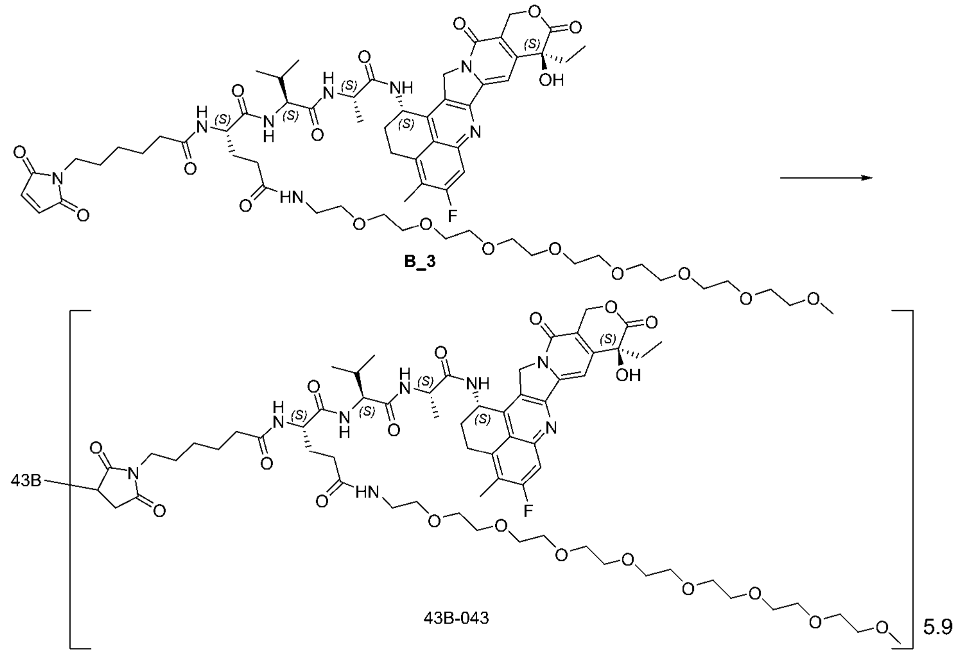

B_3

43B-043

5.9

**Engineering procedure 1:**

Reduction of antibody:

The buffer for the 43B antibody prepared in the laboratory was replaced with PBS 7.0/EDTA to give an antibody concentration of 10 mg/mL. The resulting solution (1.0 mL) was placed into a 1.5 mL EP tube, and then a 100 mM aqueous TCEP (J&K Scientific) solution (8.16 μL; 12 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of B_3 in DMSO (68.0 μL; 10 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous N-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to the purification described in common procedure A to give a solution containing compound 43B-043.

Determination of drug-to-antibody ratio (DAR) of antibody-drug conjugate:

DAR determination was performed using common procedure B. The results, as shown in FIG. 7, indicate that the DAR value of 43B-043 was 5.9.

Example 11: Preparation of Antibody-Drug Conjugate 43A-JSSW-4

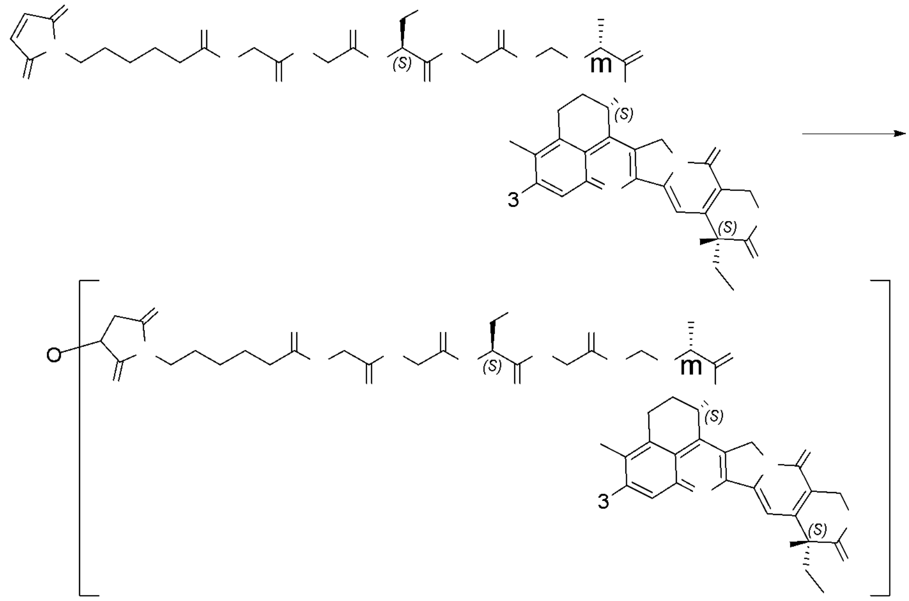

**Engineering procedure 1:**

Reduction of antibody:

The buffer for the 43A antibody prepared in the laboratory was replaced with PBS 7.0/EDTA to give an antibody concentration of 10 mg/mL. The resulting solution (1.0 mL) was placed into a 1.5 mL EP tube, and then a 10 mM aqueous TCEP (J&K Scientific) solution (15.64 μL; 2.3 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of JSSW-001 (Shanghai Haoyuan Chemexpress Co., Ltd.) in DMSO (30.6 μL; 4.5 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous N-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to the purification described in common procedure A to give a solution containing compound 43A-JSSW-4.

Determination of drug-to-antibody ratio (DAR) of antibody-drug conjugate:

DAR determination was performed using common procedure B. The results indicate that the DAR value of 43A-JSSW-4 was 3.8.

Example 12: Preparation of Antibody-Drug Conjugate 43A-JSSW-6

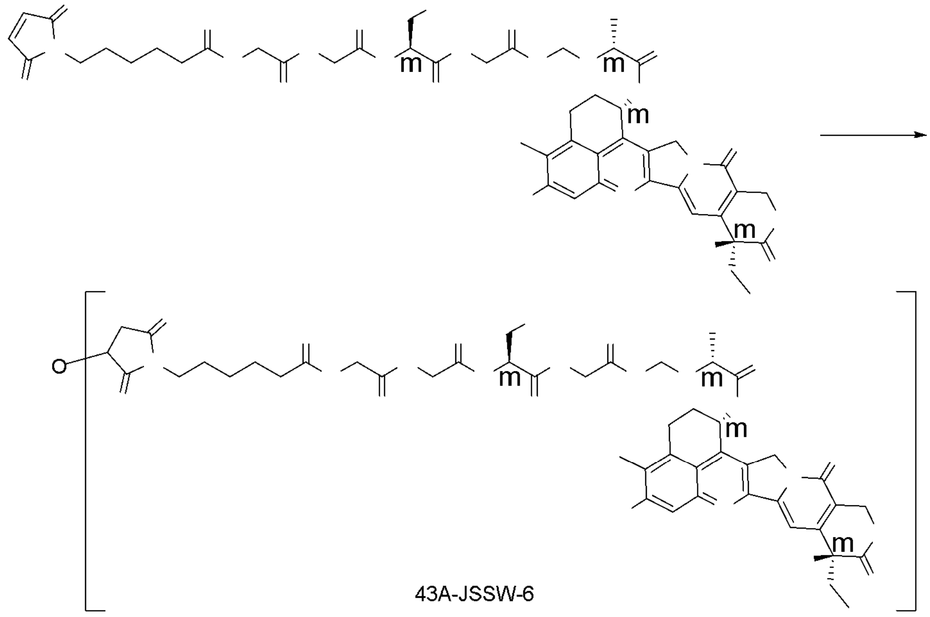

43A-JSSW-6

**Engineering procedure 1:**

Reduction of antibody:

The buffer for the 43A antibody prepared in the laboratory was replaced with PBS 7.0/EDTA to give an antibody concentration of 10 mg/mL. The resulting solution (1.0 mL) was placed into a 1.5 mL EP tube, and then a 10 mM aqueous TCEP (J&K Scientific) solution (22.44 μL; 3.3 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of JSSW-001 (Shanghai Haoyuan Chemexpress Co., Ltd.) in DMSO (43.5 μL; 6.4 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous *N*-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to the purification described in common procedure A to give a solution containing compound 43A-JSSW-6.

Determination of drug-to-antibody ratio (DAR) of antibody-drug conjugate:

DAR determination was performed using common procedure B. The results indicate that the DAR value of 43A-JSSW-6 was 5.8.

Example 13: Preparation of Antibody-Drug Conjugate 43A-JSSW-8

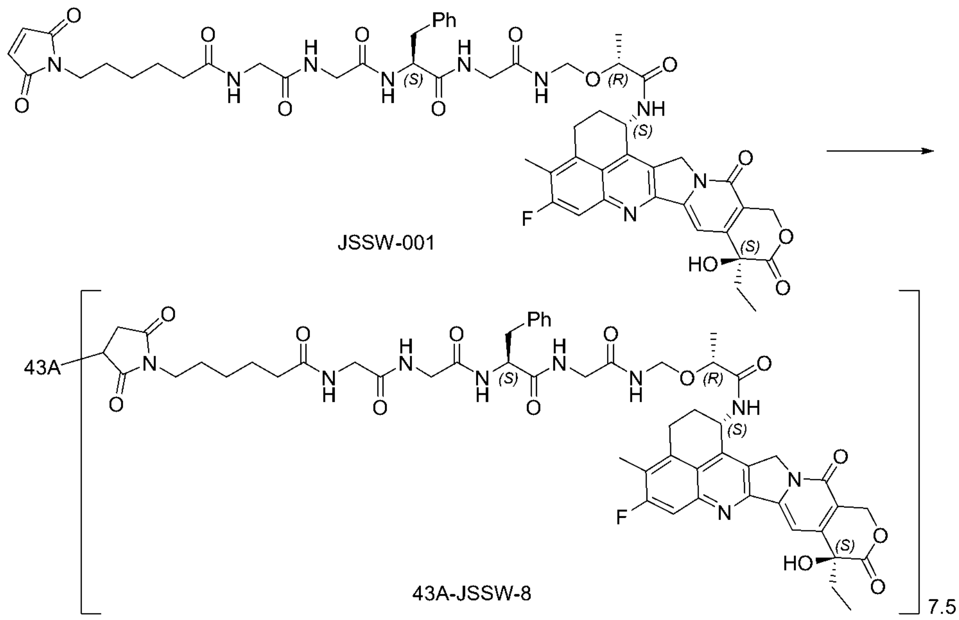

JSSW-001

43A-JSSW-8

**Engineering procedure 1:**

Reduction of antibody:

The buffer for the 43A antibody prepared in the laboratory was replaced with PBS 7.0/EDTA to give an antibody concentration of 10 mg/mL. The resulting solution (1.0 mL) was placed into a 1.5 mL EP tube, and then a 100 mM aqueous TCEP (J&K Scientific) solution (8.16 μL; 12 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of JSSW-001 (Shanghai Haoyuan Chemexpress Co., Ltd.) in DMSO (68.0 μL; 10 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous N-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to the purification described in common procedure A to give a solution containing compound 43A-JSSW-8.

Determination of drug-to-antibody ratio (DAR) of antibody-drug conjugate:

DAR determination was performed using common procedure B. The results indicate that the DAR value of 43A-JSSW-8 was 7.5.

Example 14: Preparation of Antibody-Drug Conjugate 43B-JSSW-6

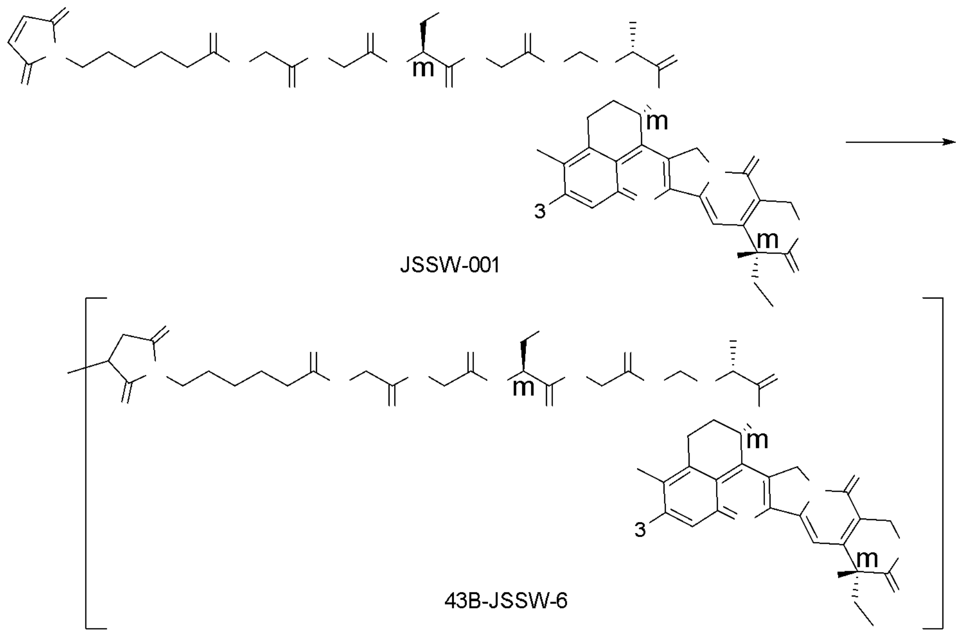

JSSW-001

43B-JSSW-6

**Engineering procedure 1:**

Reduction of antibody:

The buffer for the 43B antibody prepared in the laboratory was replaced with PBS 7.0/EDTA to give an antibody concentration of 10 mg/mL. The resulting solution (1.0 mL) was placed into a 1.5 mL EP tube, and then a 100 mM aqueous TCEP (J&K Scientific) solution (8.16 μL; 12 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of JSSW-001 (Shanghai Haoyuan Chemexpress Co., Ltd.) in DMSO (68.0 μL; 10 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous N-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to the purification described in common procedure A to give a solution containing compound 43B-JSSW-6.

Determination of drug-to-antibody ratio (DAR) of antibody-drug conjugate:

DAR determination was performed using common procedure B. The results indicate that the DAR value of 43B-JSSW-6 was 5.6.

Example 15: Preparation of Antibody-Drug Conjugate 43B-045-4

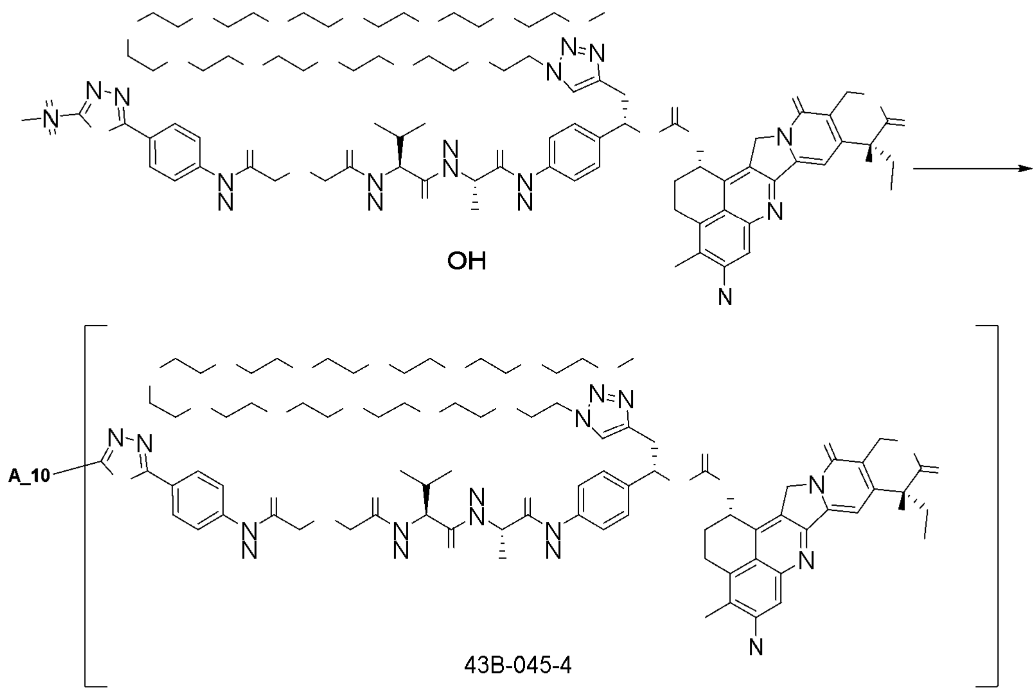

43B-045-4

**Engineering procedure 1:**

Reduction of antibody:

The buffer for the 43B antibody prepared in the laboratory was replaced with PBS 7.0/EDTA to give an antibody concentration of 10 mg/mL. The resulting solution (1.0 mL) was placed into a 1.5 mL EP tube, and then a 10 mM aqueous TCEP (J&K Scientific) solution (15.64 μL; 2.3 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of compound 045 in DMSO (29.92 μL; 4.4 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous *N*-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to the purification described in common procedure A to give a solution containing compound 43B-045-4.

Determination of drug-to-antibody ratio (DAR) of antibody-drug conjugate:

DAR determination was performed using common procedure B. The results indicate that the DAR value of 43B-045-4 was 4.0.

Example 16: Preparation of Antibody-Drug Conjugate 43B-045-6

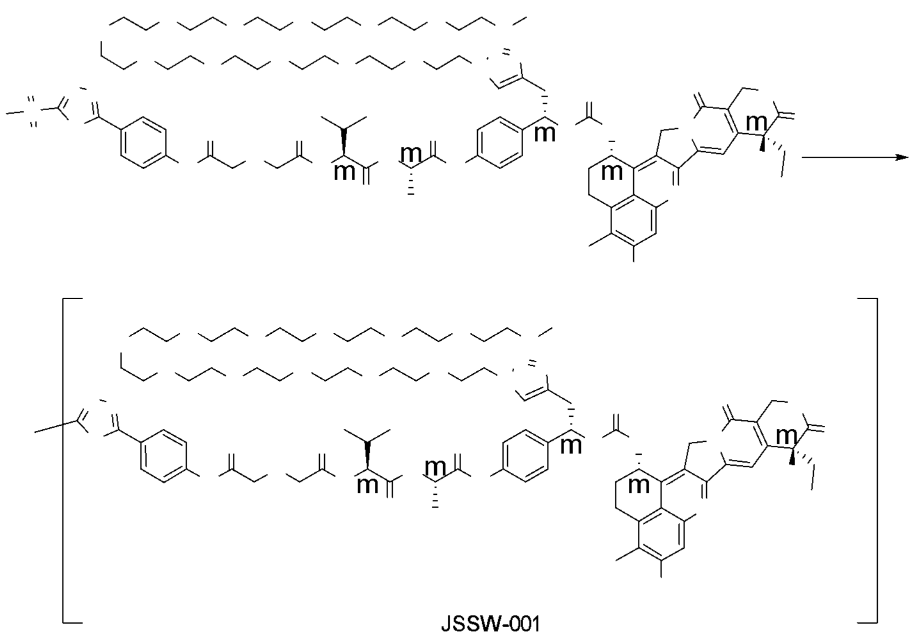

JSSW-001

**Engineering procedure 1:**

Reduction of antibody:

The buffer for the 43B antibody prepared in the laboratory was replaced with PBS 7.0/EDTA to give an antibody concentration of 10 mg/mL. The resulting solution (1.0 mL) was placed into a 1.5 mL EP tube, and then a 100 mM aqueous TCEP (J&K Scientific) solution (8.16 μL; 12 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of compound 045 in DMSO (68.0 μL; 10 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous *N*-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to purification using common procedure A to give a solution containing compound 43B-045-6.

Determination of drug-to-antibody ratio (DAR) of antibody-drug conjugate:

DAR determination was performed using common procedure B. The results indicate that the DAR value of 43B-045-6 was 5.7.

Example 17: Preparation of Antibody-Drug Conjugate 43A-045-8

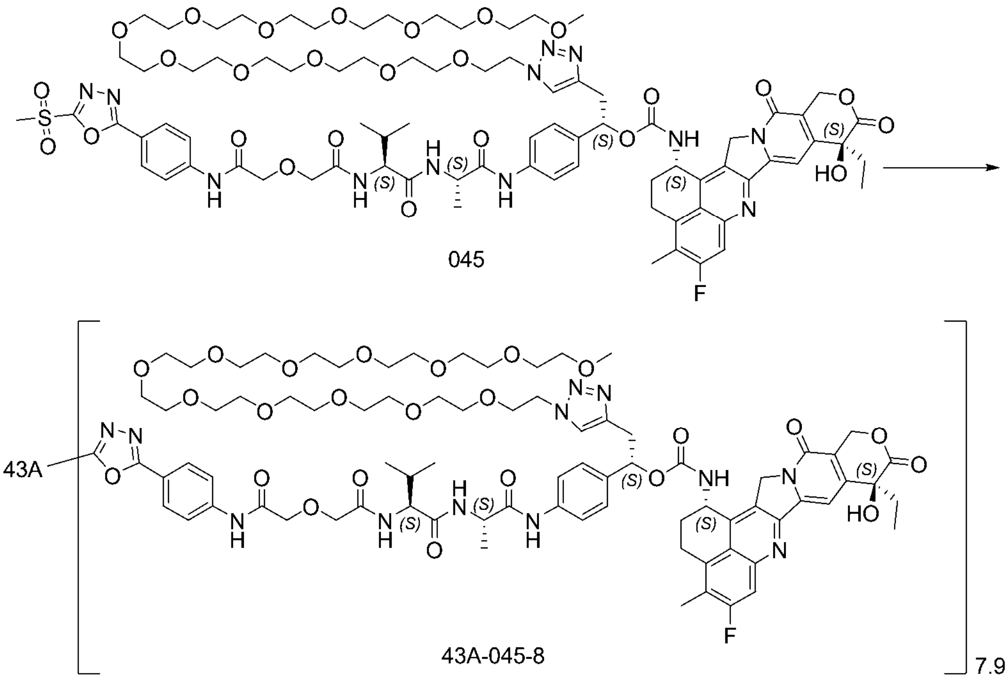

045

43A-045-8

7.9

**Engineering procedure 1:**

Reduction of antibody:

The buffer for the 43A antibody prepared in the laboratory was replaced with PBS 7.0/EDTA to give an antibody concentration of 10 mg/mL. The resulting solution (1.0 mL) was placed into a 1.5 mL EP tube, and then a 100 mM aqueous TCEP (J&K Scientific) solution (8.16 μL; 12 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of compound 045 in DMSO (68.0 μL; 10 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous N-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to the purification described in common procedure A to give a solution containing compound 43A-045-8.

Determination of drug-to-antibody ratio (DAR) of antibody-drug conjugate:

DAR determination was performed using common procedure B. The results indicate that the DAR value of 43A-045-8 was 7.9.

Example 18: Preparation of Antibody-Drug Conjugate M30-DXd-4

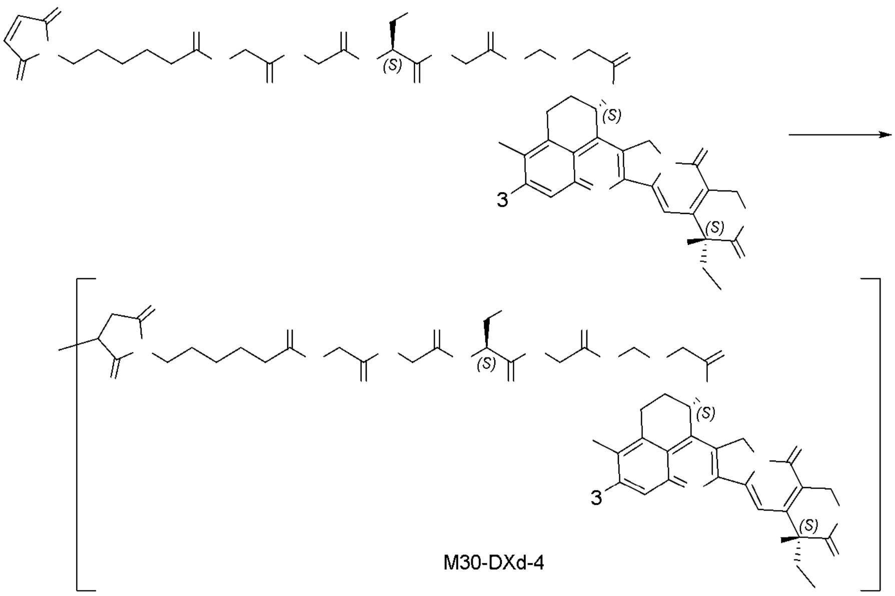

M30-DXd-4

**Engineering procedure 1:**

Reduction of antibody:

The buffer for the M30 antibody prepared in the laboratory was replaced with PBS 7.0/EDTA to give an antibody concentration of 10 mg/mL. The resulting solution (1.0 mL) was placed into a 1.5 mL EP tube, and then a 10 mM aqueous TCEP (J&K Scientific) solution (15.64 μL; 2.3 equivalents relative to one molecule of the antibody) was added to the tube. The mixture was incubated at 37 °C for 3 h, thereby reducing the disulfide bonds in the antibody.

Conjugation of antibody with linker-drug compound:

A 10 mM solution of deruxtecan (Shanghai Haoyuan Chemexpress Co., Ltd.) in DMSO (30.6 μL; 4.5 equivalents relative to one molecule of the antibody) was added to the above solution at room temperature. The mixture was well mixed and allowed to react at room temperature for 30 min, thereby conjugating the linker-drug compound to the antibody. Subsequently, a 100 mM aqueous N-ethylmaleimide (J&K Scientific) solution (10.2 μL; 15 equivalents relative to one molecule of the antibody) was added, and the resulting mixture was further allowed to react at room temperature for 20 min to terminate the conjugation reaction.

Purification of antibody-drug conjugate:

The above solution was subjected to the purification described in common procedure A to give a solution containing compound M30-DXd-4.

Determination of drug-to-antibody ratio (DAR) of antibody-drug conjugate:

DAR determination was performed using common procedure B. The results indicate that the DAR value of M30-DXd-4 was 4.2.

Example 19: Detection of Hydrophobicity of Antibody-Drug Conjugates

Hydrophobic interaction chromatography was used to compare the hydrophobicity of 43A-042, 43B-042, 43A-043, 43B-043, and 43A-DXd. Detection was performed on an e2695 high performance liquid chromatograph using a TSKgel Butyl-NPR chromatographic column (4.6 mm × 10 cm, Cat. No. 0042168/TOSOH). Mobile phase A was 1.0 M ammonium sulfate in 25 mM phosphate buffer, and mobile phase B was 25% isopropanol in 75% 25 mM phosphate buffer. The flow rate was 0.8 mL/min; the detection wavelength was 280 nm; the column temperature was 30 °C; the mobile phase gradient was as follows: 100%-0% A for 0-20 min, 0%-0% A for 20-23 min, 0%-100% A for 23-24 min, 100%-100% A for 24-28 min.

The results, as shown in FIG. 8, indicate that 43A-042, 43B-042, 43A-043, 43B-043, and 43A-DXd all had good

hydrophilicity; among them, 43A-043 exhibited superior hydrophilicity compared to 43A-DXd and 43A-042 with similar DAR values, and their retention times were 7.130 min, 8.809 min, and 9.189 min, respectively. Similarly, 43B-043 exhibited stronger hydrophilicity compared to 43B-042 with an identical DAR value, and their retention times were 6.426 min and 8.643 min, respectively.

Example 20: Assay for Affinity of Antibodies by Bio-Layer Interferometry

The affinity of the anti-B7H3 antibodies 43A and 43B for human B7H3 was analyzed using a ForteBio Octet red 96e instrument (Sartorius). Different test samples were immobilized using a Protein A capture sensor at 5 μg/mL. The B7H3 antigen was subjected to 2-fold gradient dilution starting from an initial concentration of 100 nM to give a total of 7 gradients. The buffer system was PBS pH 7.4 + 0.02% Tween 20. The steps were set as follows: (1) Baseline for 60 s, (2) Loading for 800 s (antibody immobilization, with a threshold set at 0.50 nm), (3) Baseline 2 for 120 s, (4) Association for 120 s, (5) Dissociation for 300 s, and (6) Regeneration for 300 s (sensor regeneration). A blank buffer was used for blank subtraction. The baseline was aligned with the y-axis. Fitting was then performed using Savitzky-Golay in the Octet data analysis software. The fitted curves for 43A and 43B are shown in FIGs. 9 and 10, respectively, and the experimental results are shown in Table 1.

Table 1. Affinity of different antibodies for human B7H3

| Antibody | $K_D$ (M) | kon (1/Ms) | kdis (1/s) | Full R^2 |
|---|---|---|---|---|
| 43A | 6.05E-10 | 7.54E+05 | 4.57E-04 | 0.9981 |
| 43B | 6.09E-10 | 7.18E+05 | 4.37E-04 | 0.9983 |

Example 21: Assay for Cell Binding Activity

The affinity of the anti-B7H3 antibodies 43A and 43B was determined using the B7H3-positive human melanoma cell strain A375, with the M30 antibody used as a positive reference. Each sample was diluted to 15000 ng/mL with a PBS solution containing 2% BSA and then serially diluted across 11 gradients to achieve sample concentrations ranging from 0.014 ng/mL to 15000 ng/mL. A375 cells were taken, centrifuged at 500× g for 5 min, washed 3 times with the PBS solution containing 2% BSA, and incubated with the diluted samples with different concentrations at 4 °C for 2 h. After washing, the cells were incubated with a goat anti-human Alexa Fluor 488 fluorescent dye at 4 °C in the dark for 1 h. After further washing, the cells were resuspended, and the mean fluorescence intensity (MFI) of the cells was measured using an Attune NxT flow cytometer (Thermo Fisher Scientific, Inc.).

The results, as shown in FIG. 11, indicate that both 43A and 43B could bind to B7H3-positive A375 cells with high affinity, and their $EC_{50}$ values were 54.5 ng/mL and 61.2 ng/mL, respectively, both of which were significantly superior to M30's 125.3 ng/mL.

Conjugate 43B-045-6 also bound to B7H3-positive A375 cells with high affinity, exhibiting an $EC_{50}$ value of 37.78 ng/mL.

Example 22: Study on Internalization Activity

The M30, 43A, and 43B antibodies were separately co-incubated with human melanoma A375 cells in a plate placed in a refrigerator at 4 °C for 2 h. After the co-incubation was completed, the cells were washed twice with PBS. A culture medium was added, and then the cells were placed in an incubator at 37 °C with 5% $CO_2$ for internalization. The cells were harvested at the end of the indicated incubation time. Then, the cells were stained with a goat anti-human Alexa Fluor 488 fluorescent dye and incubated at 4 °C in the dark for 60 min. After the incubation was completed, the cells were washed twice with an assay buffer (2% FBS/PBS), resuspended in the buffer, and then assayed using a flow cytometer. The median fluorescence signal value of the corresponding wells in the assay plate was measured using a plate reader.

Internalization rate % = (initial mean median fluorescence value - mean median fluorescence value at a different time point)/initial mean median fluorescence value × 100%.

The experimental results, as shown in Table 2, indicate that after binding to A375 cells, both 43A and 43B could be rapidly internalized into the cells, with the internalization rates faster than that of M30.

Table 2. Internalization rates of antibodies at different time points

| Time | 43A | 43B | M30 |
|---|---|---|---|
| 2 h | 18% | 16% | 11% |
| 4 h | 29% | 34% | 20% |
| 6 h | 46% | 47% | 30% |
| 24 h | 84% | 86% | 71% |

Example 23: Assay for Antigen-Binding Activity

The His-tagged human B7H3 protein (purchased from Beijing ACROBiosystems Co., Ltd.) was diluted to 1 μg/mL with a coating solution and added to a microplate at 100 μL/well. The microplate was incubated at 4 °C overnight. Then, a blocking solution was added at 100 μL/well to the microplate containing the coating solution. The plate was shaken at room temperature for 2 h and then washed 3 times with a washing buffer. Samples and reference standards were subjected to gradient dilution and then added at 100 μL/well. The plate was shaken at room temperature for 2 h and washed 4 times with the washing buffer. An enzyme-labeled antibody was added. Goat anti-human IgG-HRP was diluted with the sample diluent and then added at 100 μL/well. The plate was shaken at room temperature for 1 h and washed 4 times with the washing buffer. A substrate solution (freshly prepared TMB substrate solution) was added at 100 μL/reaction well for color development. The reaction proceeded at room temperature for 5-10 min. Then, a terminating solution was added at 100 μL/well, and the mixture was gently and well mixed to stop the reaction. The absorbance was measured using a microplate reader at wavelengths of 450 nm/650 nm. Plotting was then performed using four-parameter fitting in software, where the C value in the model represents the half-maximal effective concentration $EC_{50}$. Using the 43A and 43B antibodies as reference standards, the binding activities of the corresponding antibody-drug conjugates were determined. The experimental results, as shown in Table 3, indicate that the binding activities of the antibodies and the corresponding antibody-drug conjugates were comparable.

Table 3. Summary of binding activity to human B7H3 protein

| | 43A | 43A-DXd | 43A-042 | 43A-043 | 43B | 43B-042 | 43B-043 |
|---|---|---|---|---|---|---|---|
| $EC_{50}$ (ng/mL) | 1.02 | 1.19 | 1.17 | 1.09 | 1.05 | 1.16 | 1.05 |

Example 24: *In Vitro* Growth Inhibitory Effects of Antibody-Drug Conjugates on SK-OV-3 and SK-BR-3 Cells

The target cells were collected and resuspended to form a single-cell suspension, and the cell viability and cell count were determined by trypan blue staining. The cell density was adjusted to $1 \times 10^5$ cells/mL. The cell suspension was added to a 96-well black flat-bottom cell culture plate at 100 μL/well. The commercially available Enhertu (an anti-HER2 antibody-drug conjugate) was used as a reference standard. The diluted test samples were added to the 96-well black flat-bottom cell culture plate (pre-seeded with cells) at 20 μL/well. The plate was incubated in a cell incubator (37 °C, 5% $CO_2$) for 168±8 h. A resazurin sodium solution (0.03%) was added at 20 μL/well. The plate was then incubated at 37 °C for 3-4 h. Fluorescence values were measured using a microplate reader at 550 nm/610 nm. Plotting was performed using Prism or similar plotting software, and then the half-maximal inhibitory concentration $IC_{50}$ values of the reference standard and the test samples were calculated by fitting.

The experimental results, as shown in FIGs. 12 and 13, indicate that in the *in vitro* experiments using the human ovarian adenocarcinoma SK-OV-3 cells and human breast cancer SK-BR-3 cells, the inhibitory activity of DP001-042 was significantly superior to that of Enhertu. The half-maximal inhibitory concentration data are summarized in Table 4.

Table 4. Inhibitory activity of antibody-drug conjugates against *in vitro* cell growth

| | Cell | Enhertu | DP001-042 | DP001-043 |
|---|---|---|---|---|
| $IC_{50}$ (ng/mL) | SK-OV-3 | 112.7 | 57.03 | 100.2 |
| | SK-BR-3 | 53.74 | 26.92 | 55.58 |

Example 25: *In Vitro* Growth Inhibitory Effects of Antibody-Drug Conjugates on Human Melanoma A375 Cells

The target cells were collected and resuspended to form a single-cell suspension, and the cell viability and cell

count were determined by trypan blue staining. The cell density was adjusted to $1 \times 10^5$ cells/mL. The cell suspension was added to a 96-well black flat-bottom cell culture plate at 100 μL/well. 43A-DXd was used as a reference standard. The diluted test samples were added to the 96-well black flat-bottom cell culture plate (pre-seeded with cells) at 20 μL/well. The plate was incubated in a cell incubator (37 °C, 5% $CO_2$) for 168±8 h. A resazurin sodium solution (0.03%) was added at 20 μL/well. The plate was then incubated at 37 °C for 3-4 h. Fluorescence values were measured using a microplate reader at 550 nm/610 nm. Plotting was performed using Prism or similar plotting software, and then the half-maximal inhibitory concentration $IC_{50}$ values of the reference standard and the test samples were calculated by fitting.

In the *in vitro* experiment using the human melanoma A375 cells, 43A-042, 43B-042, 43A-DXd, 43A-043, and 43B-043 all exhibited good inhibitory activity against cell growth. The results are shown in Tables 5 and 6 and FIGs. 14 and 15.

Table 5. In vitro growth inhibitory activity of antibody-drug conjugates

| | Cell | 43A-DXd | 43A-042 | 43B-042 |
|---|---|---|---|---|
| $IC_{50}$ (ng/mL) | A375 | 24.19 | 9.41 | 14.05 |

Table 6. In vitro cell growth inhibitory activity of antibody-drug conjugates

| | Cell | 43A-DXd | 43A-043 | 43B-043 |
|---|---|---|---|---|
| $IC_{50}$ (ng/mL) | A375 | 24.29 | 22.69 | 36.78 |

Example 26: Anti-Tumor Experiment of Antibody-Drug Conjugates on Human Lung Cancer Calu-6 Cell Xenograft Tumors in Mice

In this experiment, age-appropriate female NOD/SCID mice were selected to be inoculated with human lung cancer Calu-6 cells. When the tumor volume reached about 100-200 $mm^3$, 48 animals with good tumor growth were selected and evenly divided into 6 groups based on the tumor volume. The animal grouping and dosing regimen are shown in Table 7.

Table 7. Animal grouping and dosing regimen

| Group | Number of animals (mice) | Dose (mg/kg) | Route/frequency of administration |
|---|---|---|---|
| Vehicle group (0.9% INJ NS) | 8 | 0 | Intravenous administration/single dose |
| 43A-DXd | 8 | 3.0 | Intravenous administration/single dose |
| 43A-042 | 8 | 3.0 | Intravenous administration/single dose |
| 43A-043 | 8 | 3.0 | Intravenous administration/single dose |
| 43B-042 | 8 | 3.0 | Intravenous administration/single dose |
| 43B-043 | 8 | 3.0 | Intravenous administration/single dose |

After grouping was completed, administration was performed, and the body weight of the mice was measured. The data were recorded. By measuring the tumor diameters at different time points after administration, the tumor growth was dynamically observed. The tumor volume was calculated using the following formula:

$$\text{Tumor volume } (mm^3) = 1/2 \times \text{long diameter (mm)} \times [\text{short diameter (mm)}]^2$$

On day 25 after administration, the mice were euthanized by carbon dioxide asphyxiation, and then the tumors were excised and weighed.

At the end of the experiment, compared to the vehicle group, all the antibody-drug conjugates were able to significantly inhibit the tumor growth.

The tumor weight inhibition rate of 43B-042 was up to 84.8%, and the efficacy of 43B-043, 43A-042, and 43A-043 was superior to that of 43A-DXd. The detailed information is shown in FIG. 16 and Table 8.

Table 8. Tumor parameters for each group on day 25 after administration

| Group | Tumor volume on D25 ($mm^3$) | Tumor growth inhibition rate (%) | Tumor weight (g) | Tumor weight inhibition rate (%) |
|---|---|---|---|---|
| Vehicle group | 1737.6±282.1 | - | 1.369±0.240 | - |
| 43A-DXd | 725.4±208.4 | 63.3 | 0.583±0.153 | 57.4 |
| 43A-042 | 475.8±194.2 | 78.9 | 0.389±0.167 | 71.6 |
| 43A-043 | 619.6±229.3 | 85.2 | 0.498±0.189 | 63.6 |
| 43B-042 | 273.2±194.2 | 91.6 | 0.167±0.000 | 84.8 |
| 43B-043 | 376.1±119.3 | 69.9 | 0.275±0.079 | 79.9 |

Example 27: *In Vitro* Growth Inhibitory Effects of Antibody-Drug Conjugates on Human Melanoma A375 Cells

The target cells were collected and resuspended to form a single-cell suspension, and the cell viability and cell count were determined by trypan blue staining. The cell density was adjusted to $1 \times 10^5$ cells/mL. The cell suspension was added to a 96-well black flat-bottom cell culture plate at 100 μL/well. The diluted test samples were added to the 96-well black flat-bottom cell culture plate (pre-seeded with cells) at 20 μL/well. The plate was incubated in a cell incubator (37 °C, 5% CO2) for 168+8 h. A resazurin sodium solution (0.03%) was added at 20 μL/well. The plate was then incubated at 37 °C for 3-4 h. Fluorescence values were measured using a microplate reader at 550 nm/610 nm. Plotting was performed using Prism or similar plotting software, and then the half-maximal inhibitory concentration $IC_{50}$ values of the test samples were calculated by fitting.

In the *in vitro* experiment using the human melanoma A375 cells, 43A-JSSW-4, 43A-JSSW-6, 43A-JSSW-8, 43B-JSSW-6, 43B-045-4, 43B-045-6, and 43A-045-8 all exhibited good inhibitory activity against cell growth. The results are shown in Table 9.

Table 9. *In vitro* cell growth inhibitory activity of antibody-drug conjugates

| Test sample | $IC_{50}$ (ng/mL) |
|---|---|
| 43A-JSSW-4 | 73.66 |
| 43A-JSSW-6 | 38.64 |
| 43A-JSSW-8 | 27.13 |
| 43B-JSSW-6 | 42.55 |
| 43B-045-4 | 31.27 |
| 43B-045-6 | 17.06 |
| 43A-045-8 | 13.35 |

Example 28: Anti-Tumor Experiment of Antibody-Drug Conjugates on Human Melanoma A375 Cell Xenograft Tumors in Mice

In this experiment, age-appropriate female NU/NU mice were selected to be inoculated with human melanoma A375 cells. When the tumor volume reached about 100-200 $mm^3$, 28 animals with good tumor growth were selected and evenly divided into 4 groups based on the tumor volume. The animal grouping and dosing regimen are shown in Table 10.

Table 10. Animal grouping and dosing regimen

| Group | Number of animals (mice) | Dose (mg/kg) | Route/frequency of administration |
|---|---|---|---|
| Vehicle group | 7 | 0 | Intravenous administration/single dose |
| 43A-JSSW-4 | 7 | 1.5 | Intravenous administration/single dose |
| 43A-JSSW-6 | 7 | 1.5 | Intravenous administration/single dose |
| 43A-JSSW-8 | 7 | 1.5 | Intravenous administration/single dose |

After grouping was completed, administration was performed, and the body weight of the mice was measured. The data were recorded. By measuring the tumor diameters at different time points after administration, the tumor growth was dynamically observed. The tumor volume was calculated using the following formula:

$$\text{Tumor volume (mm}^3\text{)} = 1/2 \times \text{long diameter (mm)} \times [\text{short diameter (mm)}]^2$$

At the end of the experiment, compared to the vehicle group, all the antibody-drug conjugates were able to significantly inhibit the tumor growth. The tumor growth inhibition rates of 43A-JSSW-4, 43A-JSSW-6, and 43A-JSSW-8 were 53.6%, 65.2%, and 87.7%, respectively. The detailed information is shown in FIG. 18 and Table 11.

Table 11. Efficacy of ADCs on A375 xenograft tumors in tumor-bearing mice

| Group | Mean tumor volume on D0 ($mm^3$) | Mean tumor volume on D14 ($mm^3$) | Tumor growth inhibition (TGI) rate (%) |
|---|---|---|---|
| Vehicle group | 192.1±33.8 | 1,948.5±373.9 | N/A |
| 43A-JSSW-4 | 192.4±27.7 | 1,006.8±211.3 | 53.6 |
| 43A-JSSW-6 | 192.9±26.1 | 803.5±586.8 | 65.2 |
| 43A-JSSW-8 | 192.7±25.3 | 409.0±370.9 | 87.7 |

Example 29: Anti-Tumor Experiment of Antibody-Drug Conjugates on Human Lung Cancer Calu-6 Cell Xenograft Tumors in Mice

In this experiment, age-appropriate female NOD/SCID mice were selected to be inoculated with human lung cancer Calu-6 cells. When the tumor volume reached about 100-200 $mm^3$, 35 animals with good tumor growth were selected and evenly divided into 5 groups based on the tumor volume. The animal grouping and dosing regimen are shown in Table 12.

Table 12. Animal grouping and dosing regimen

| Group | Number of animals (mice) | Dose (mg/kg) | Route/frequency of administration |
|---|---|---|---|
| Vehicle group | 7 | 0 | Intravenous administration/single dose |
| 43A-JSSW-4 | 7 | 3.0 | Intravenous administration/single dose |
| 43A-JSSW-6 | 7 | 3.0 | Intravenous administration/single dose |
| 43A-JSSW-8 | 7 | 3.0 | Intravenous administration/single dose |
| 43B-JSSW-6 | 7 | 3.0 | Intravenous administration/single dose |

After grouping was completed, administration was performed, and the body weight of the mice was measured. The data were recorded. By measuring the tumor diameters at different time points after administration, the tumor growth was dynamically observed. The tumor volume was calculated using the following formula:

$$\text{Tumor volume (mm}^3\text{)} = 1/2 \times \text{long diameter (mm)} \times [\text{short diameter (mm)}]^2$$

At the end of the experiment, compared to the vehicle group, all the antibody-drug conjugates were able to significantly inhibit the tumor growth. The detailed information is shown in FIG. 19 and Table 13.

Table 13. Efficacy of ADCs on Calu-6 xenograft tumors in tumor-bearing mice

| Group | Mean tumor volume on D0 ($mm^3$) | Mean tumor volume on D14 ($mm^3$) | Tumor growth inhibition (TGI) rate (%) |
|---|---|---|---|
| Vehicle group | 128.2±17.4 | 982.7±170.1 | N/A |
| 43A-JSSW-4 | 127.8±21.8 | 532.0±75.7 | 52.7 |
| 43A-JSSW-6 | 129.2±27.8 | 437.3±156.1 | 63.9 |

(continued)

| Group | Mean tumor volume on D0 ($mm^3$) | Mean tumor volume on D14 ($mm^3$) | Tumor growth inhibition (TGI) rate (%) |
|---|---|---|---|
| 43A-JSSW-8 | 128.9±30.5 | 422.7±263.6 | 65.6 |
| 43B-JSSW-6 | 127.3±21.9 | 447.7±62.3 | 62.5 |

Example 30: Anti-Tumor Experiment of Antibody-Drug Conjugate on Human Melanoma A375 Cell Xenograft Tumors in Mice

In this experiment, age-appropriate female NU/NU mice were selected to be inoculated with human melanoma A375 cells. When the tumor volume reached about 100-200 $mm^3$, animals with good tumor growth were selected and evenly divided into groups based on the tumor volume. The animal grouping and dosing regimen are shown in Table 14.

Table 14. Animal grouping and dosing regimen

| Group | Number of animals (mice) | Dose (mg/kg) | Route/frequency of administration |
|---|---|---|---|
| Vehicle group | 7 | 0 | Intravenous administration/single dose |
| 43B-045-6 | 7 | 2.0 | Intravenous administration/single dose |

After grouping was completed, administration was performed, and the body weight of the mice was measured. The data were recorded. By measuring the tumor diameters at different time points after administration, the tumor growth was dynamically observed. The tumor volume was calculated using the following formula:

$$\text{Tumor volume } (mm^3) = 1/2 \times \text{long diameter (mm)} \times [\text{short diameter (mm)}]^2$$

At the end of the experiment, compared to the vehicle group, the antibody-drug conjugate was able to significantly inhibit the tumor growth. The detailed information is shown in FIG. 20 and Table 15.

Table 15. Efficacy of ADC on A375 xenograft tumors in tumor-bearing mice

| Group | Mean tumor volume on D0 ($mm^3$) | Mean tumor volume on D26 ($mm^3$) |
|---|---|---|
| Vehicle group | 128.7+32.9 | N/A |
| 43B-045-6 | 128.9+39.0 | 144.6+90.1 |
| Note: On day 22, the mean tumor volume of the animals in the vehicle group reached 3000 $mm^3$, and the animals were euthanized in accordance with animal welfare guidelines. | | |

Example 31: Anti-Tumor Experiment of Antibody-Drug Conjugates on Human Esophageal Squamous Cell Carcinoma KYSE-150 Xenograft Tumors in Mice

In this experiment, age-appropriate female NU/NU mice were selected to be inoculated with human esophageal squamous cell carcinoma KYSE-150 cells. When the tumor volume reached about 100 $mm^3$, animals with good tumor growth were selected and evenly divided into groups based on the tumor volume. The animal grouping and dosing regimen are shown in Table 16.

Table 16. Animal grouping and dosing regimen

| Group | Number of animals (mice) | Dose (mg/kg) | Route/frequency of administration |
|---|---|---|---|
| Vehicle group | 6 | 0 | Intravenous administration/single dose |
| 43B-JSSW-6 | 6 | 2.0 | Intravenous administration/single dose |
| M30-DXd-4 | 6 | 2.0 | Intravenous administration/single dose |

After grouping was completed, administration was performed, and the body weight of the mice was measured.

The data were recorded. By measuring the tumor diameters at different time points after administration, the tumor growth was dynamically observed. The tumor volume was calculated using the following formula:

$$\text{Tumor volume (mm}^3\text{)} = 1/2 \times \text{long diameter (mm)} \times [\text{short diameter (mm)}]^2$$

At the end of the experiment, compared to the vehicle group, 43B-JSSW-6 was able to significantly inhibit the tumor growth. The detailed information is shown in FIG. 21 and Table 17.

Table 17. Efficacy of ADCs on KYSE-150 xenograft tumors in tumor-bearing mice

| Group | Mean tumor volume on D0 ($mm^3$) | Mean tumor volume on D25 ($mm^3$) | Tumor growth inhibition (TGI) rate (%) |
|---|---|---|---|
| Vehicle group | 79.6±24.2 | 1,536.7±1,854.6 | N/A |
| 43B-JSSW-6 | 79.2±13.1 | 563.6±466.6 | 66.8 |
| M30-DXd-4 | 79.9+18.5 | 2,021.4+888.0 | -33.2 |

Example 32: Anti-Tumor Experiment of Antibody-Drug Conjugates on Human Non-Small Cell Lung Cancer SK-MES-1 Xenograft Tumors in Mice

In this experiment, age-appropriate female NU/NU mice were selected to be inoculated with human non-small cell lung cancer SK-MES-1 cells. When the tumor volume reached about 100 $mm^3$, animals with good tumor growth were selected and evenly divided into groups based on the tumor volume. The animal grouping and dosing regimen are shown in Table 18.

Table 18. Animal grouping and dosing regimen

| Group | Number of animals (mice) | Dose (mg/kg) | Route/frequency of administration |
|---|---|---|---|
| Vehicle group | 6 | 0 | Intravenous administration/single dose |
| 43B-JSSW-6 | 6 | 2.0 | Intravenous administration/single dose |
| M30-DXd-4 | 6 | 2.0 | Intravenous administration/single dose |

After grouping was completed, administration was performed, and the body weight of the mice was measured. The data were recorded. By measuring the tumor diameters at different time points after administration, the tumor growth was dynamically observed. The tumor volume was calculated using the following formula:

$$\text{Tumor volume (mm}^3\text{)} = 1/2 \times \text{long diameter (mm)} \times [\text{short diameter (mm)}]^2$$

At the end of the experiment, compared to the vehicle group, 43B-JSSW-6 was able to significantly inhibit the tumor growth. The detailed information is shown in FIG. 22 and Table 19.

Table 19. Efficacy of ADCs on SK-MES-1 xenograft tumors in tumor-bearing mice

| Group | Mean tumor volume on D0 ($mm^3$) | Mean tumor volume on D22 ($mm^3$) | Tumor growth inhibition (TGI) rate (%) |
|---|---|---|---|
| Vehicle group | 135.0±35.1 | 727.4±489.0 | N/A |
| 43B-JSSW-6 | 134.3±32.7 | 322.9±140.6 | 68.2 |
| M30-DXd-4 | 134.6±35.1 | 588.8±266.4 | 23.3 |

Example 33: Anti-Tumor Experiment of Antibody-Drug Conjugates on Human Liver Cancer Hep3B Xenograft Tumors in Mice

In this experiment, age-appropriate female NU/NU mice were selected to be inoculated with human liver cancer Hep3B cells. When the tumor volume reached about 100-200 $mm^3$, animals with good tumor growth were selected and

evenly divided into 3 groups based on the tumor volume. The animal grouping and dosing regimen are shown in Table 20.

Table 20. Animal grouping and dosing regimen

| Group | Number of animals (mice) | Dose (mg/kg) | Route/frequency of administration |
|---|---|---|---|
| Vehicle group | 6 | 0 | Intravenous administration/single dose |
| 43B-JSSW-6 | 6 | 2.0 | Intravenous administration/single dose |
| 43B-045-6 | 6 | 2.0 | Intravenous administration/single dose |

After grouping was completed, administration was performed, and the body weight of the mice was measured. The data were recorded. By measuring the tumor diameters at different time points after administration, the tumor growth was dynamically observed. The tumor volume was calculated using the following formula:

$$\text{Tumor volume (mm}^3\text{)} = 1/2 \times \text{long diameter (mm)} \times [\text{short diameter (mm)}]^2$$

At the end of the experiment, compared to the vehicle group, the test sample groups were all able to significantly inhibit the tumor growth. The detailed information is shown in FIG. 23 and Table 21.

Table 21. Efficacy of ADCs on Hep3B xenograft tumors in tumor-bearing mice

| Group | Mean tumor volume on D0 ($mm^3$) | Mean tumor volume on D22 ($mm^3$) | Tumor growth inhibition (TGI) rate (%) |
|---|---|---|---|
| Vehicle group | 152.9±46.8 | 2434.6±529.4 | N/A |
| 43B-JSSW-6 | 153.5±56.6 | 1008.6±611.5 | 62.5 |
| 43B-045-6 | 153.0±57.1 | 917.5±405.8 | 66.5 |

Example 34: Anti-Tumor Experiment of Antibody-Drug Conjugates on Human Lung Cancer Calu-6 Xenograft Tumors in Mice

In this experiment, age-appropriate NOD/SCID mice were selected to be inoculated with human lung cancer Calu-6 cells. When the tumor volume reached about 100-200 $mm^3$, 24 animals with good tumor growth were selected and evenly divided into 4 groups based on the tumor volume. The animal grouping and dosing regimen are shown in Table 22.

Table 22. Animal grouping and dosing regimen

| Group | Number of animals (mice) | Dose (mg/kg) | Route/frequency of administration |
|---|---|---|---|
| Vehicle group | 6 | 0 | Intravenous administration/once every two weeks |
| 43B-JSSW-6 | 6 | 3.0 | Intravenous administration/once every two weeks |
| 43B-045-4 | 6 | 3.0 | Intravenous administration/once every two weeks |
| M30-DXd-4 | 6 | 3.0 | Intravenous administration/once every two weeks |

After grouping was completed, administration was performed, and the body weight of the mice was measured. The data were recorded. By measuring the tumor diameters at different time points after administration, the tumor growth was dynamically observed. The tumor volume was calculated using the following formula:

$$\text{Tumor volume (mm}^3\text{)} = 1/2 \times \text{long diameter (mm)} \times [\text{short diameter (mm)}]^2$$

At the end of the experiment, compared to the vehicle group, the test sample groups were all able to significantly inhibit the tumor growth. Furthermore, the efficacy of 43B-JSSW-6 and 43B-045-4 was significantly superior to that of the positive reference M30-DXd-4. The detailed information is shown in FIG. 24 and Table 23.

Table 23. Efficacy of ADCs on Calu-6 xenograft tumors in tumor-bearing mice

| Group | Mean tumor volume on D0 ($mm^3$) | Mean tumor volume on D28 ($mm^3$) | Tumor growth inhibition (TGI) rate (%) |
|---|---|---|---|
| Vehicle group | 157.7±51.2 | 3041.5±1395.0 | N/A |
| 43B-JSSW-6 | 158.3±58.3 | 117.8±68.0 | 101.4 |
| 43B-045-4 | 158.5±55.3 | 276.2±123.7 | 95.9 |
| M30-DXd-4 | 158.5±44.2 | 1604.8±403.6 | 49.8 |

## Claims

1. An antibody-drug conjugate having a structure represented by formula (I), and a tautomer, a mesomer, a racemate, an enantiomer, a diastereoisomer, a pharmaceutically acceptable salt, a hydrate, a solvate or an isotopically labeled compound thereof:

$$A\text{-}(L\text{-}D)_d \qquad (I)$$

wherein A is selected from an antibody (e.g., a monoclonal antibody) or an antigen-binding fragment thereof;
L is a linker moiety, with one end linked to A and the other end linked to a bioactive molecule D;
D is a bioactive molecule;
d represents the molar ratio of the bioactive molecule to A (also known as DAR, i.e., drug-to-antibody ratio), and is an integer or a decimal selected from 1 to 12 (e.g., 1, 2, 3, 4, 5, 6, 7, 8, 9, 10, 11, or 12);
when d is a decimal, it refers to the average number of linker-bioactive molecules (L-D) conjugated per A.

2. The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to claim 1, wherein
A is selected from an antibody or an antigen-binding fragment thereof targeting HER2 (ErbB2), HER3 (ErbB3), HER4 (ErbB4), EGFR, DLL3, TROP2, B7H3, B7H4, TF (Tissue factor), c-Met, CD20, CD22, CD30, CD33, CD44, CD47, CD56, CD70, CD73, CD79b, CD105, CEA, A33, Cripto, EphA2, G250, MUC1, Lewis Y, EDB-FN, VEGFR, VEGF, PD-1, PD-L1, MET, RET, GPNMB, Integrin, PSMA, Tenascin-C, SLC44A4, Claudin18.2, and Mesothelin.

3. The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to any one of claims 1-2, wherein
A is an antibody or an antigen-binding fragment thereof targeting B7H3; the antibody or the antigen-binding fragment thereof comprises a heavy chain and/or a light chain, wherein the heavy chain comprises 3 complementarity determining regions (CDRs), wherein the amino acid sequence of the heavy chain complementarity determining region 1 (HCDR1) is set forth in SEQ ID NO. 1, the amino acid sequence of the heavy chain complementarity determining region 2 (HCDR2) is set forth in SEQ ID NO. 2, and the amino acid sequence of the heavy chain complementarity determining region 3 (HCDR3) is set forth in SEQ ID NO. 3; the light chain comprises 3 complementarity determining regions (CDRs), wherein the amino acid sequence of the light chain complementarity determining region 1 (LCDR1) is set forth in SEQ ID NO. 4, the amino acid sequence of the light chain complementarity determining region 2 (LCDR2) is set forth in SEQ ID NO. 5, and the amino acid sequence of the light chain complementarity determining region 3 (LCDR3) is set forth in SEQ ID NO. 6; the CDRs are determined according to the Kabat numbering scheme.

4. The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to any one of claims 1-3, wherein
A is an antibody or an antigen-binding fragment thereof targeting B7H3; the anti-B7H3 antibody or the antigen-binding fragment thereof comprises a heavy chain and/or a light chain, wherein the sequence of a variable region of the heavy chain (HV) is set forth in SEQ ID NO. 7, and the sequence of a variable region of the light chain (LV) is set forth in SEQ ID

NO. 8.

5. The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to any one of claims 1-4, wherein
A is an antibody targeting B7H3, wherein the anti-B7H3 antibody further comprises a heavy chain constant region sequence or a variant thereof, and/or a light chain constant region sequence or a variant thereof.

6. The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to any one of claims 1-5, wherein
A is an antigen-binding fragment targeting B7H3, wherein the antigen-binding fragment is selected from a Fab, a Fab', a Fab'-SH, an Fv, an scFv, and a $F(ab')_2$.

7. The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to any one of claims 1-6, wherein
A is an antibody or an antigen-binding fragment thereof targeting B7H3, wherein the anti-B7H3 antibody or the antigen-binding fragment thereof is a humanized or fully human antibody or an antigen-binding fragment thereof.

8. The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to any one of claims 1-7, wherein
A is an antibody or an antigen-binding fragment thereof targeting B7H3; the anti-B7H3 antibody comprises a heavy chain and/or a light chain, wherein the amino acid sequence of the heavy chain is set forth in SEQ ID NO. 9 or SEQ ID NO. 10, and the amino acid sequence of the light chain is set forth in SEQ ID NO. 11.

9. The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to claim 1, wherein
A is selected from 43A or 43B targeting B7H3, DP001 targeting HER2, sacituzumab targeting TROP2, alsevalimab targeting B7H4, tisotumab targeting TF, L19A targeting EDB-FN, zolbetuximab targeting Claudin18.2, and biosimilar thereof, wherein 43A comprises 2 identical heavy chains and 2 identical light chains, wherein the amino acid sequence of the heavy chain is set forth in SEQ ID NO. 9, and the amino acid sequence of the light chain is set forth in SEQ ID NO. 11; 43B comprises 2 identical heavy chains and 2 identical light chains, wherein the amino acid sequence of the heavy chain is set forth in SEQ ID NO. 10, and the amino acid sequence of the light chain is set forth in SEQ ID NO. 11.

10. The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to any one of claims 1-9, wherein
-L- is selected from $-L_1-L_2-L_3-L_4-L_5-$, wherein $L_1$ is a covalent linker unit covalently linked to A, $L_2$ is an extension unit, $L_3$ is selected from a bond and an amino acid residue optionally substituted with a polar hydrophilic group, $L_4$ is selected from a peptide residue consisting of 2-8 amino acids, and $L_5$ is a bond, a self-immolative fragment, or a self-immolative fragment substituted with a polar hydrophilic group.

11. The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to claim 10, wherein

$L_1$ is selected from

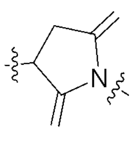 , 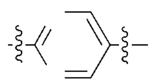 , 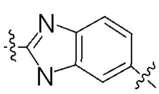 , 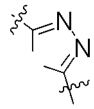 , and 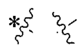 ,

wherein * indicates linkage to A;

$L_2$ is selected from $-L_{2a}-$, $-L_{2a}-C(O)-$, $-C(O)-L_{2a}-C(O)-NH-L_{2b}-C(O)-$, and $-L_{2a}-NH-C(O)-L_{2b}-C(O)-$, wherein $L_{2a}$ and $L_{2b}$ are each independently selected from $-C_1-C_8$ alkylene-, $-C_1-C_8$ alkylene-$C_3-C_8$ cycloalkylene-, -alkynylene-$C_1-C_6$ alkylene-, -phenylene-, -phenylene-$C_1-C_3$ alkylene-, and linear or branched heteroalkylene having 1-50 (preferably 9-30, and more preferably 9-26, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, wherein the alkylene, cycloalkylene, and heterocyclylene are each optionally substituted with one or more substituents independently selected from $C_1-C_6$ alkyl, heteroalkyl having 1-6 atoms, $C_1-C_6$ alkoxy, hydroxyl, amino, carboxyl, and $C_3-C_8$ cycloalkyl, and the heteroalkylene contains 1-12 (preferably 1-8, and more preferably 3-8, e.g., 1, 2, 3, 4, 5, 6, 7, 8, 9, 10, 11, or 12) heteroatoms selected from one or more of N, O, and S (preferably O);
$L_3$ is selected from a bond and an amino acid residue substituted with a polar hydrophilic group, wherein the polar hydrophilic group comprises a saccharide residue and a derivative thereof, a polyethylene glycol residue and a derivative thereof, a polysarcosine residue and a derivative thereof, or a combination thereof;
$L_4$ is selected from a peptide residue consisting of 2-8 amino acids selected from phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine;
$L_5$ is selected from a bond,

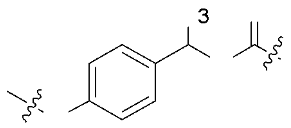

, and

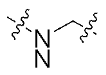

,

wherein $R_3$ is selected from hydrogen and

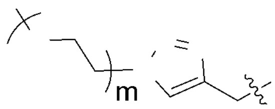

,

m is an integer selected from 3-50, preferably an integer selected from 8-24, and further preferably 8, 9, 10, 11, 12, 13, 14, 15, 16, 17, 18, 19, 20, 21, 22, 23, or 24; * indicates linkage to D;
preferably, $L_5$ is selected from

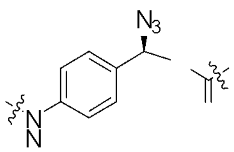

,

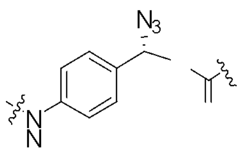

, and

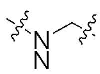

.

**12.** The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to claim 11, wherein
$L_2$ is selected from $-L_{2a}-$, $-L_{2a}-C(O)-$, $-C(O)-L_{2a}-C(O)-NH-L_{2b}-C(O)-$, and $-L_{2a}-NH-C(O)-L_{2b}-C(O)-$, wherein $L_{2a}$ and $L_{2b}$ are each independently selected from $-C_1-C_6$ alkylene-, $-C_1-C_3$ alkylene-$C_3-C_6$ cycloalkylene-, -ethynylene-$C_1-C_6$ alkylene-, $-(CH_2CH_2O)_s-$, $-(CH_2CH_2O)_sC_{1-3}$ alkylene-, $-C_{1-3}$ alkylene$(CH_2CH_2O)_sC_{1-3}$ alkylene-, phenylene, and phenylenemethylene, wherein s is an integer selected from 1-12 (preferably 3-8, e.g., 1, 2, 3, 4, 5, 6, 7, 8, 9, 10, 11, or 12).

**13.** The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to claims 11-12, wherein
$L_2$ is selected from $-L_{2a}-$, $-L_{2a}-C(O)-$, $-C(O)-L_{2a}-C(O)-NH-L_{2b}-C(O)-$, and $-L_{2a}-NH-C(O)-L_{2b}-C(O)-$, wherein $L_{2a}$ and $L_{2b}$ are each independently selected from methylene, ethylene, n-propylene, isopropylene, n-pentylene, methylenecyclohexylene, ethynylenemethylene, ethynyleneethylene, ethynylenen-n-propylene, ethynylenen-n-butylene, ethynylenen-n-pentylene, ethynylenen-n-hexylene, phenylene, phenylenemethylene, $-(CH_2CH_2O)_sCH_2-$,

$-(CH_2CH_2O)_sCH_2CH_2-$, $-CH_2(OCH_2CH_2)_s-$, $-CH_2CH_2(OCH_2CH_2)_s-$, $-CH_2CH_2(OCH_2CH_2)_sCH_2-$, $-CH_2CH_2(OCH_2CH_2)_sCH_2CH_2-$, and $-CH_2(OCH_2CH_2)_sCH_2-$, wherein s is an integer selected from 1-12 (preferably 3-8, e.g., 1, 2, 3, 4, 5, 6, 7, 8, 9, 10, 11, or 12).

**14.** The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to claims 11-13, wherein
$L_2$ is selected from:

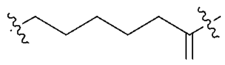,
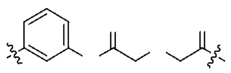,
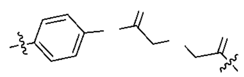,
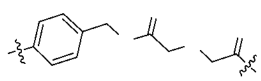,
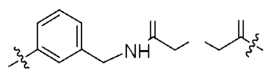,

$-(CH_2CH_2O)_sCH_2C(O)-$, $-(CH_2CH_2O)_sCH_2CH_2-$,

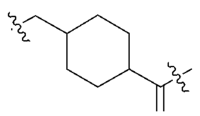, and
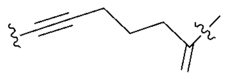,

wherein s is an integer selected from 1-12 (preferably 3-8, e.g., 1, 2, 3, 4, 5, 6, 7, 8, 9, 10, 11, or 12).

**15.** The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to claims 11-14, wherein
$L_3$ is selected from glutamic acid substituted with a polar hydrophilic group, wherein the polar hydrophilic group comprises a saccharide residue and a derivative thereof, a polyethylene glycol residue and a derivative thereof, a polysarcosine residue and a derivative thereof, or a combination thereof.

**16.** The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to claims 11-15, wherein
$L_3$ is selected from:

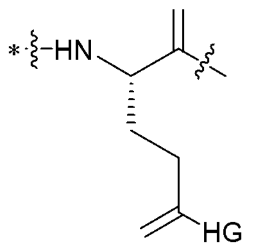,

wherein HG is selected from a group comprising a saccharide residue and a derivative thereof, a group comprising a polyethylene glycol residue and a derivative thereof, a group comprising a polysarcosine residue and a derivative thereof, and a combination thereof; * indicates linkage to $L_2$.

**17.** The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to claims 11-16, wherein
HG is selected from: $-NH(CH_2CH_2O)nCH_3$,

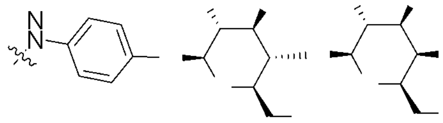,

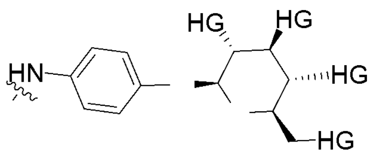, 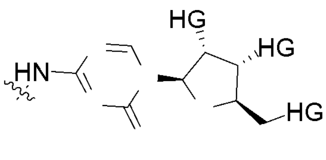, 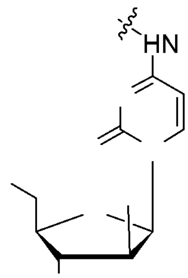, 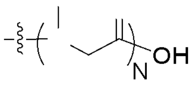,

and

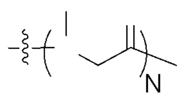,

wherein n and q are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50), preferably 1-24, and further preferably 3-12.

**18.** The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to claims 11-17, wherein
$L_4$ is selected from:

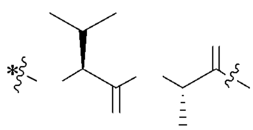, 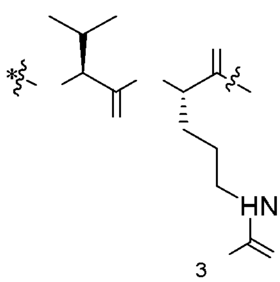, 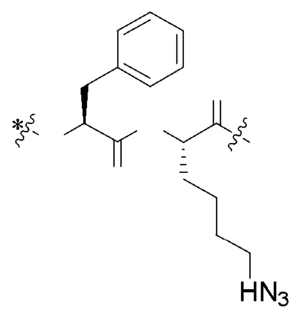,

and

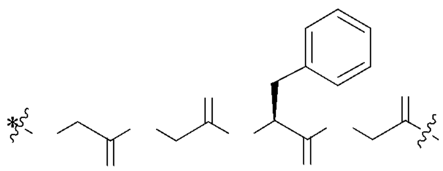,

wherein * indicates linkage to $L_3$.

**19.** The antibody-drug conjugate having the structure represented by formula (I), and the tautomer, the mesomer, the racemate, the enantiomer, the diastereoisomer, the pharmaceutically acceptable salt, the hydrate, the solvate or the

isotopically labeled compound thereof according to any one of claims 1-9, wherein -L- is selected from:

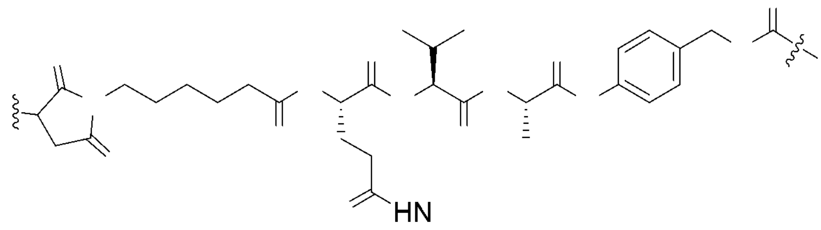

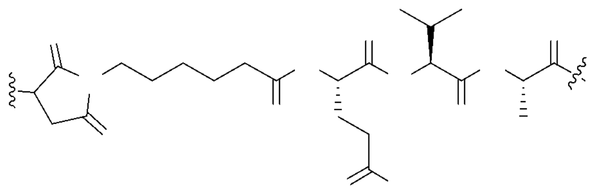

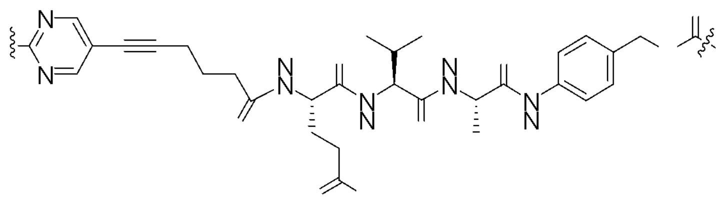

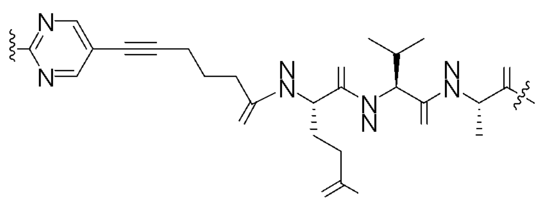

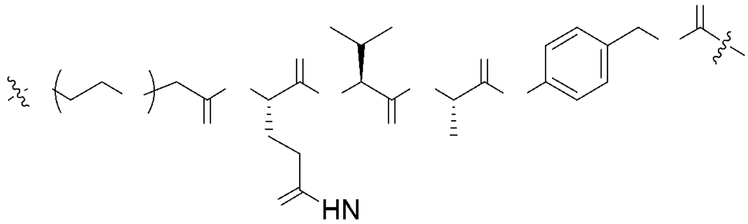

and

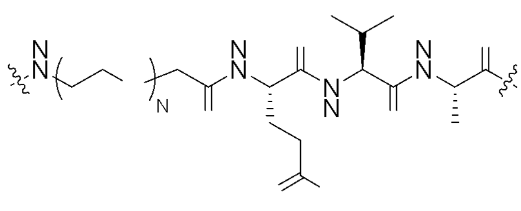

wherein s is an integer selected from 1-12 (preferably 3-8, e.g., 1, 2, 3, 4, 5, 6, 7, 8, 9, 10, 11, or 12), HG is as defined in claim 17, and * indicates linkage to A.

**20.** The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to any one of claims 1-9, wherein -L- is selected from -$L_1$-$L_2$-$L_4$-$L_5$-, wherein $L_1$, $L_2$, $L_4$, and $L_5$ are as defined in claims 11-18.

**21.** The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to any one of claims 1-9, wherein -L- is selected from:

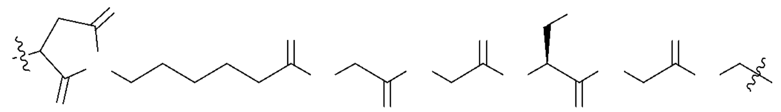

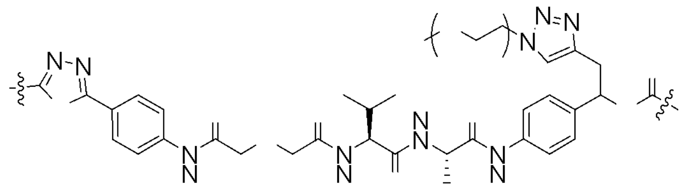

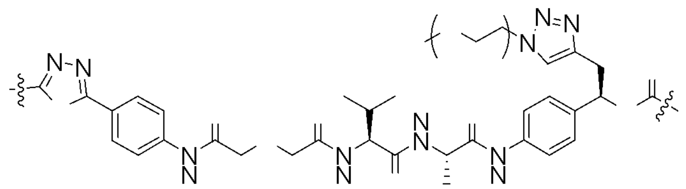

and

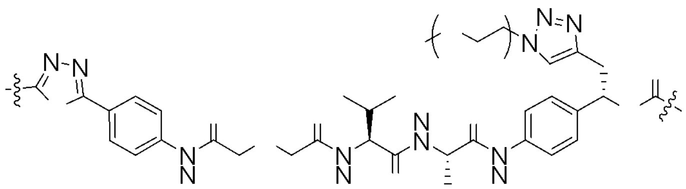

wherein m is an integer selected from 3-50, preferably an integer selected from 8-24, and further preferably 8, 9, 10, 11, 12, 13, 14, 15, 16, 17, 18, 19, 20, 21, 22, 23, or 24.

**22.** The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to any one of claims 1-21, wherein D is selected from a topoisomerase I inhibitor (e.g., a camptothecin derivative), a tubulin inhibitor (e.g., MMAE, MMAF, or eribulin), and a DNA damaging agent (e.g., a pyrrolobenzodiazepine).

**23.** The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to any one of claims 1-22, wherein D is selected from:

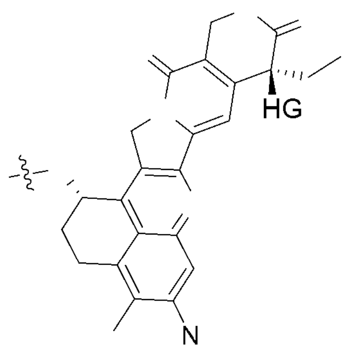

and

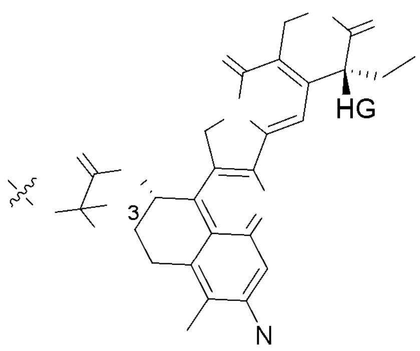

,

wherein $R_1$ and $R_2$ are each independently selected from H, C1-3 alkyl (preferably methyl, ethyl, n-propyl, or isopropyl), and 3-to 6-membered cycloalkyl (preferably cyclopropyl, cyclobutyl, cyclopentyl, or cyclohexyl); or $R_1$ and $R_2$, together with the carbon atom attached thereto, form 3- to 6-membered cycloalkyl (preferably cyclopropyl, cyclobutyl, cyclopentyl, or cyclohexyl).

**24.** The antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to any one of claims 1-23, wherein the antibody-drug conjugate has the following structure:

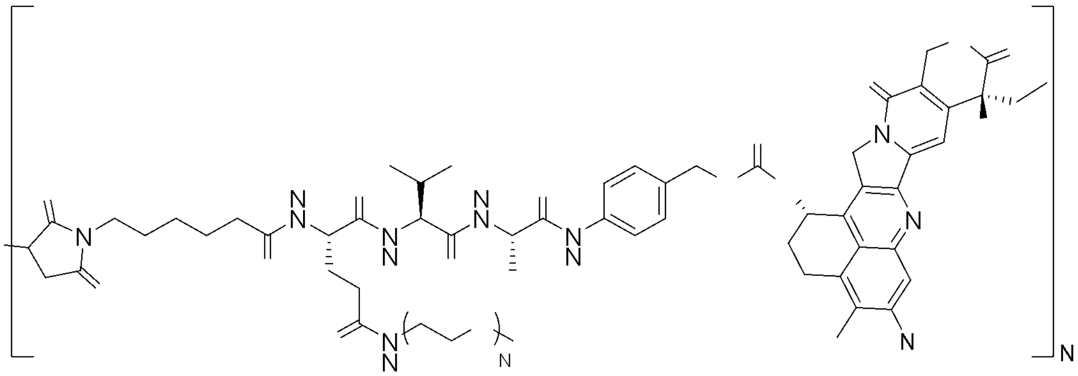

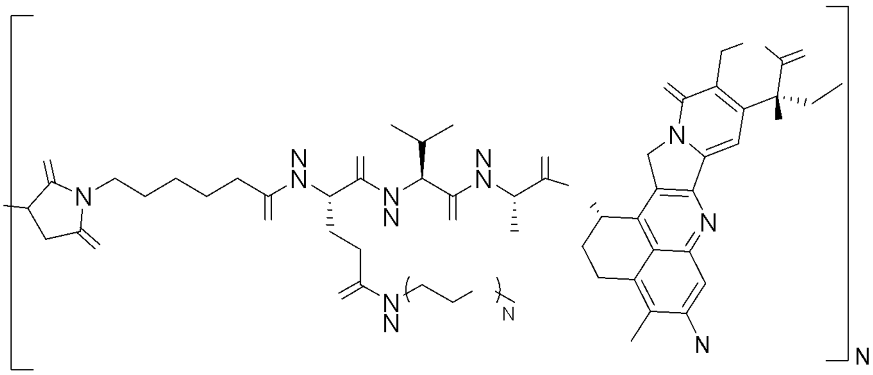

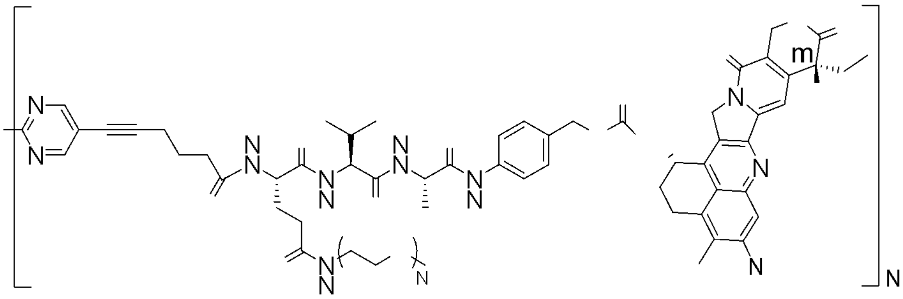

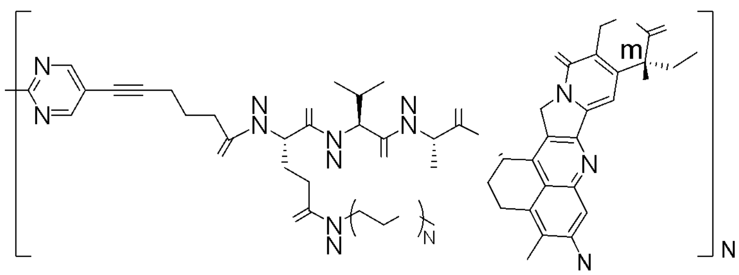

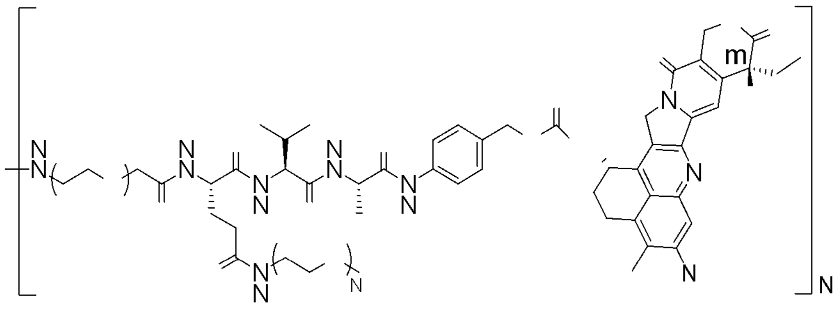

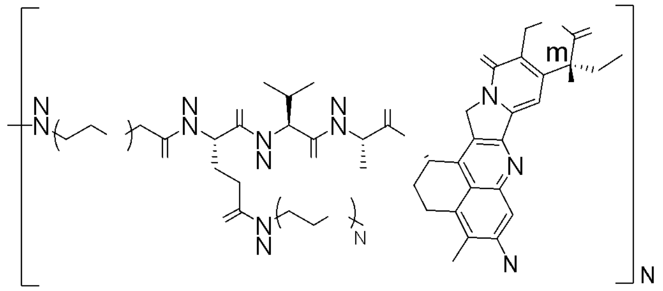

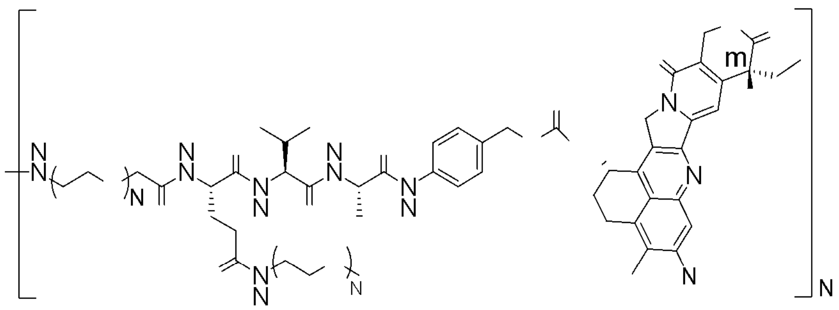

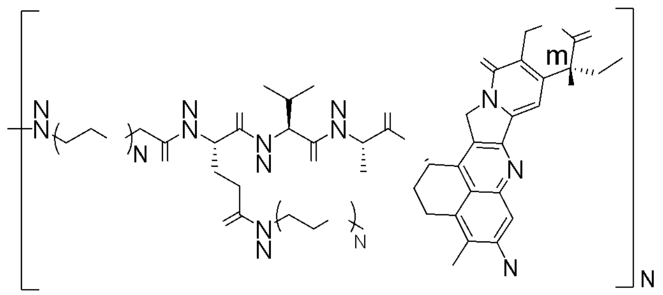

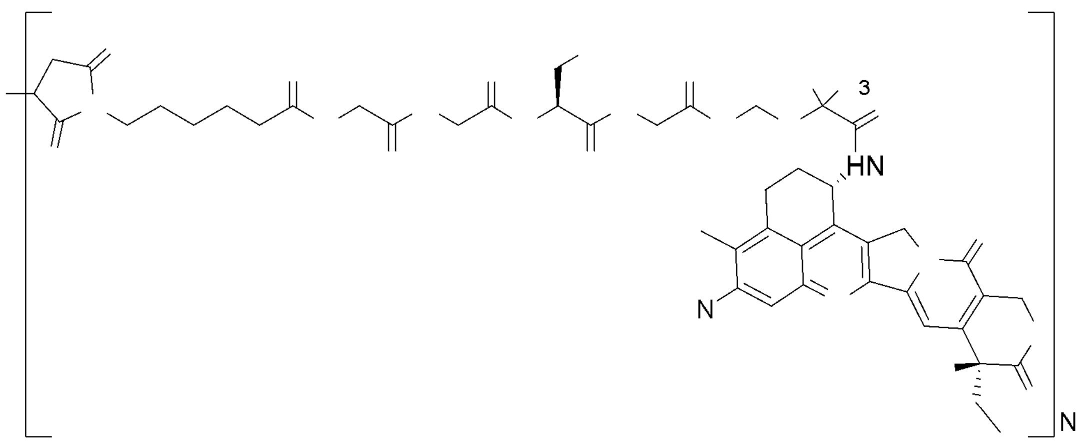

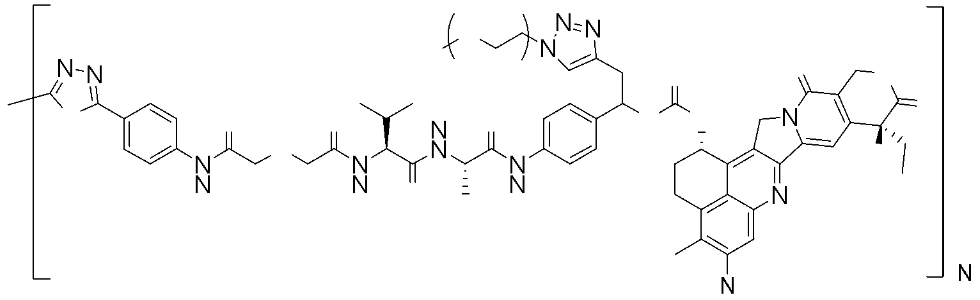

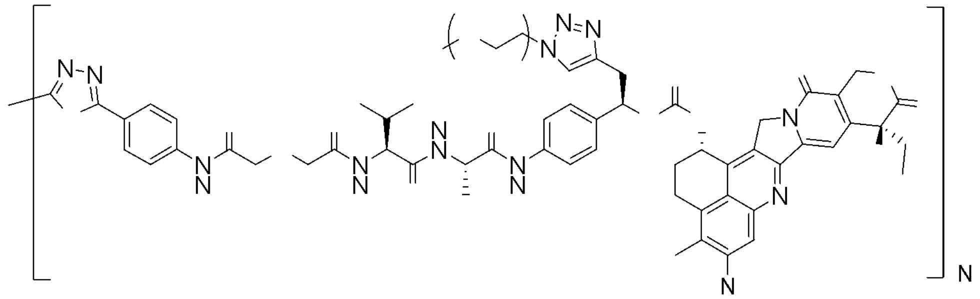

or

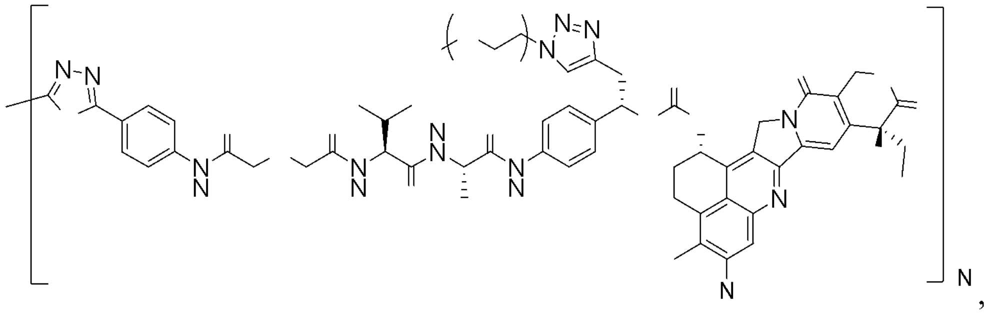

wherein n is an integer selected from 1-50, preferably 1-24, and further preferably 3-12; d is an integer or a decimal selected from 1 to 12; s is an integer selected from 1-12; m is an integer selected from 3-50, preferably an integer selected from 8-24, and further preferably 8, 9, 10, 11, 12, 13, 14, 15, 16, 17, 18, 19, 20, 21, 22, 23, or 24.

**25.** A pharmaceutical composition, comprising the antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to any one of claims 1-24.

**26.** Use of the antibody-drug conjugate having the structure represented by formula (I), and the tautomer, the mesomer, the racemate, the enantiomer, the diastereoisomer, the pharmaceutically acceptable salt, the hydrate, the solvate or the isotopically labeled compound thereof according to any one of claims 1-24 for the manufacturing of a medicament for the treatment of a proliferative disease.

**27.** The use according to claim 26, wherein the proliferative disease is preferably a disease associated with abnormal expression of HER2 or B7H3, including a cancer; the cancer is preferably breast cancer, ovarian cancer, cervical cancer, uterine cancer, prostate cancer, kidney cancer, urethral cancer, bladder cancer, liver cancer, gastric cancer, endometrial cancer, salivary gland cancer, esophageal cancer, melanoma, glioma, neuroblastoma, sarcoma, lung cancer (e.g., small cell lung cancer and non-small cell lung cancer), colon cancer, rectal cancer, colorectal cancer, leukemia (e.g., acute lymphoblastic leukemia, acute myeloid leukemia, acute promyelocytic leukemia, chronic myeloid leukemia, or chronic lymphocytic leukemia), bone cancer, skin cancer, thyroid cancer, pancreatic cancer, or lymphoma (e.g., Hodgkin’s lymphoma, non-Hodgkin’s lymphoma, or recurrent anaplastic large cell lymphoma).

**28.** A linker-drug compound having a structure represented by formula (II), and a tautomer, a mesomer, a racemate, an enantiomer, a diastereoisomer, a pharmaceutically acceptable salt, a hydrate, a solvate or an isotopically labeled compound thereof:

$$L_1'\text{-}L_2\text{-}L_3\text{-}L_4\text{-}L_5\text{-}D \qquad \text{(II)}$$

wherein $L_1'$ is selected from

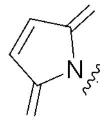, 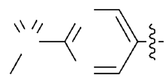, 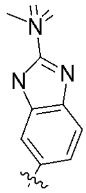, 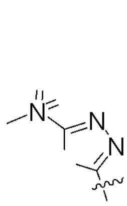, and 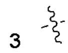;

$L_2$, $L_3$, $L_4$, $L_5$ and D are as defined in formula (I).

**29.** The linker-drug compound having the structure represented by formula (II), and the tautomer, the mesomer, the racemate, the enantiomer, the diastereoisomer, the pharmaceutically acceptable salt, the hydrate, the solvate or the isotopically labeled compound thereof according to claim 28, selected from:

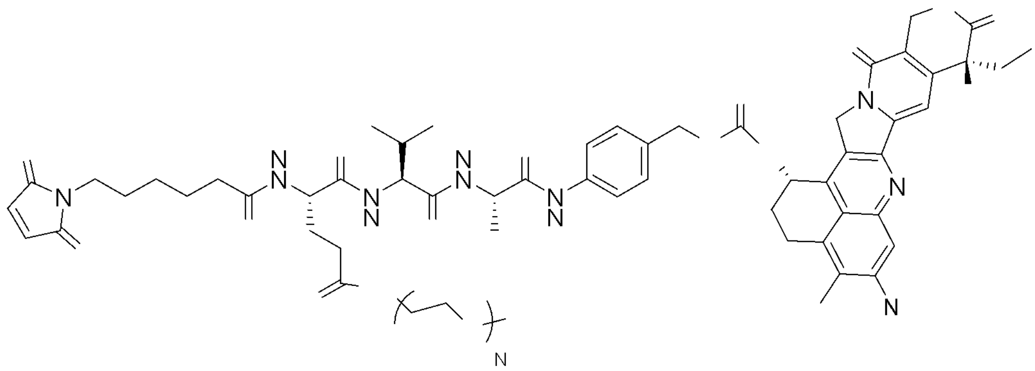

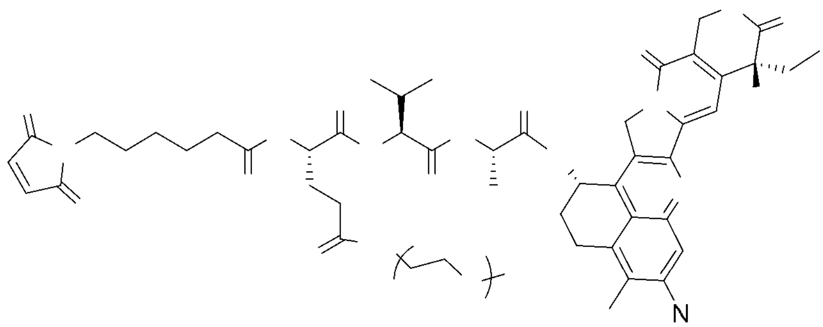

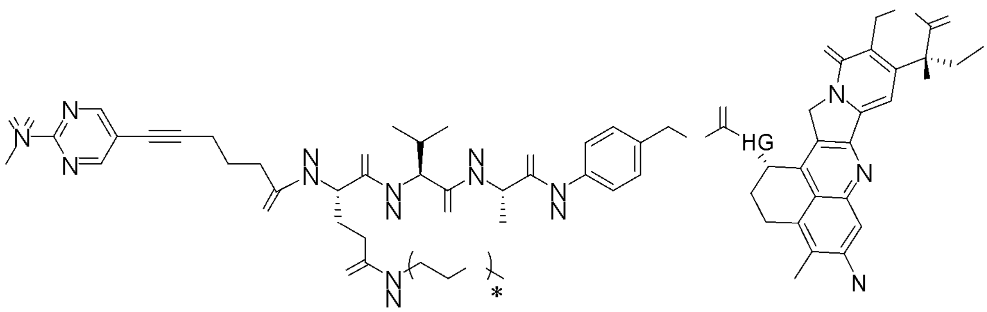

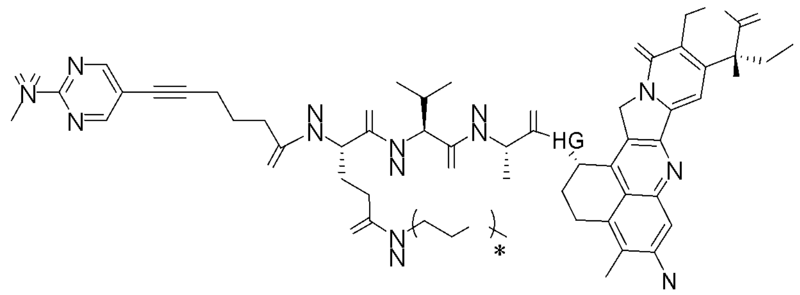

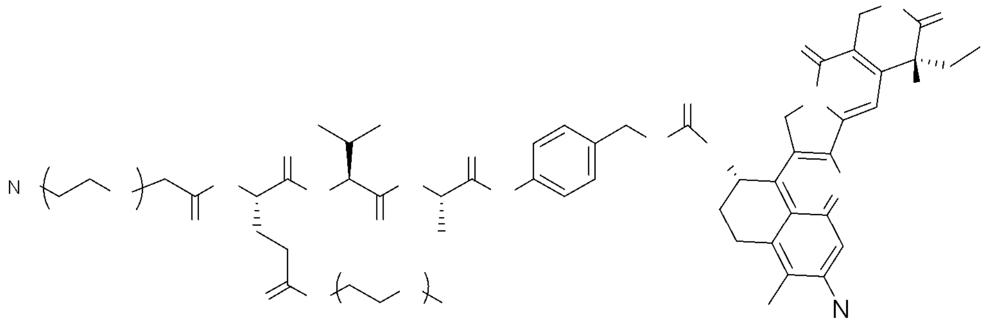

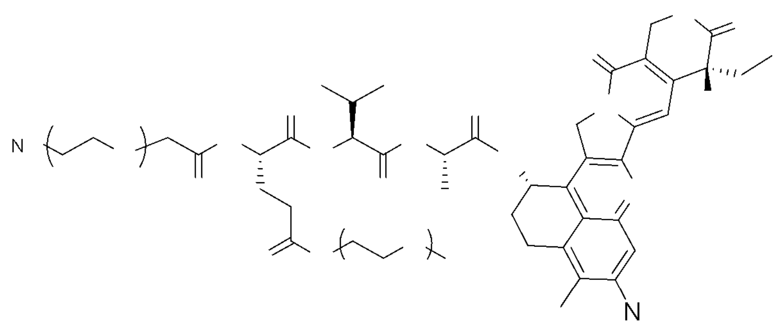

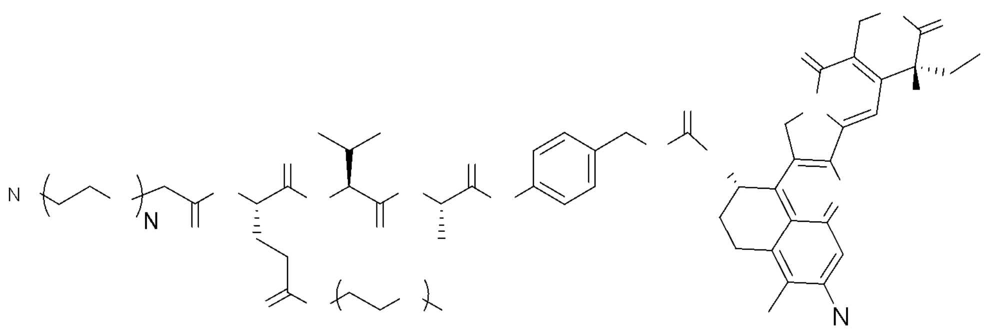

and

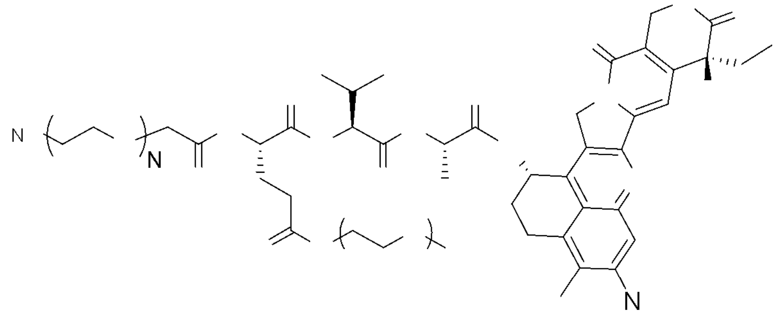

wherein n is an integer selected from 1-50, preferably 1-24, and further preferably 3-12; d is an integer or a decimal selected from 1 to 12; s is an integer selected from 1-12.

**30.** An antibody or an antigen-binding fragment thereof targeting B7H3, comprising a heavy chain and/or a light chain, wherein the heavy chain comprises 3 complementarity determining regions (CDRs), wherein the amino acid sequence of the heavy chain complementarity determining region 1 (HCDR1) is set forth in SEQ ID NO. 1, the amino acid sequence of the heavy chain complementarity determining region 2 (HCDR2) is set forth in SEQ ID NO. 2, and the amino acid sequence of the heavy chain complementarity determining region 3 (HCDR3) is set forth in SEQ ID NO. 3; the light chain comprises 3 complementarity determining regions (CDRs) of the light chain, wherein the amino acid sequence of the light chain complementarity determining region 1 (LCDR1) is set forth in SEQ ID NO. 4, the amino acid sequence of the light chain complementarity determining region 2 (LCDR2) is set forth in SEQ ID NO. 5, and the amino acid sequence of the light chain complementarity determining region 3 (LCDR3) is set forth in SEQ ID NO. 6; the CDRs are determined according to the Kabat numbering scheme.

**31.** The antibody or the antigen-binding fragment thereof targeting B7H3 according to claim 30, comprising a heavy chain and/or a light chain, wherein the sequence of a variable region of the heavy chain (HV) is set forth in SEQ ID NO. 7, and the sequence of a variable region of the light chain (LV) is set forth in SEQ ID NO. 8.

**32.** The antibody or the antigen-binding fragment thereof targeting B7H3 according to claims 30-31, comprising a heavy chain and/or a light chain, wherein the amino acid sequence of the heavy chain is set forth in SEQ ID NO. 9 or SEQ ID NO. 10, and the amino acid sequence of a variable region of the light chain is set forth in SEQ ID NO. 11.

**33.** An antibody-drug conjugate, comprising the antibody or the antigen-binding fragment thereof targeting B7H3 according to claims 30-31.

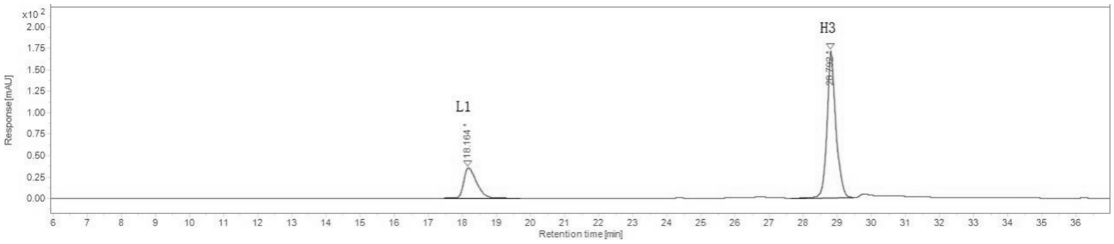


FIG. 1

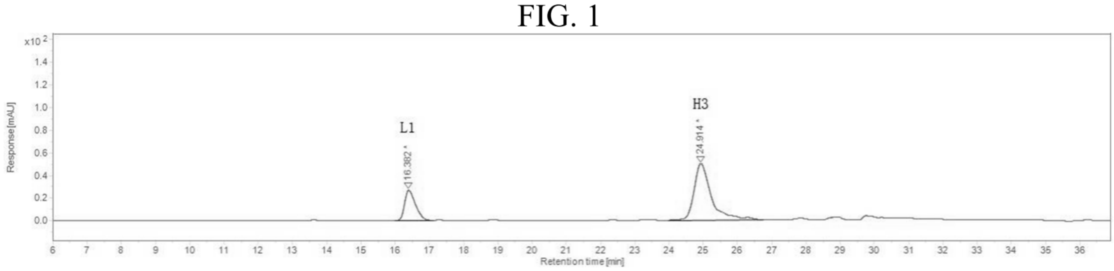


FIG. 2

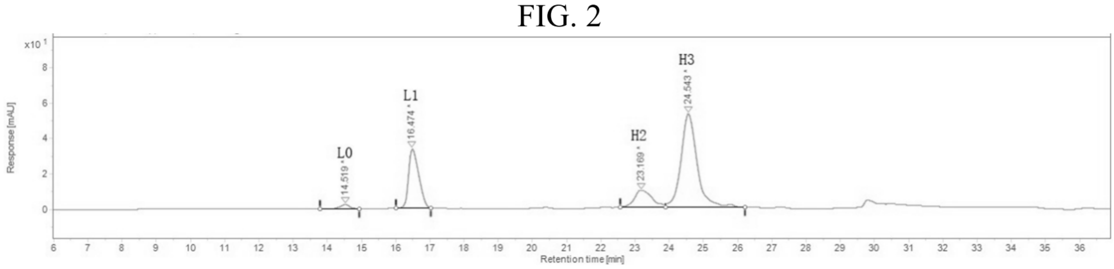


FIG. 3

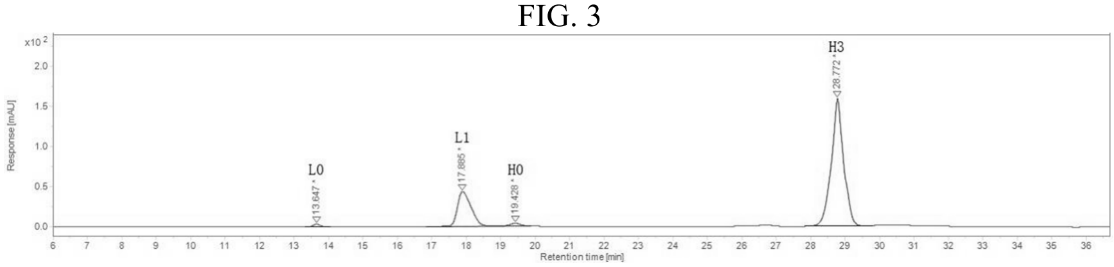


FIG. 4

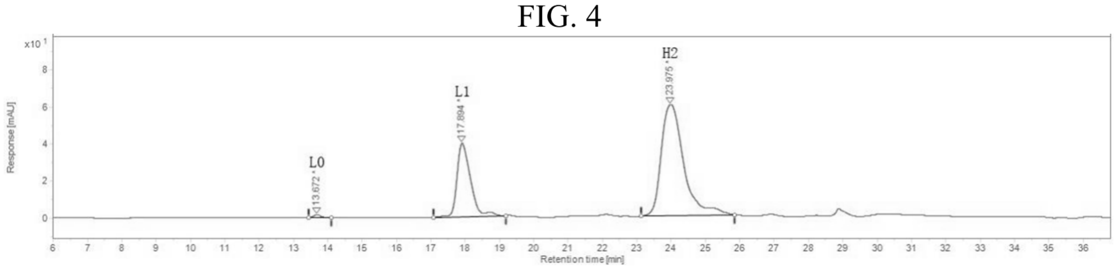


FIG. 5

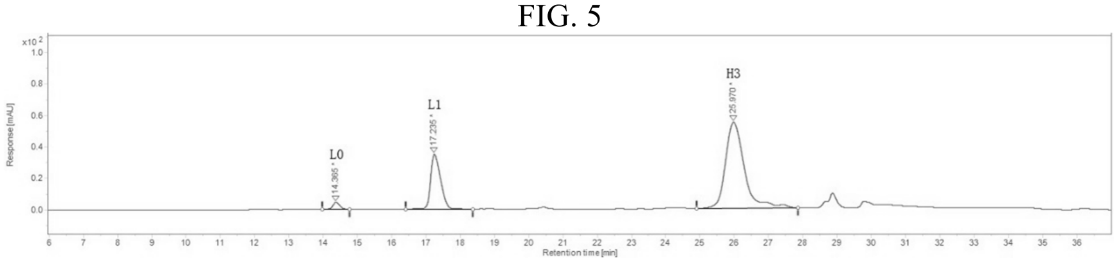


FIG. 6

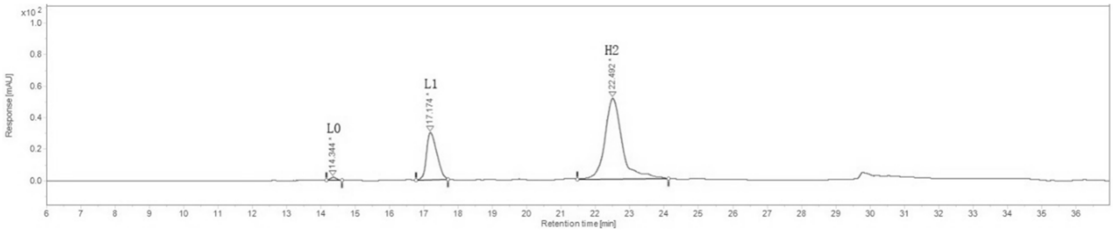


FIG. 7

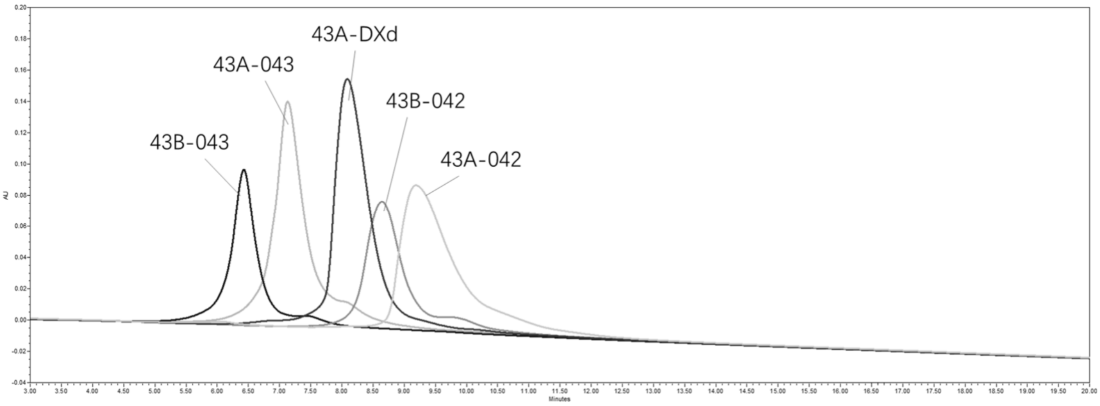


FIG. 8

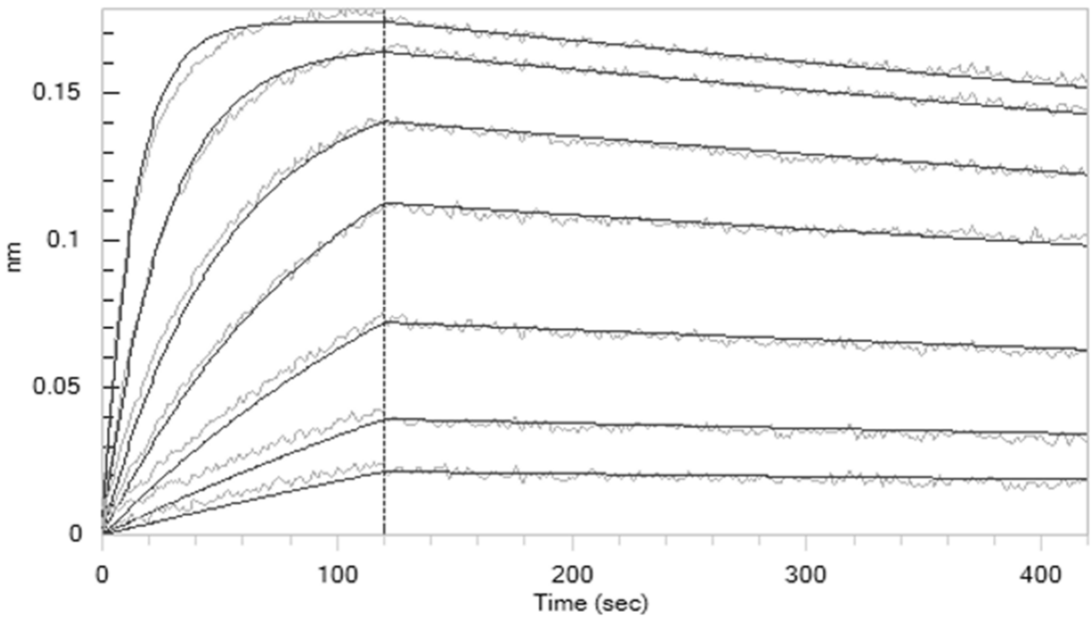


FIG. 9

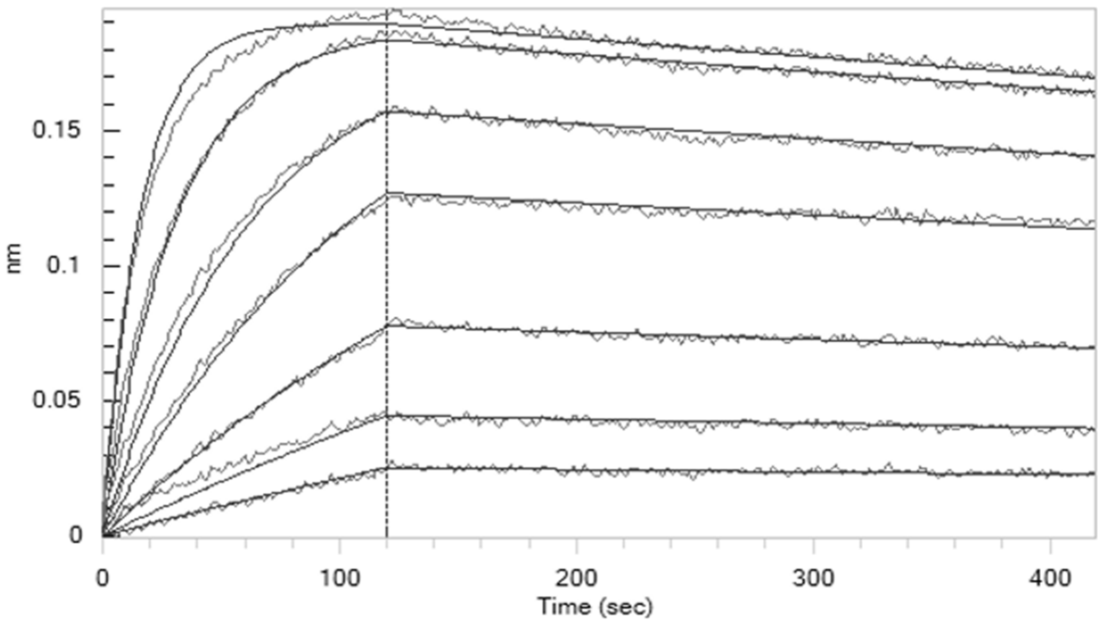
0.15
0.1
0.05
0
nm
0
100
200
300
400
Time (sec)

FIG. 10

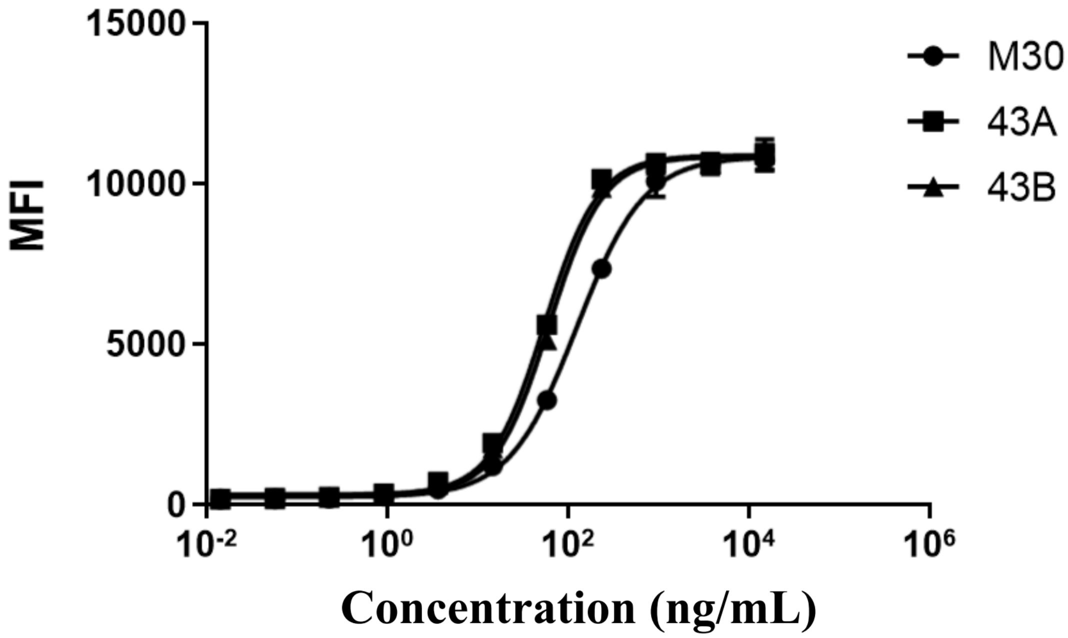
15000
10000
5000
0
MFI
M30
43A
43B
$10^{-2}$
$10^{0}$
$10^{2}$
$10^{4}$
$10^{6}$
Concentration (ng/mL)

FIG. 11

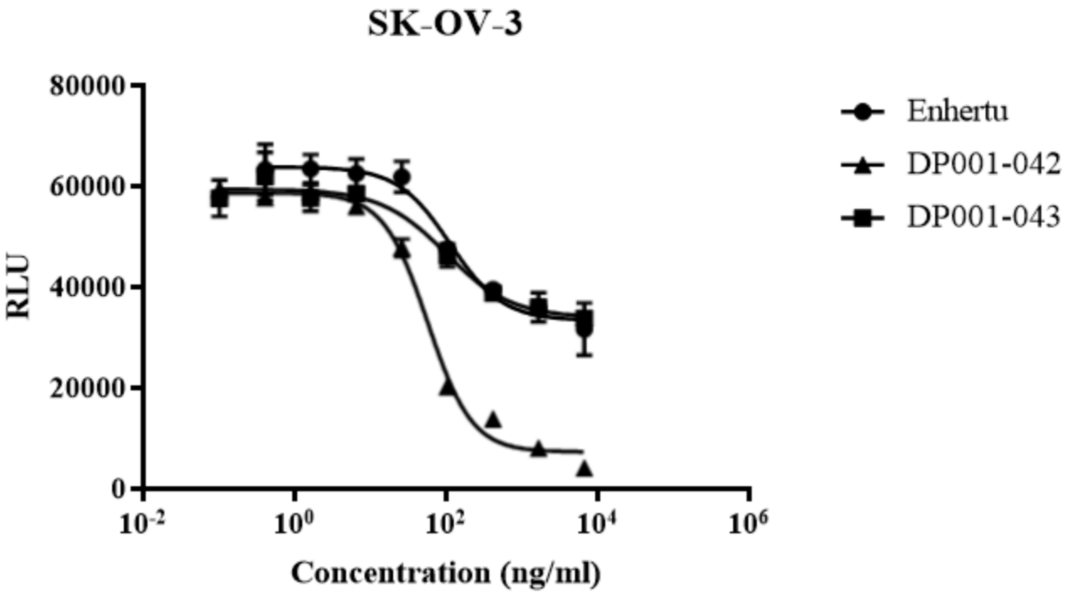
SK-OV-3
80000
60000
40000
20000
0
RLU
10-2
100
102
104
106
Concentration (ng/ml)
Enhertu
DP001-042
DP001-043

FIG. 12

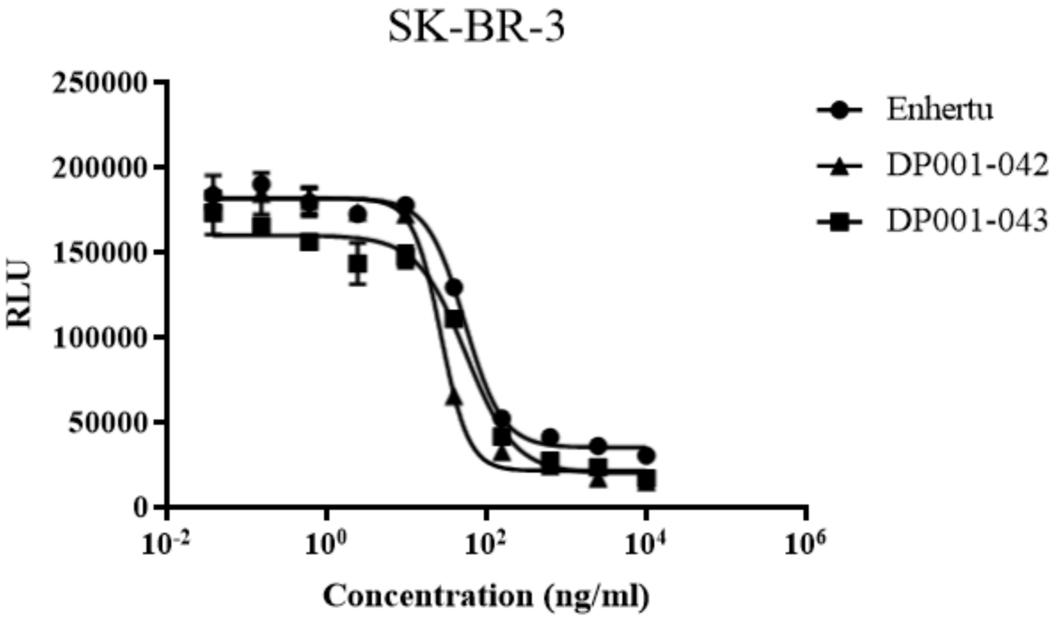
SK-BR-3
250000
200000
150000
100000
50000
0
RLU
10-2
100
102
104
106
Concentration (ng/ml)
Enhertu
DP001-042
DP001-043

FIG. 13

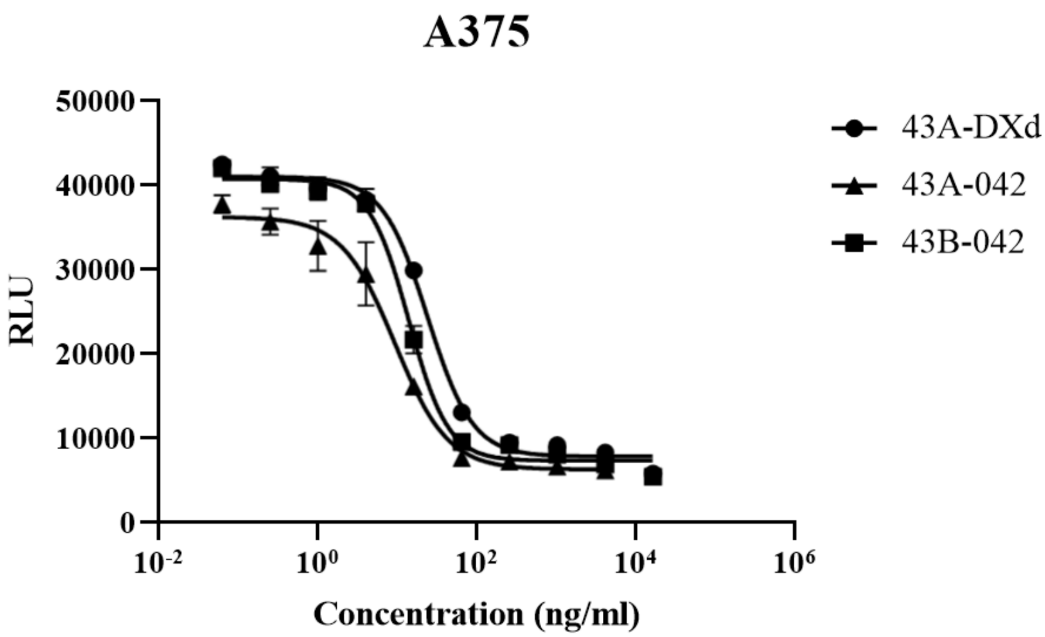
A375
50000
40000
30000
20000
10000
0
RLU
$10^{-2}$
$10^{0}$
$10^{2}$
$10^{4}$
$10^{6}$
Concentration (ng/ml)
43A-DXd
43A-042
43B-042

FIG. 14

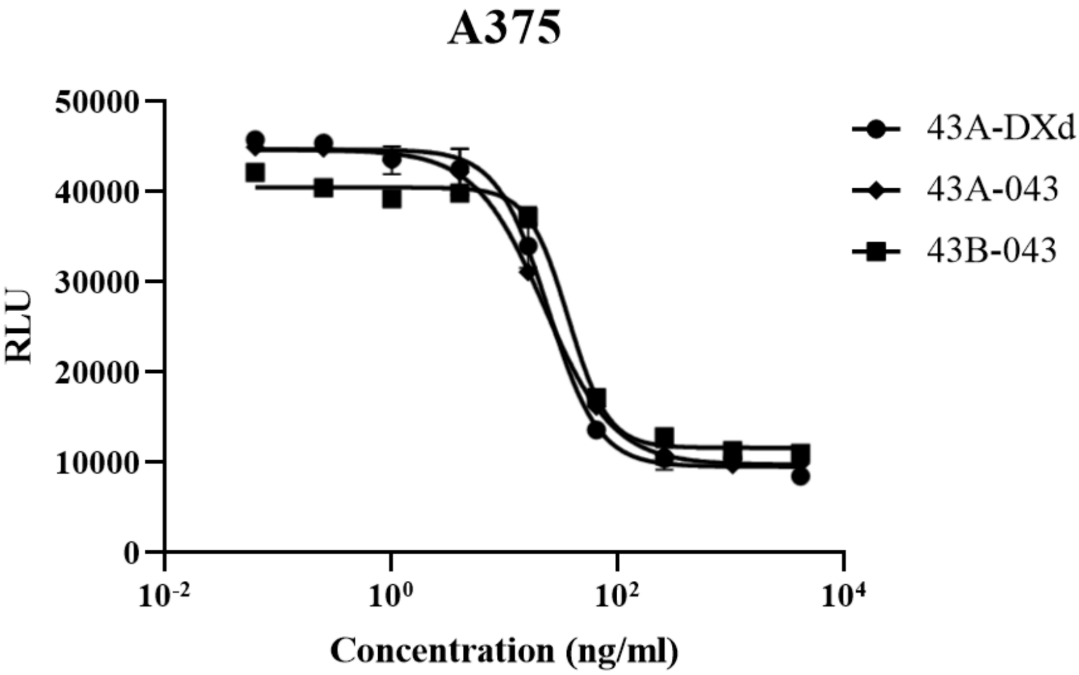
A375
50000
40000
30000
20000
10000
0
RLU
$10^{-2}$
$10^{0}$
$10^{2}$
$10^{4}$
Concentration (ng/ml)
43A-DXd
43A-043
43B-043

FIG. 15

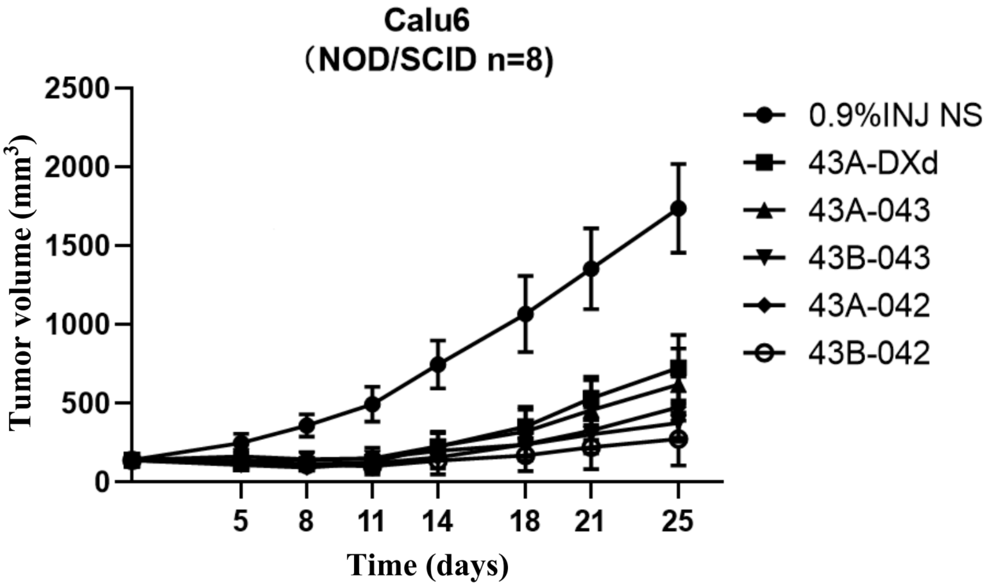
Calu6
（NOD/SCID n=8)
Tumor volume (mm³)
2500
2000
1500
1000
500
0
5
8
11
14
18
21
25
Time (days)
0.9%INJ NS
43A-DXd
43A-043
43B-043
43A-042
43B-042

FIG. 16

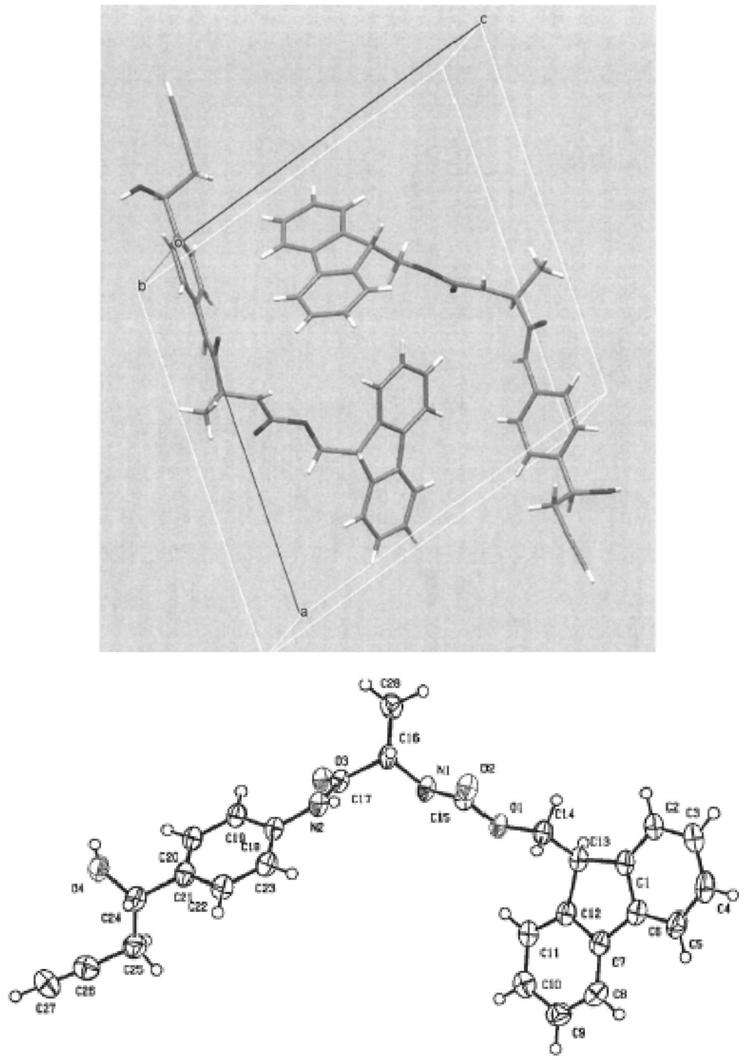

FIG. 17

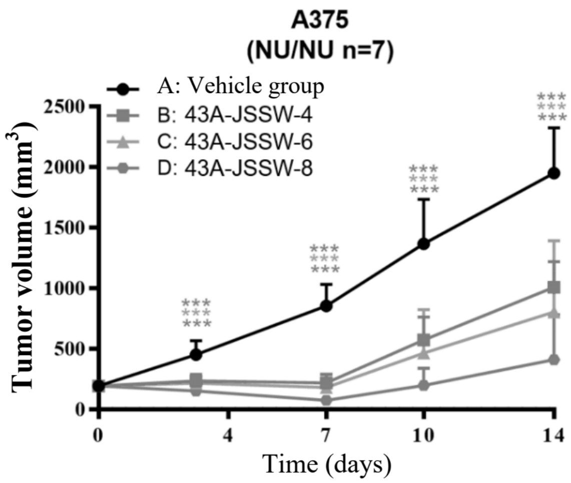


FIG. 18

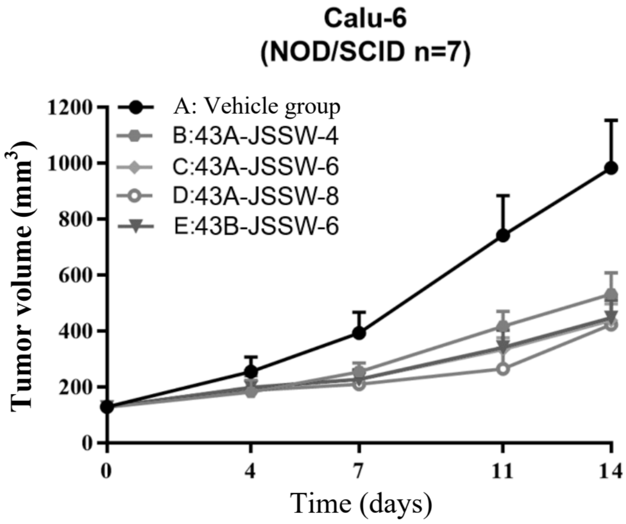


FIG. 19

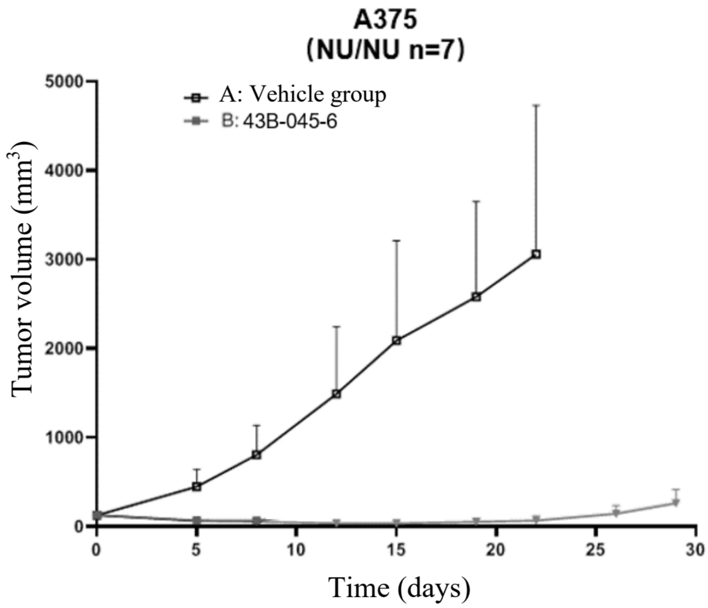


FIG. 20

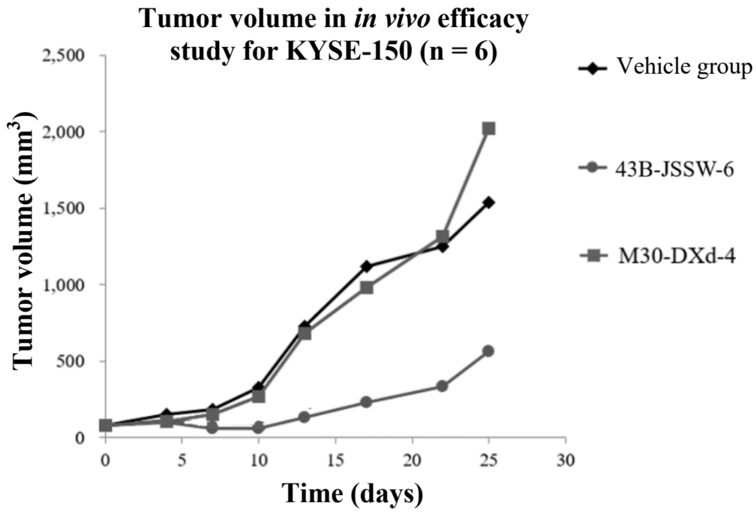


FIG. 21

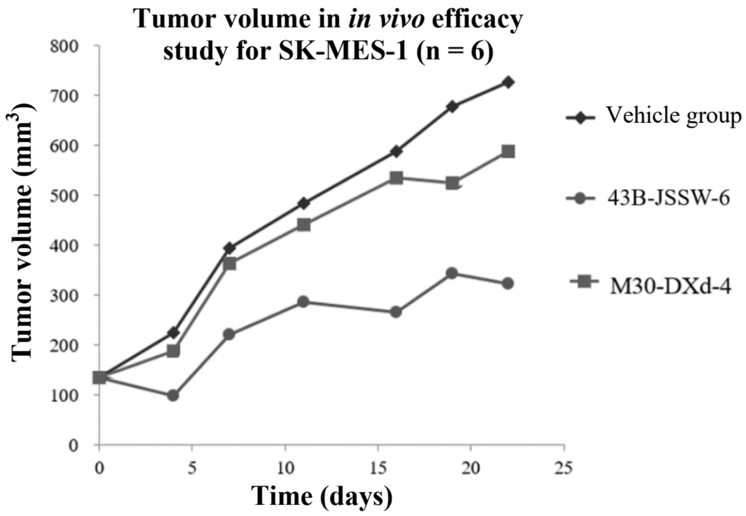


FIG. 22

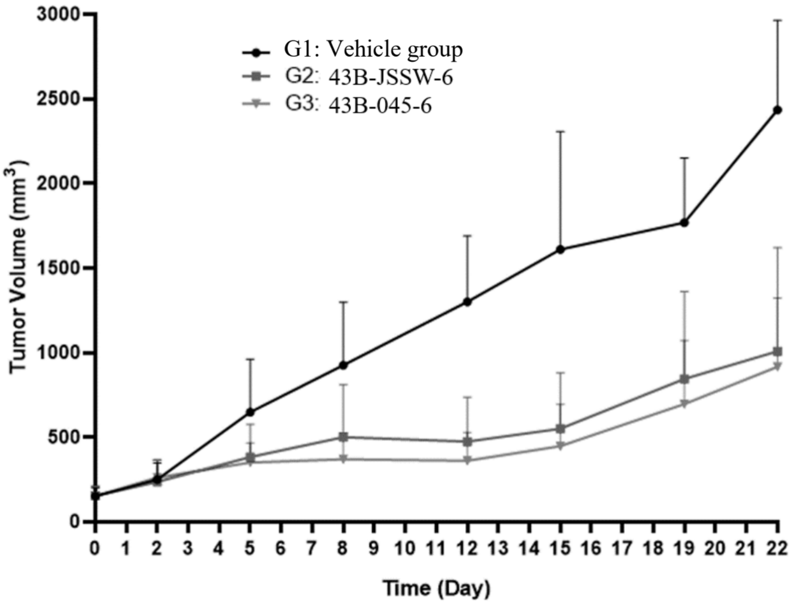


FIG. 23

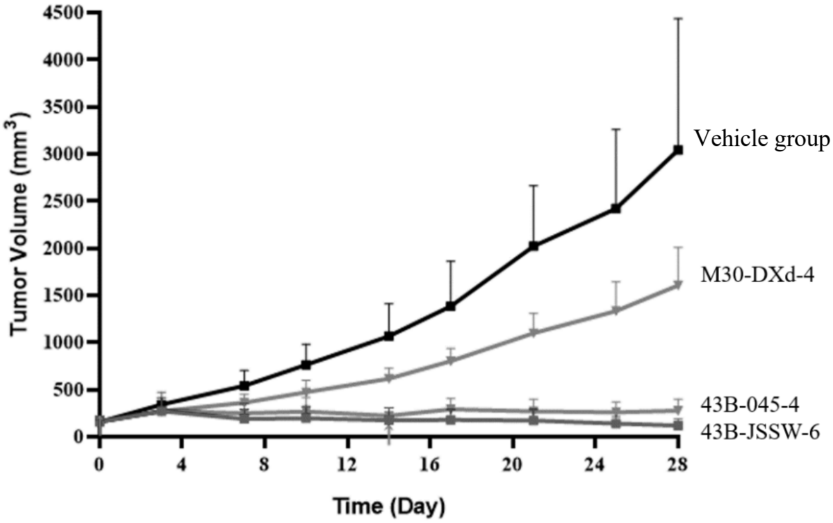
Tumor Volume (mm3)
4500
4000
3500
3000
2500
2000
1500
1000
500
0
0
4
8
12
16
20
24
28
Time (Day)
Vehicle group
M30-DXd-4
43B-045-4
43B-JSSW-6

FIG. 24

## INTERNATIONAL SEARCH REPORT

International application No.
**PCT/CN2024/102249**

**A. CLASSIFICATION OF SUBJECT MATTER**

A61K47/68(2017.01)i; C07K16/28(2006.01)i; A61K39/395(2006.01)i; A61K31/4745(2006.01)i; A61P35/00(2006.01)i; A61P35/02(2006.01)i

According to International Patent Classification (IPC) or to both national classification and IPC

**B. FIELDS SEARCHED**

Minimum documentation searched (classification system followed by classification symbols)

IPC: A61K C07K A61P

Documentation searched other than minimum documentation to the extent that such documents are included in the fields searched

Electronic data base consulted during the international search (name of data base and, where practicable, search terms used)

VCN, CJFD, DWPI, ENTXT, 生物序列检索, biological sequence search, STN REGISTER CAPLUS MARPAT, BING, NCBI, EBI: 石药集团巨石生物制药有限公司, 高晓, 潘福君, 惠希武, 赵璐, 范丽雪, 淡墨, 武玉芬, 孙雄飞, 王明晓, 姚兵, 序列检索, sequence search, 结构扩展检索, structure extended search, 喜树碱, 依喜替康, Camptothecin, Exatecan, 43A, 43B, B7H3, B7-H3, CD276, Conjugate, CAS171335-80-1扩展检索, extended search for CAS171335-80-1, CAS7689-03-4扩展检索, extended search for CAS7689-03-4

**C. DOCUMENTS CONSIDERED TO BE RELEVANT**

| Category* | Citation of document, with indication, where appropriate, of the relevant passages | Relevant to claim No. |
|---|---|---|
| X | CN 115429893 A (BIO-THERA SOLUTIONS, LTD.) 06 December 2022 (2022-12-06) claims 7, 10, and 32, and description, paragraph [0836] | 1-2, 5-7, 10-29 |
| X | WO 2022078524 A2 (HANGZHOU DAC BIOTECH CO., LTD.) 21 April 2022 (2022-04-21) description, page 125, line 1, page 183, line 3, page 247, line 5, and page 249, line 18 to page 250, line 9 | 1-2, 5-7, 10-29 |
| X | CN 115969996 A (BIO-THERA SOLUTIONS, LTD.) 18 April 2023 (2023-04-18) description, paragraphs [0007], [1037], [1055], [1126], [1127], and [1545] | 1-2, 5-7, 10-29 |
| X | WO 2023280227 A2 (PROFOUNDBIO US CO.) 12 January 2023 (2023-01-12) claims 120, 129, and 134, and description, paragraph [0533] | 1-2, 5-7, 10-29 |
| X | WO 2022102634 A1 (DAIICHI SANKYO CO., LTD.) 19 May 2022 (2022-05-19) description, paragraphs [0016]-[0017] and [0087], and claim 29 | 1-2, 5-7, 10-29 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 September 2024** | **25 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

**INTERNATIONAL SEARCH REPORT**

International application No.
**PCT/CN2024/102249**

**C. DOCUMENTS CONSIDERED TO BE RELEVANT**

| Category* | Citation of document, with indication, where appropriate, of the relevant passages | Relevant to claim No. |
|---|---|---|
| X | US 2015352224 A1 (DAIICHI SANKYO CO., LTD.) 10 December 2015 (2015-12-10) description, paragraph [1600] | 1-2, 5-7, 10-29 |
| X | CN 104755494 A (DAIICHI SANKYO CO., LTD.) 01 July 2015 (2015-07-01) description, paragraphs [0227] and [1708] | 1-2, 5-7, 10-29 |
| X | CN 107922477 A (DAIICHI SANKYO CO., LTD.) 17 April 2018 (2018-04-17) description, paragraphs [0050], [0053], and [0056] | 1-2, 5-7, 10-29 |
| X | CN 112533958 A (DAIICHI SANKYO CO., LTD.) 19 March 2021 (2021-03-19) claims 6 and 9 | 1-2, 5-7, 10-29 |
| X | CN 112512587 A (DAIICHI SANKYO CO., LTD.) 16 March 2021 (2021-03-16) claims 1 and 7 | 1-2, 5-7, 10-25, 28-29 |
| X | CN 110944667 A (DAIICHI SANKYO CO., LTD.) 31 March 2020 (2020-03-31) claims 1 and 7 | 1-2, 5-7, 10-29 |
| X | CN 112805036 A (DAIICHI SANKYO CO., LTD.) 14 May 2021 (2021-05-14) claims 1 and 7 | 1-2, 5-7, 10-29 |
| X | CN 114642739 A (TAIZHOU FUDAN ZHANGJIANG PHARMACEUTICAL CO., LTD. et al.) 21 June 2022 (2022-06-21) description, paragraphs [0088], [0101], and [0205] | 1-2, 5-7, 10-29 |
| X | CN 112543771 A (JIANGSU HENGRUI MEDICINE CO., LTD. et al.) 23 March 2021 (2021-03-23) claims 1, 19, and 28 | 1-2, 5-7, 10-29 |
| X | CN 112138171 A (SHANGHAI FUDAN-ZHANGJIANG BIO-PHARMACEUTICAL CO., LTD.) 29 December 2020 (2020-12-29) claim 7, and description, paragraph [0092] | 1-2, 5-7, 10-29 |
| X | CN 113827736 A (SICHUAN BAILI PHARMACEUTICAL CO., LTD. et al.) 24 December 2021 (2021-12-24) claims 9, 10, 12, and 20 | 1-2, 5-7, 10-29 |

Form PCT/ISA/210 (second sheet) (July 2022)

**INTERNATIONAL SEARCH REPORT**

International application No.
**PCT/CN2024/102249**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed.
   - b. ☐ furnished subsequent to the international filing date for the purposes of international search (Rule 13*ter*.1(a)),
     - ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/102249**

| Patent document cited in search report | Publication date (day/month/year) | Patent family member(s) | Publication date (day/month/year) |
|---|---|---|---|
| CN 115429893 A | 06 December 2022 | EP 4349371 A1 | 10 April 2024 |
| | | JP 2024520674 A | 24 May 2024 |
| | | WO 2022253284 A1 | 08 December 2022 |
| | | US 2024269315 A1 | 15 August 2024 |
| WO 2022078524 A2 | 21 April 2022 | WO 2022078524 A3 | 25 August 2022 |
| | | WO 2022078524 A4 | 08 December 2022 |
| | | CA 3236754 A1 | 11 May 2023 |
| | | WO 2023078021 A1 | 11 May 2023 |
| | | TW 202334217 A | 01 September 2023 |
| | | CA 3236930 A1 | 21 April 2022 |
| | | CA 3236852 A1 | 11 May 2023 |
| | | KR 20240095442 A | 25 June 2024 |
| | | KR 20240095316 A | 25 June 2024 |
| | | WO 2023078273 A1 | 11 May 2023 |
| | | AU 2022383265 A1 | 13 June 2024 |
| | | AU 2022381163 A1 | 13 June 2024 |
| | | AU 2021362997 A1 | 16 May 2024 |
| CN 115969996 A | 18 April 2023 | WO 2023061457 A1 | 20 April 2023 |
| WO 2023280227 A2 | 12 January 2023 | None | |
| WO 2022102634 A1 | 19 May 2022 | None | |
| US 2015352224 A1 | 10 December 2015 | TW 201420118 A | 01 June 2014 |
| | | US 9872924 B2 | 23 January 2018 |
| | | WO 2014061277 A1 | 24 April 2014 |
| | | JPWO 2014061277 A1 | 05 September 2016 |
| | | JP 6272230 B2 | 31 January 2018 |
| | | EP 2910573 A1 | 26 August 2015 |
| | | EP 2910573 A4 | 15 June 2016 |
| | | EP 2910573 B1 | 19 February 2020 |
| | | ES 2782248 T3 | 11 September 2020 |
| | | US 2018071403 A1 | 15 March 2018 |
| | | US 10729782 B2 | 04 August 2020 |
| CN 104755494 A | 01 July 2015 | US 2016279259 A1 | 29 September 2016 |
| | | US 9808537 B2 | 07 November 2017 |
| | | LT 2907824 T | 11 June 2018 |
| | | JP 2022008581 A | 13 January 2022 |
| | | JP 7170812 B2 | 14 November 2022 |
| | | JP 2023011799 A | 24 January 2023 |
| | | JP 7523506 B2 | 26 July 2024 |
| | | BR 122021014396 B1 | 05 July 2022 |
| | | CA 3021435 A1 | 17 April 2014 |
| | | CA 3021435 C | 12 October 2021 |
| | | JP 6030267 B1 | 24 November 2016 |
| | | JP 2017036274 A | 16 February 2017 |
| | | BR 112015006521 A2 | 05 September 2017 |
| | | BR 112015006521 B1 | 03 March 2022 |
| | | SI 2907824 T1 | 29 June 2018 |
| | | NZ 746440 A | 29 November 2019 |
| | | CY 1122789 T1 | 05 May 2021 |
| | | PT 3342785 T | 25 February 2020 |

Form PCT/ISA/210 (patent family annex) (July 2022)

**INTERNATIONAL SEARCH REPORT**
**Information on patent family members**

International application No.
**PCT/CN2024/102249**

| Patent document cited in search report | Publication date (day/month/year) | Patent family member(s) | | | Publication date (day/month/year) |
|---|---|---|---|---|---|
| | | KR | 20220100727 | A | 15 July 2022 |
| | | KR | 102456726 | B1 | 20 October 2022 |
| | | SG | 10201804788 | XA | 30 July 2018 |
| | | PT | 2907824 | T | 07 June 2018 |
| | | US | 2020282073 | A1 | 10 September 2020 |
| | | US | 11633493 | B2 | 25 April 2023 |
| | | JP | 5953378 | B2 | 20 July 2016 |
| | | JPWO | 2014057687 | A1 | 05 September 2016 |
| | | WO | 2014057687 | A1 | 17 April 2014 |
| | | CA | 2885800 | A1 | 17 April 2014 |
| | | CA | 2885800 | C | 04 December 2018 |
| | | ES | 2773710 | T3 | 14 July 2020 |
| | | US | 2024082413 | A1 | 14 March 2024 |
| | | US | 2015297748 | A1 | 22 October 2015 |
| | | US | 10195288 | B2 | 05 February 2019 |
| | | IL | 302494 | A | 01 June 2023 |
| | | IL | 302494 | B1 | 01 July 2024 |
| | | AU | 2020200548 | A1 | 13 February 2020 |
| | | AU | 2020200548 | B2 | 25 August 2022 |
| | | TW | 202233186 | A | 01 September 2022 |
| | | HRP | 20180870 | T1 | 13 July 2018 |
| | | KR | 20150067149 | A | 17 June 2015 |
| | | KR | 101841818 | B1 | 26 March 2018 |
| | | MX | 2020010964 | A | 09 November 2020 |
| | | KR | 20180030734 | A | 23 March 2018 |
| | | KR | 101901558 | B1 | 21 September 2018 |
| | | TR | 201809636 | T4 | 23 July 2018 |
| | | SG | 11201502887 | WA | 28 May 2015 |
| | | KR | 20190135559 | A | 06 December 2019 |
| | | KR | 102087017 | B1 | 10 March 2020 |
| | | CY | 1120363 | T1 | 10 July 2019 |
| | | KR | 20180105271 | A | 27 September 2018 |
| | | KR | 102052319 | B1 | 05 December 2019 |
| | | TW | 201420117 | A | 01 June 2014 |
| | | TWI | 615152 | B | 21 February 2018 |
| | | DK | 3342785 | T3 | 23 March 2020 |
| | | TW | 201811746 | A | 01 April 2018 |
| | | TWI | 650312 | B | 11 February 2019 |
| | | TW | 202033499 | A | 16 September 2020 |
| | | TWI | 757740 | B | 11 March 2022 |
| | | PL | 3342785 | T3 | 07 September 2020 |
| | | MY | 170251 | A | 13 July 2019 |
| | | EP | 3632471 | A1 | 08 April 2020 |
| | | KR | 20220029776 | A | 08 March 2022 |
| | | KR | 102417310 | B1 | 05 July 2022 |
| | | RS | 57278 | B1 | 31 August 2018 |
| | | DK | 2907824 | T3 | 23 July 2018 |
| | | ZA | 201501825 | B | 25 May 2022 |
| | | PL | 2907824 | T3 | 31 August 2018 |
| | | PH | 12018501433 | A1 | 27 February 2019 |

Form PCT/ISA/210 (patent family annex) (July 2022)

**INTERNATIONAL SEARCH REPORT**
**Information on patent family members**

International application No.
**PCT/CN2024/102249**

| Patent document cited in search report | Publication date (day/month/year) | Patent family member(s) | | | Publication date (day/month/year) |
|---|---|---|---|---|---|
| | | KR | 20210132240 | A | 03 November 2021 |
| | | KR | 102369419 | B1 | 02 March 2022 |
| | | KR | 20210038728 | A | 07 April 2021 |
| | | KR | 102320907 | B1 | 02 November 2021 |
| | | HK | 1256631 | A1 | 27 September 2019 |
| | | IL | 238143 | A0 | 31 May 2015 |
| | | IL | 238143 | B | 30 January 2020 |
| | | JP | 2016196484 | A | 24 November 2016 |
| | | JP | 6186045 | B2 | 23 August 2017 |
| | | KR | 20200026323 | A | 10 March 2020 |
| | | KR | 102237639 | B1 | 07 April 2021 |
| | | SI | 3342785 | T1 | 28 February 2020 |
| | | NZ | 705394 | A | 26 October 2018 |
| | | KR | 20230142808 | A | 11 October 2023 |
| | | AU | 2013328111 | A1 | 12 March 2015 |
| | | AU | 2013328111 | B2 | 02 November 2017 |
| | | RU | 2015113767 | A | 10 December 2016 |
| | | RU | 2664465 | C2 | 17 August 2018 |
| | | RS | 60000 | B1 | 30 April 2020 |
| | | EP | 2907824 | A1 | 19 August 2015 |
| | | EP | 2907824 | A4 | 08 June 2016 |
| | | EP | 2907824 | B1 | 11 April 2018 |
| | | IL | 271760 | A | 27 February 2020 |
| | | IL | 271760 | B | 01 August 2022 |
| | | TW | 201920098 | A | 01 June 2019 |
| | | TWI | 696611 | B | 21 June 2020 |
| | | ES | 2671644 | T3 | 07 June 2018 |
| | | MX | 2015003903 | A | 17 July 2015 |
| | | MX | 364484 | B | 29 April 2019 |
| | | JP | 2020180124 | A | 05 November 2020 |
| | | JP | 6952835 | B2 | 27 October 2021 |
| | | NZ | 740948 | A | 29 November 2019 |
| | | EP | 3342785 | A1 | 04 July 2018 |
| | | EP | 3342785 | B1 | 25 December 2019 |
| | | IL | 313147 | A | 01 July 2024 |
| | | HK | 1210477 | A1 | 22 April 2016 |
| | | NZ | 746439 | A | 29 November 2019 |
| | | BR | 122021014365 | B1 | 05 July 2022 |
| | | JP | 2018188455 | A | 29 November 2018 |
| | | JP | 6715888 | B2 | 01 July 2020 |
| | | KR | 20220146669 | A | 01 November 2022 |
| | | KR | 102498405 | B1 | 09 February 2023 |
| | | HUE | 039000 | T2 | 28 December 2018 |
| | | KR | 20230086800 | A | 15 June 2023 |
| | | KR | 102584005 | B1 | 27 September 2023 |
| | | LT | 3342785 | T | 10 February 2020 |
| | | ZA | 201900820 | B | 31 August 2022 |
| | | PH | 12015500667 | A1 | 18 May 2015 |
| | | MX | 2019004502 | A | 21 August 2019 |
| | | US | 2019008981 | A1 | 10 January 2019 |

Form PCT/ISA/210 (patent family annex) (July 2022)

**INTERNATIONAL SEARCH REPORT**
**Information on patent family members**

International application No.
**PCT/CN2024/102249**

| Patent document cited in search report | Publication date (day/month/year) | Patent family member(s) | Publication date (day/month/year) |
|---|---|---|---|
| | | US 10973924 B2 | 13 April 2021 |
| | | JP 2018008982 A | 18 January 2018 |
| | | JP 6371452 B2 | 08 August 2018 |
| | | AU 2022203560 A1 | 16 June 2022 |
| | | KR 20230024433 A | 20 February 2023 |
| | | KR 102540419 B1 | 05 June 2023 |
| | | RU 2018128384 A | 02 October 2018 |
| | | RU 2018128384 A3 | 20 December 2021 |
| | | AU 2018200308 A1 | 01 February 2018 |
| | | AU 2018200308 B2 | 07 November 2019 |
| | | IL 294622 A | 01 September 2022 |
| | | IL 294622 B1 | 01 June 2023 |
| | | IL 294622 B2 | 01 October 2023 |
| | | HUE 048748 T2 | 28 August 2020 |
| | | HRP 20200353 T1 | 12 June 2020 |
| CN 107922477 A | 17 April 2018 | BR 112017027690 A2 | 09 October 2018 |
| | | IL 256558 A | 28 February 2018 |
| | | IL 256558 B | 01 April 2022 |
| | | US 2018147292 A1 | 31 May 2018 |
| | | US 11173213 B2 | 16 November 2021 |
| | | CA 2990572 A1 | 05 January 2017 |
| | | CA 2990572 C | 26 July 2022 |
| | | HK 1247211 A1 | 21 September 2018 |
| | | TW 201705982 A | 16 February 2017 |
| | | TWI 725037 B | 21 April 2021 |
| | | IL 290959 A | 01 April 2022 |
| | | IL 290959 B | 01 December 2022 |
| | | IL 290959 B2 | 01 April 2023 |
| | | EP 4180455 A1 | 17 May 2023 |
| | | JP 2021020956 A | 18 February 2021 |
| | | JP 7118117 B2 | 15 August 2022 |
| | | US 2021386865 A1 | 16 December 2021 |
| | | HUE 061408 T2 | 28 June 2023 |
| | | KR 20180021723 A | 05 March 2018 |
| | | TW 202130368 A | 16 August 2021 |
| | | TWI 827921 B | 01 January 2024 |
| | | ES 2938186 T3 | 05 April 2023 |
| | | JPWO 2017002776 A1 | 31 May 2018 |
| | | JP 6787890 B2 | 18 November 2020 |
| | | AU 2016286898 A1 | 04 January 2018 |
| | | AU 2016286898 B2 | 08 December 2022 |
| | | EP 3315512 A1 | 02 May 2018 |
| | | EP 3315512 A4 | 13 February 2019 |
| | | EP 3315512 B1 | 23 November 2022 |
| | | WO 2017002776 A1 | 05 January 2017 |
| CN 112533958 A | 19 March 2021 | CA 3107732 A1 | 30 January 2020 |
| | | JPWO 2020022475 A1 | 12 August 2021 |
| | | JP 7406488 B2 | 27 December 2023 |
| | | US 2021169852 A1 | 10 June 2021 |
| | | WO 2020022475 A1 | 30 January 2020 |

Form PCT/ISA/210 (patent family annex) (July 2022)

**INTERNATIONAL SEARCH REPORT**
**Information on patent family members**

International application No.
**PCT/CN2024/102249**

| Patent document cited in search report | Publication date (day/month/year) | Patent family member(s) | Publication date (day/month/year) |
|---|---|---|---|
| | | IL 280296 A | 25 March 2021 |
| | | AU 2019311596 A1 | 28 January 2021 |
| | | EP 3831853 A1 | 09 June 2021 |
| | | EP 3831853 A4 | 01 June 2022 |
| | | TW 202019973 A | 01 June 2020 |
| | | JP 2024045098 A | 02 April 2024 |
| | | JP 7502549 B2 | 18 June 2024 |
| | | KR 20210040059 A | 12 April 2021 |
| CN 112512587 A | 16 March 2021 | TW 202019487 A | 01 June 2020 |
| | | BR 112021001750 A2 | 27 April 2021 |
| | | EP 3834843 A1 | 16 June 2021 |
| | | EP 3834843 A4 | 11 May 2022 |
| | | EA 202190471 A1 | 24 May 2021 |
| | | JP 2024075668 A | 04 June 2024 |
| | | WO 2020031936 A1 | 13 February 2020 |
| | | CA 3108754 A1 | 13 February 2020 |
| | | AU 2019320336 A1 | 04 March 2021 |
| | | JPWO 2020031936 A1 | 10 August 2021 |
| | | JP 7458981 B2 | 01 April 2024 |
| | | KR 20210042120 A | 16 April 2021 |
| | | US 2021290775 A1 | 23 September 2021 |
| CN 110944667 A | 31 March 2020 | JPWO 2019039483 A1 | 01 October 2020 |
| | | JP 7204651 B2 | 16 January 2023 |
| | | RU 2020111448 A | 24 September 2021 |
| | | RU 2020111448 A3 | 29 November 2021 |
| | | WO 2019039483 A1 | 28 February 2019 |
| | | JP 2023036900 A | 14 March 2023 |
| | | EP 3673918 A1 | 01 July 2020 |
| | | EP 3673918 A4 | 19 May 2021 |
| | | IL 272721 A | 30 April 2020 |
| | | KR 20200044044 A | 28 April 2020 |
| | | MA 49987 A | 01 July 2020 |
| | | US 2021128741 A1 | 06 May 2021 |
| | | TW 201919710 A | 01 June 2019 |
| | | SG 11202000996 UA | 30 March 2020 |
| | | BR 112020003474 A2 | 20 October 2020 |
| | | CA 3073383 A1 | 28 February 2019 |
| | | CA 3073383 C | 31 October 2023 |
| | | AU 2018320470 A1 | 13 February 2020 |
| CN 112805036 A | 14 May 2021 | TW 202415410 A | 16 April 2024 |
| | | WO 2020027100 A1 | 06 February 2020 |
| | | JPWO 2020027100 A1 | 02 August 2021 |
| | | JP 7473474 B2 | 23 April 2024 |
| | | KR 20210038625 A | 07 April 2021 |
| | | IL 280510 A | 25 March 2021 |
| | | US 2021290777 A1 | 23 September 2021 |
| | | TW 202011998 A | 01 April 2020 |
| | | TWI 822822 B | 21 November 2023 |
| | | SG 11202100947 SA | 30 March 2021 |
| | | CA 3108044 A1 | 06 February 2020 |

Form PCT/ISA/210 (patent family annex) (July 2022)

**INTERNATIONAL SEARCH REPORT**
**Information on patent family members**

International application No.
**PCT/CN2024/102249**

| Patent document cited in search report | Publication date (day/month/year) | Patent family member(s) | Publication date (day/month/year) |
|---|---|---|---|
| | | EP 3831412 A1 | 09 June 2021 |
| | | EP 3831412 A4 | 27 April 2022 |
| | | EA 202190403 A1 | 24 May 2021 |
| | | JP 2024095789 A | 10 July 2024 |
| | | BR 112021001509 A2 | 27 April 2021 |
| | | AU 2019315177 A1 | 25 February 2021 |
| CN 114642739 A | 21 June 2022 | None | |
| CN 112543771 A | 23 March 2021 | MX 2021003446 A | 15 June 2021 |
| | | JP 2022512568 A | 07 February 2022 |
| | | JP 7408646 B2 | 05 January 2024 |
| | | EP 3854816 A1 | 28 July 2021 |
| | | EP 3854816 A4 | 07 September 2022 |
| | | WO 2020063673 A1 | 02 April 2020 |
| | | TW 202028242 A | 01 August 2020 |
| | | ZA 202102696 B | 25 October 2023 |
| | | CA 3114474 A1 | 02 April 2020 |
| | | AU 2019351427 A1 | 15 April 2021 |
| | | US 2021347894 A1 | 11 November 2021 |
| | | KR 20210068457 A | 09 June 2021 |
| | | BR 112021004829 A2 | 08 June 2021 |
| CN 112138171 A | 29 December 2020 | None | |
| CN 113827736 A | 24 December 2021 | US 2023226207 A1 | 20 July 2023 |
| | | MX 2022015695 A | 21 March 2023 |
| | | IL 298787 A | 01 February 2023 |
| | | AU 2021289927 A1 | 19 January 2023 |
| | | CA 3186295 A1 | 16 December 2021 |
| | | BR 112022024930 A2 | 27 December 2022 |
| | | JP 2023529415 A | 10 July 2023 |
| | | KR 20230022211 A | 14 February 2023 |
| | | EP 4162954 A1 | 12 April 2023 |
| | | WO 2021249228 A1 | 16 December 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023107806802
- CN 2024101255800
- WO 2020233174 A1
- CN 103687945 B


### Non-patent literature cited in the description


- **BECK A** ; **REICHERT JM**. Antibody-drug conjugates: Present and future. *MAbs*, 2014, vol. 6, 15-17
- **MCCOMBS J R** ; **OWEN S C**. Antibody drug conjugates: design and selection of linker, payload and conjugation chemistry. *The AAPS journal*, 2015, vol. 17, 339-351